(19) Europäisches Patentamt
European Patent Office
Office européen des brevets

(11) Veröffentlichungsnummer : **0 165 897**
**B1**

(12) # EUROPÄISCHE PATENTSCHRIFT

(45) Veröffentlichungstag der Patentschrift : **15.03.89**

(21) Anmeldenummer : **85810237.9**

(22) Anmeldetag : **17.05.85**

(51) Int. Cl.[4] : **C 07 C103/38**, C 07 C121/80, C 07 D257/04, A 61 K 31/225, A 61 K 31/19, A 61 K 31/275, A 61 K 31/41

(54) **Neue Resorcinäther.**

(30) Priorität : **24.05.84 CH 2558/84**
**15.02.85 CH 702/85**

(43) Veröffentlichungstag der Anmeldung : **27.12.85 Patentblatt 85/52**

(45) Bekanntmachung des Hinweises auf die Patenterteilung : **15.03.89 Patentblatt 89/11**

(84) Benannte Vertragsstaaten : **AT BE CH DE FR GB IT LI LU NL SE**

(56) Entgegenhaltungen :
**EP--A-- 0 147 973**
**US--A-- 3 966 965**
**US--A-- 4 448 729**

(73) Patentinhaber : **CIBA-GEIGY AG**
**Klybeckstrasse 141**
**CH-4002 Basel (CH)**

(72) Erfinder : **Wenk, Paul, Dr.**
**Quellenweg 7**
**CH-4123 Allschwil (CH)**
Erfinder : **Sallmann, Alfred, Dr.**
**Joachimsackerstrasse 12**
**CH-4103 Bottmingen (CH)**
Erfinder : **Beck, Andreas, Dr.**
**Kohlerweg 23**
**D-7800 Freiburg (DE)**

**EP 0 165 897 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 4-Acylresorcinäther der Formel

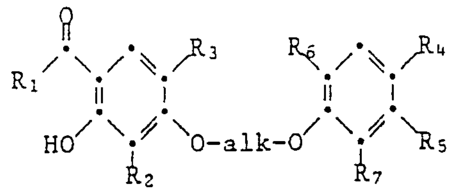

(I)

worin $R_1$ Niederalkyl bedeutet, $R_2$ Niederalkyl, Niederalkenyl oder Niederalkinyl darstellt, $R_3$ Wasserstoff, Niederalkoxy, Trifluormethyl oder Halogen bedeutet, alk einen Alkylenrest darstellt, einer der Reste $R_4$, $R_5$ und $R_7$ für eine Gruppe der Formel —NH—C(=O)—$R_8$, ein davon verschiedener Rest $R_4$ oder $R_5$ für einen Rest $R_9$ und ein davon verschiedener Rest $R_7$ für einen Rest $R_{10}$ steht, $R_6$ Wasserstoff, Halogen, Niederalkyl, Trifluormethyl, gegebenenfalls verestertes oder amidiertes Carboxy, Cyano oder Niederalkanoyl darstellt, $R_8$ gegebenenfalls verestertes oder amidiertes Carboxy oder 5-Tetrazolyl bedeutet, $R_9$ Wasserstoff, Niederalkyl, Niederalkoxy, Halogen oder Trifluormethyl bedeutet, und $R_{10}$ für Wasserstoff, Niederalkyl, Niederalkoxy, Halogen, Trifluormethyl, Cyano oder gegebenenfalls verestertes oder amidiertes Carboxy steht, mit der Massgabe, dass in Verbindungen der Formel I, worin $R_4$ eine Gruppe —NH—C(=O)—$R_8$ und $R_8$ 5-Tetrazolyl bedeutet, mindestens einer der Reste $R_3$, $R_6$, $R_9$ und $R_{10}$ von Wasserstoff verschieden ist, und ihre Salze.

Alkylenreste alk weisen 2 bis 9 Kettenglieder auf und sind beispielsweise geradkettige Alkylenreste der Formel

$$—(CH_2)_m— \qquad (Ia),$$

worin m eine ganze Zahl von 2 bis und mit 9 bedeutet, können aber auch verzweigte, insbesondere in höherer als der α- und in niederer als der ω-Stellung verzweigte, Alkylenreste sein und sind vorzugsweise Niederalkylenreste der genannten Art.

Verestertes Carboxy ist beispielsweise Niederalkoxycarbonyl, kann im Falle von $R_8$ aber auch N,N-Diniederalkylaminoniederalkoxycarbonyl, N,N-Niederalkylenaminoniederalkoxycarbonyl, gegebenenfalls substituiertes N,N-(Aza)niederalkylenaminoniederalkoxycarbonyl, N,N-(Oxa)-niederalkylenaminoniederalkoxycarbonyl oder N,N-(Thia)niederalkylenaminoniederalkoxycarbonyl sein.

Amidiertes Carboxy ist beispielsweise Carbamyl, N-Mono- oder N,N-Diniederalkylcarbamyl, ferner N,N-Niederalkylen- bzw. N,N-(Aza)-niederalkylen-, N,N-(Oxa)niederalkylen- oder N,N-(Thia) niederalkylencarbamyl, kann im Falle von $R_8$ aber auch N-(N',N'-Diniederalkylaminoniederalkyl)carbamyl, N-(N',N'-Niederalkylenaminoniederalkyl)-carbamyl bzw. gegebenenfalls substituiertes N-[N',N'-(Aza) niederalkylenaminoniederalkyl]-carbamyl, N-[N',N'-(Oxa) niederalkylenaminoniederalkyl]- oder N-[N',N'-(Thia) niederalkylenaminoniederalkyl]-carbamyl sein.

Vor- und nachstehend sind unter « niederen » organischen Verbindungen und davon abgeleiteten Gruppen solche zu verstehen, die bis und mit 7, insbesondere bis und mit 4, Kohlenstoffatome (C-Atome) aufweisen.

Niederalkyl bedeutet beispielsweise Methyl, Aethyl, Propyl, Isopropyl oder Butyl, ferner Sekundär- oder Tertiärbutyl.

Niederalkoxy bedeutet beispielsweise Methoxy, Aethoxy, Propyloxy, Isopropyloxy oder Butyloxy.

Halogen weist beispielsweise die Atomnummer bis und mit 53, insbesondere 17 bis und mit 53, auf und bedeutet beispielsweise Fluor, Chlor, Brom oder Jod.

Niederalkenyl ist beispielsweise Allyl, ferner Methallyl oder But-4-enyl.

Niederalkinyl ist beispielsweise Propargyl.

Niederalkanoyl ist beispielsweise Formyl, Acetyl, Propionyl, Butyryl, Valeroyl oder Pivaloyl.

Geradkettiges Niederalkylen ist beispielsweise Aethylen, 1,3-Propylen, 1,4-Butylen, 1,5-Pentylen, 1,6-Hexylen oder 1,7-Heptylen kann aber auch 1,2-Propylen, 1,3-Butylen oder 2,4-Pentylen sein.

Verzweigtes Niederalkylen ist beispielsweise 1,3-(2-Methyl)-propylen oder 1,3-(2,2-Dimethyl) propylen.

Niederalkoxycarbonyl ist beispielsweise Methoxy-, Aethoxy-, Propyloxy-, Isopropyloxy- oder Butyloxycarbonyl.

N,N-Diniederalkylaminoniederalkoxycarbonyl ist beispielsweise 2-(Dimethylamino) äthoxycarbonyl, 2-(Diäthylamino) äthoxycarbonyl oder 3-(Dimethylamino) propyloxycarbonyl.

N,N-Niederalkylenaminoniederalkoxycarbonyl ist beispielsweise 2-(Pyrrolidino)-, 2-(Piperidino)- oder 2-(Tetrahydroazepino) äthoxycarbonyl.

Gegebenenfalls substituiertes N,N-(Aza) niederalkylenaminoniederalkoxycarbonyl ist beispielsweise 2-(Piperazino) äthoxycarbonyl, 2-(4-Methylpiperazino) äthoxycarbonyl oder im Phenylteil gegebenenfalls

durch Niederalkyl, Niederalkoxy, Halogen und/oder Trifluormethyl substituiertes 2-(4-Phenylpiperazino) äthoxycarbonyl.

N,N-(Oxa)- bzw. N,N-(Thia) niederalkylaminoniederalkoxycarbonyl ist beispielsweise 2-(Morpholino)- bzw. 2-(Thiomorpholino) äthoxycarbonyl.

N-Mono- oder N,N-Diniederalkylcarbamyl ist beispielsweise N-Methyl-, N-Aethyl- oder N,N-Dimethylcarbamyl.

N,N-Niederalkylencarbamyl bzw. N,N-(Aza)-, N,N-(Oxa)- oder N,N-(Thia) niederalkylencarbamyl ist beispielsweise Pyrrolidino-, Piperidino-, Pyridazino-, (4-Methyl) piperazino-, Morpholino- oder Thiomorpholinocarbonyl.

N-(N',N'-Diniederalkylaminoniederalkyl) carbamyl ist beispielsweise N-[2-(N',N'-Dimethylamino) äthyl]- oder N-[2-(N',N'-Diäthylamino)-äthyl]-carbamyl.

N-(Niederalkylenaminoniederalkyl) carbamyl ist beispielsweise N-[2-(Pyrrolidino) äthyl]- oder N-[2-(Piperidino) äthyl]-carbamyl.

Gegebenenfalls substituiertes N-[N',N'-(Aza) niederalkylenaminoniederalkyl]-carbamyl ist beispielsweise N-[2-(Piperazino) äthyl]-carbamyl, N-[2-(4-Methylpiperazino) äthyl]-carbamyl oder im Phenylteil gegebenenfalls durch Niederalkyl, Niederalkoxy, Halogen und/oder Trifluormethyl substituiertes N-[2-(4-Phenylpiperazino) äthyl]-carbamyl.

N-[N',N'-(Oxa) niederalkylenaminoniederalkyl]-carbamyl ist beispielsweise N-[2-(Morpholino) äthyl]-carbamyl.

N-[N,N'-(Thia) niederalkylenaminoniederalkyl]-carbamyl bedeutet z. B. N-[2-(Thiomorpholino) äthyl]-carbamyl.

Als Salze von Verbindungen der Formel I kommen vorzugsweise pharmazeutisch verwendbare Salze in Frage, wie Metallsalze, Ammoniumsalze oder Salze mit organischen Basen. Metallsalze sind beispielsweise entsprechende Alkali-, Erdalkalimetallsalze, z. B. Lithium-, Natrium-, Kalium-, Magnesium- oder Calciumsalze, ferner pharmazeutisch verwendbare Uebergangsmetall-, wie Zink- oder Kupfersalze. Salze mit organischen Basen werden beispielsweise von Verbindungen der Formel I, worin $R_6$ und/oder $R_7$ Carboxy und/oder $R_8$ Carboxy oder 5-Tetrazolyl ist, mit mono-, di- oder trisubstituierten organischen Aminen, wie entsprechenden Alkylaminen, Hydroxyalkylaminen, geeigneten, mindestens ein N-Atom aufweisenden Heterocyclen, wie Morpholin, Thiomorpholin, Piperidin oder Pyrrolidin, gegebenenfalls N-substituierten Amino-sacchariden, z. B. mit N-Methyl-D-glucamin oder basischen Aminosäuren, wie Lysin, Arginin, Histidin oder Ornithin, wobei jene mit L-Konfiguration bevorzugt sind, gebildet. Als Alkylamine kommen z. B. Mono-, Di- oder Triniederalkylamine, wie Aethyl-, tert-Butyl-, Diäthyl-, Diisopropyl-, Trimethyl- oder Triäthylamin, in Betracht. Hydroxyalkylamine sind beispielsweise Mono-, Di- oder Trihydroxyalkylamine, wie Mono-, Di- bzw. Triäthanolamin oder Diisopropanolamin, oder Hydroxyniederalkyl-niederalkylamine, wie N,N-Dimethyl- bzw. N,N-Diäthylamino-äthanol oder Tri-(hydroxymethyl) methylamin.

Als weitere Salze sind pharmazeutisch verwendbare Säureadditionssalze, wie Hydrohalogenide, Methansulfonate, N-Cyclohexylsulfaminate, Maleinate, Fumarate, Maleate oder Tartrate von Verbindungen der Formel I, worin der Rest $R_8$ zur Bildung entsprechender Salze befähigt ist, zu nennen.

Die Verbindungen der Formel I mit chiralen C-Atomen können je nach der Anzahl derselben in zueinander enantiomeren bzw. diastereomeren Formen oder als Gemische derselben, wie Diastereomerengemische, Racemate oder Racematgemische vorliegen.

Die neuen Verbindungen zeichnen sich durch wertvolle pharmakologische Eigenschaften aus.

So weisen sie auf einem ausgeprägten $LTD_4$-(Leucotrien-$D_4$)- sowie PAF-(PAF-Acetäther)-Antagonismus beruhende antiallergische Wirkung auf. Die $LTD_4$-antagonistischen Eigenschaften der erfindungsgemässen Verbindungen können beispielsweise in vitro anhand ihrer in Konzentrationen ab etwa 0,03 bis etwa 0,10 µMol/l nachweisbaren Hemmwirkung auf durch $LTD_4$ ausgelöste Kontraktionen des isolierten Meerschweinchen-Ileums und in vivo anhand ihrer bei intravenöser Behandlung in Dosen ab etwa 0,08 mg/kg bzw. bei Aerosolbehandlung ab einem Wirkstoffgehalt von etwa 0,025 Gew.-% nachweisbaren Hemmwirkung auf durch $LTD_4$ ausgelöste Bronchospasmen des Meerschweinchens gezeigt werden.

Antiallergisch wirksame Verbindungen ähnlicher Struktur sind bereits bekannt. So sind in der US-Patentschrift Nr. 4,448,729 Verbindungen der Formel I, worin $R_1$ sowie $R_2$ $C_1$-$C_6$-Alkyl, $R_5$ eine Gruppe der Formel $—NH—C(=O)—R_8$, in der $R_8$ Carboxy oder $C_1$-$C_6$-Alkoxycarbonyl darstellt, $R_3$, $R_4$ und $R_6$ Wasserstoff, $R_7$ Wasserstoff, Cyano, Carboxy oder $C_1$-$C_6$-Alkoxycarbonyl und alk eine Gruppe der Formel $—(CH_2)_m$(Ia), in der m eine Zahl von 2 bis 6 ist, bzw. eine Gruppe der Formel $—(CH_2)_n—CH_2—CH(OH)—CH_2—(CH_2)_n$, in der einer der Indizes n 0, 1, 2, 3 oder 4 und der andere 0 oder 1 bedeutet, darstellen, als Antiallergika vorgeschlagen werden. Zudem sind die durch die vorstehende Ausnahmebestimmung vom Schutzumfang ausgeschlossenen Verbindungen der Formel I in der nicht vorpublizierten EP-A-147973 beschrieben.

Die erfindungsgemässen Verbindungen zeichnen sich den in der genannten US-Patentschrift konkret beschriebenen Verbindungen, die statt der Gruppe alk der Formel I eine Gruppe der Formel $—(CH_2)_n—CH_2—CH(OH)—CH_2—(CH_2)_n—$ aufweisen, gegenüber durch längere Wirkungsdauer aus und weisen zusätzlich zu der erwähnten $LTD_4$-antagonistischen Wirkung für diese Verbindungsklasse neuartige Phospholipase-hemmende Eigenschaften und eine ausgeprägte anti-inflammatorische bzw. hautphlogistatische Wirkung auf, die selbst schon sehr wertvoll ist und zudem die antiallergische

Wirkung in wünschenswerter Weise ergänzt. Die Phospholipase-hemmenden Eigenschaften können beispielsweise in vitro anhand der in Konzentrationen ab etwa 10 µMol/l nachweisbaren Hemmung der Aktivität von Phospholipasen $A_2$ (aus menschlichen Leukozyten) und C (aus menschlichen Thrombozyten) und die antiinflammatorischen bzw. hautphlogistatischen Eigenschaften in vivo anhand der Hemmwirkung auf das experimentelle Crotonöl-Ohroedem der Ratte in Konzentrationen ab etwa 10 mg/ml gezeigt werden.

Die erfindungsgemässen Verbindungen können dementsprechend als Antiallergika, beispielsweise zur Behandlung von Asthma, Heufieber, Rhinitis und Hautallergien, insbesondere aber als Antiinflammatorika, vor allem zur Behandlung entzündlicher Erkrankungen des rheumatischen Formenkreises, sowie als Haut- bzw. Schleimhautphlogistatika, zur Behandlung entzündlicher Dermatosen verschiedenartiger, insbesondere jedoch allergischer, Genese, beispielsweise zur Behandlung entzündlicher Hautirritationen, Kontaktdermatiden, Exanthemen, Verbrennungen und von Mukosaentzündungen der Augen, Lippen, des Mundes sowie der Genital-bzw. Analregion eingesetzt werden.

Die Hemmwirkung auf das experimentelle Ohroedem der Ratte kann nach G. Tonelli und L. Thibault, Endocrinology 77, 625 (1965) erfolgen. In diesem Modell wurden z. B. für N-{3-[3-(4-Acetyl-3-hydroxy-2-n-propyl-phenoxy)-propyloxy]-2-cyano-phenyl}-1H-tetrazol-5-carboxamid eine Hemmkonzentration $IC_{50}$ von 15 mg/ml ermittelt. Zur Ermittlung der übrigen der erwähnten Eigenschaften können z. B. folgende Versuchsanordnungen herangezogen werden.

Hemmwirkung auf $LTD_4$-induzierte Kontraktionen des Meerschweinchen-Ileums

An Ileumsegmenten, die aus Meerschweinchen von 300 bis 400 g Körpergewicht entnommen, in einem Organbad in Tyrodelösung (38 °C, Begasung mit 95 %$O_2$ und 5 % $CO_2$) befestigt und mit Ip belastet wurden, werden durch synthetisches $LTD_4$ (Leukotrien $D_4$, Kaliumsalz) Kontraktionen ausgelöst, deren Ausmass registriert wird. Das Ausmass der auf die $LTD_4$-antagonistische Wirkung der Testsubstanz zurückführende Hemmung dieser Kontraktionen wird gemessen. Man ermittelt diejenige, als $IC_{50}$ bezeichnete Konzentration der Testsubstanz, die durch $LTD_4$ induzierte Kontraktionen auf 50 % des Ausgangwertes reduziert. in dieser Versuchsanordnung wurde beispielsweise für das Triäthanolammoniumsalz der N-{3-[3-(4-Acetyl-3-hydroxy-2-propylphenoxy)-propyloxy]-6-methyl-phenyl}-oxaminsäure ein $IC_{50}$-Wert von 0,06 µMol/l und das Triäthanolammoniumsalz der N-{3-[3-(4-Acetyl-3-hydroxy-2-propyl-phenoxy)-propyloxy]-4-brom-6-methyl-phenyl}-1H-tetrazol-5-carboxamid ein $IC_{50}$-Wert von 0,03 µMol/l erhalten.

Meerschweinchen-Bronchokonstriktionstest (in vivo, intravenös)

Man anästhesiert männliche, 400-700 g schwere Meerschweinchen intraperitoneal mit 1,4 g/kg Urethan und führt eine Polyäthylenkanüle in die Vene jugularis ein, durch welche Wirkstoffe intravenös verabreicht werden können. Eine zweite Polyäthylenkanüle wird in die Trachea eingeführt. Mittels einer in die Speiseröhre eingeführten Kanüle, welche mit einem Statham-Druck-Transduktor verbunden ist, wird der Druck in der Speiseröhre aufgezeichnet. Das Tier wird in eine luftdicht verschliessbare Plexiglaskammer gelegt, die mit einer Fleisch'schen Röhre Nr. 000 und einem Validyne-Transducer MP 45-1 verbunden ist. Mit dieser Anordnung wird der Flow gemessen.

Nach der chirurgischen Präparierung der Versuchstiere wartet man eine gewisse Zeit, damit sich die pulmonalen Funktionen stabilisieren können. Die zu testende Verbindung wird anschliessend gemäss dem nachfolgenden Protokoll verabreicht.

Zu Beginn des Versuchs wird den Versuchstieren $LTD_4$ (300 ng/kg i.v.) verabreicht. 10 Minuten später wird zum zweiten Mal $LTD_4$ (300 ng/kg i.v.) verabreicht. Weitere 10 Minuten später wird die Testsubstanz i.v. 1 Minute vor der dritten Application von $LTD_4$ (300 ng/kg i.v.) injiziert.

Es wird die Abnahme der Compliance der 2. $LTD_4$-Applikation (Kontrolle) mit der Abnahme der Compliance der 3. $LTD_4$-Applikation verglichen. Mittels folgender Formel wird die Hemmung der Compliance in der ersten Minute nach $LTD_4$ aus dem Mittelwert von drei Tieren berechnet :

$$\% \text{ Hemmung} = 100 - \frac{(100 - \text{Compliance Präparat}) \cdot 100}{(100 - \text{Compliance Kontrolle})}$$

Werden unterschiedliche Wirkstoffdosen untersucht, so wird die prozentuale Hemmung für jede Individualdosis aufgezeichnet, und zwar wird der log der Dosis auf der Abszisse gegen die prozentuale Hemmung auf der Ordinate aufgetragen. Die $IC_{50}$ wird dann durch lineare Regressionsanalyse ermittelt.

Meerschweinchen-Bronchokonstriktionstest (in vivo, Aerosol) :

Man anästhetisiert männliche, 400-700 g schwere Meerschweinchen intraperitoneal mit 1,4 g/kg Urethan und führt eine Polyäthylenkanüle in die Vena jugularis ein. Eine zweite Polyäthylenkanüle wird in die Trachea eingeführt. Mittels einer in die Speiseröhre eingeführten Kanüle, welche mit einem Statham-Druck-Transduktor verbunden ist, wird der Druck in der Speiseröhre aufgezeichnet. Das Tier wird in eine

luftdicht verschliessbare Plexiglaskammer gelegt, die mit einer Fleisch'schen Röhre Nr. 000 und einem Validyne-Transducer MP 45-1 verbunden ist. Mit dieser Anordnung wird der Flow gemessen.

Nach der chirurgischen Präparierung der Versuchstiere wartet man eine gewisse Zeit, damit die pulmonalen Funktionen sich stabilisieren können. Die zu testende Verbindung wird anschliessend gemäss dem nachfolgenden Protokoll verabreicht. Die Versuchstiere werden während einer Minute einer 1-prozentigen Aerosollösung der zu testenden Verbindung (Gew/Vol) oder destilliertem Wasser (zu Kontrollzwecken) ausgesetzt. Für alle Testverbindungen, welche durch Inhalation verabreicht werden, verwendet man ein Monaghan-Ultraschall-Sprühgerät (Modell 670) dessen Partikelgrösse sich zwischen 1 und 8 Mikron bewegt mit einem Hauptanteil von 3 Mikron.

Wässrige Lösungen werden jeweils frisch hergestellt und mit einem On-stream drug vial in die Kammer des Sprühgeräts eingeführt. Der produzierte Sprühnebel wird den Versuchstieren über eine Glaskammer von 65 ml Inhalt, die mit einer Kanüle mit der Trachea verbunden wird, verabreicht. Nach Ablauf der Behandlungszeit wird mit einem zweiten Monaghan-Ultraschall-Sprühgerät (Modell 670) und über eine gleiche Glaskammer $LTD_4$ (0,3 μg/ml) während 2 Minuten verabreicht.

Es wird die Abnahme der Compliance in der 3. Minute nach $LTD_4$-Applikation abgelesen und zwar wird der Mittelwert von drei Tieren mit dem Mittelwert von drei Kontrolltieren verglichen und die prozentuale Hemmung der Compliance nach folgender Formel errechnet :

$$\% \text{ Hemmung} = 100 - \frac{(100 - \text{Compliance Präparat}) \cdot 100}{(100 - \text{Compliance Kontrolle})}$$

Werden unterschiedliche Wirkstoffkonzentrationen untersucht, so wird die prozentuale Hemmung für jede Konzentration aufgezeichnet, und zwar wird der log Konzentration auf der Abszisse gegen die prozentuale Hemmung auf der Ordinate aufgetragen. Die $IC_{50}$ wird dann durch lineare Regressionsanalyse ermittelt.

In der letztgenannten Versuchsanordnung wurde beispielsweise für das Triäthanolammoniumsalz der N-{3-[3-(4-Acetyl-3-hydroxy-2-n-propylphenoxy)-propyloxy]-6-methyl-phenyl}-oxaminsäure ein $IC_{50}$-Wert von 0,024 % der Aerosollösung und für das Triäthanolammoniumsalz der N-{3-[3-(4-Acetyl-3-hydroxy-2-n-propyl-phenoxy)-propyloxy]-4-brom-6-methyl-phenyl}-oxaminsäure ein $IC_{50}$-Wert von 0,033 % der Aerosollösung ermittelt.

In vitro-Test zur Bestimmung der Hemmung von Phospholipase $A_2$ aus menschlichen Leukozyten

Neutrophile polymorphkernige menschliche Leukozyten werden ausgehend von « buffy coats » durch mehrstufige fraktionierte Sedimentation isoliert und tiefgefroren. Die Phospholipase $A_2$ wird aus der Zellsuspension durch Homogenisieren unter Zusatz von eiskalter 0.36 n-$H_2SO_4$ in 2n-NaCl extrahiert und der nach Zentrifugieren bei 10'000 g erhaltene Ueberstand gegen Natriumacetatpuffer pH 4.5 dialysiert.

Für die Bestimmung der Enzymaktivität wird Enzym (10-30 μg Protein) in 0,1 M Tris/HCl-Puffer pH 7 unter Zusatz von 1 mM $CaCl_2$ und Substrat, bestehend aus biosynthetisch mit $^{14}C$-Oelsäure radioaktiv markiertem Phospholipiden (2 μM) von Eschoricha coli bei 37° während 1 Stunde inkubiert. Die Reaktion wird abgestoppt durch Zugabe von Dole-Reagens (Isopropanol/Heptan/IN $H_2SO_4$ 40 : 10 : 1, v/v) und die durch Phospholipase $A_2$ selektiv freigesetzte $^{14}C$-Oelsäure extrahiert. Ebenfalls mitextrahiertes Substrat wird durch Filtration des Extraktes durch eine Säule von Kieselgel vollständig entfernt. Die Bestimmung der $^{14}C$-Oelsäure im Eluat erfolgt durch Radiometrie.

Zur Ermittlung einer Hemmwirkung von Prüfsubstanzen auf die Phospholipase $A_2$ werden diese als Lösungen in Wasser, Dimethylsulfoxid (Endkonzentration im Ansatz bis 5 Vol-%) oder Aethanol (Endkonzentration im Ansatz bis 2.5 Vol-%) dem Inkubationsansatz zugesetzt. Die Wirkungsstärke der Prüfsubstanzen wird ausgedrückt durch die $IC_{50}$, d. h. die Konzentration welche eine Hemmung von 50 % der Kontrollaktivität bewirkt. Die $IC_{50}$ wird graphisch ermittelt durch Auftragen der prozentualen Hemmung auf der Ordinate gegen den Logarithmus der Konzentration (μM) auf der Abszisse.

In diesem Modell wurden folgende Werte für die $IC_{50}$ in μMol/l erhalten :

N-{3-[3-(4-Acetyl-3-hydroxy-2-n-propyl)-propyloxy-phenoxy]-2-cyanophenyl}-oxaminsäure, Triäthanolammoniumsalz : 30 ;

N-{3-[3-(4-Acetyl-3-hydroxy-2-n-propyl)-propyloxy-phenoxy]-phenyl}-oxaminsäure, Triäthanolammoniumsalz : 23 ;

N-{3-[3-(4-Acetyl-3-hydroxy-2-n-propyl)-propyloxy-phenoxy]-4-brom-6-methyl-phenyl}-oxaminsäure, Triäthanolammoniumsalz : 15 ;

N-{3-[3-(4-Acetyl-3-hydroxy-2-n-propyl-phenoxy)-propyloxy]-4-brom-6-methyl-phenyl}-1H-1,2,4-tetrazol-5-carboxamid, Triäthanolammoniumsalz : 8 ;

N-{3-[3-(4-Acetyl-3-hydroxy-2-n-propyl-phenoxy)-propyloxy]-2-cyanophenyl}-1H-tetrazol-5-carboxamid, Triäthanolammoniumsalz : 20 ;

N-{3-[3-(4-Acetyl-3-hydroxy-2-n-propyl-phenoxy)-2-hydroxypropyloxy]-2-cyano-phenyl}-oxaminsäure, Natriumsalz : unwirksam bei 100 ;

N-{3-[3-(4-Acetyl-3-hydroxy-2-n-propyl-phenoxy)-2-hydroxy-propyloxy]-phenyl}-oxaminsäure, Natriumsalz : unwirksam bei 100.

Unter den beschriebenen Versuchsbedingungen hemmt Mepacrin die Phospholipase $A_2$ mit einer $IC_{50}$ von 1600 μMol/l.

In vitro-Test zur Bestimmung der Hemmung der Phospholipase C aus menschlichen Thrombozyten

Menschliche Thrombozyten werden aus « buffy coats » durch fraktionierte Zentrifugation gewonnen und änschliessend tiefgefroren. Die Phospholipase C wird durch Ultraschallbehandlung der Zellsuspension freigesetzt und findet sich nach Ultrazentrifugieren (150'000 g, 1 Stunde) in löslicher Form im Ueberstand.

Für die Bestimmung der Enzymaktivität wird Enzym (20-100 μg Protein) in 0,025 m-Tris/Maleat-Puffer pH 6 unter Zusatz von 0.2 mM $CaCl_2$ und 0,02 mM radioaktiv markiertem Substrat, Phosphatidyl-[$^{14}C$] inosit, bei 37° während 5 Minuten inkubiert. Die Reaktion wird abgestoppt durch Ausschütteln mit $CHCl_3/CH_3OH$ 2 : 1 (v/v). Dabei wird unverbrauchtes Substrat in die organische Phase extrahiert, während das Reaktionsprodukt $^{14}C$-Inositphosphat in der wässrigen Phase verbleibt und durch Radiometrie eines Aliquots gemessen werden kann.

Zur Ermittlung einer Hemmwirkung von Prüfsubstanzen auf die Phospholipase C werden diese als Lösungen in Wasser, Dimethylsulfoxid (Endkonzentration im Ansatz bis 5 Vol-%) dem Inkubationsansatz zugesetzt. Die Wirkungsstärke der Prüfsubstanzen wird ausgedrückt durch die $IC_{50}$, d. h. die Konzentration welche eine Hemmung von 50 % die Kontrollaktivität bewirkt. Die $IC_{50}$ wird graphisch ermittelt durch Auftragen der prozentualen Hemmung auf der Ordinate gegen den Logarithmus der Konzentration (μM) auf der Abszisse.

In diesem Modell wurden die folgenden Werte für die $IC_{50}$ in μMol/l erhalten :

N-{3-[3-(4-Acetyl-3-hydroxy-2-n-propyl-phenoxy)-propyloxy]-2-cyanophenyl}-1H-tetrazol-5-carboxamid, Triäthanolammoniumsalz : 14 ;

N-{3-[3-(4-Acetyl-3-hydroxy-2-n-propyl-phenoxy)-2-hydroxy-propyloxy]-2-cyano-phenyl}-oxaminsäure, Natriumsalz : unwirksam bei 100.

Unter den beschriebenen Versuchsbedingungen hemmt Mepacrin die Phospholipase C mit einer $IC_{50}$ von 20 μM.

Die Erfindung betrifft vor allem Verbindungen der Formeln

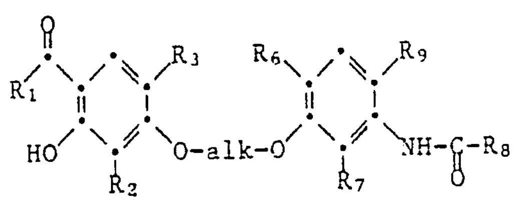

(I'),

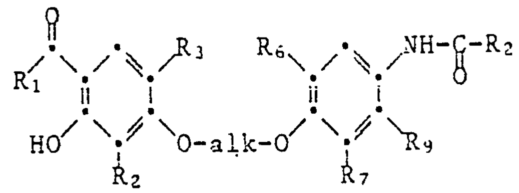

(I''),

worin $R_1$ für Niederalkyl mit bis und mit 4 C-Atomen, wie Methyl, steht, $R_8$ einerseits Carboxy, Niederalkoxycarbonyl mit bis und mit 4 C-Atomen, wie Methoxy- oder Aethoxycarbonyl, Diniederalkylaminoniederalkoxycarbonyl mit jeweils bis und mit 4 C-Atomen im Diniederalkylamino- und Niederalkoxyteil, wie 2-(Dimethylamino) äthoxycarbonyl, Carbamyl, N-Mono- oder N,N-Diniederalkylcarbamyl, worin Niederalkyl bis und mit 4 C-Atome aufweist, wie N-Methyl-, N-Aethyl- oder N,N-Dimethylcarbamyl, N-(N',N'-Diniederalkylaminoniederalkyl)-carbamoyl mit jeweils bis und mit 4 C-Atomen im Diniederalkylamino- und Niederalkylteil, wie N-[(2-Dimethylamino) äthyl]-carbamyl, oder andererseits 5-Tetrazolyl bedeutet, $R_2$ Niederalkyl mit bis und mit 4 C-Atomen, wie Propyl, darstellt, $R_3$ für Wasserstoff steht, $R_9$ für Wasserstoff, Niederalkyl mit bis und mit 4 C-Atomen, wie Methyl, Halogen der Atomnummer 17 bis und mit 53, wie Chlor, Brom oder Jod oder Trifluormethyl steht, $R_7$ Wasserstoff, Niederalkyl mit bis und mit 4 C-Atomen, wie Methyl, Niederalkoxy mit bis und mit 4 C-Atomen, wie Methoxy, oder Halogen der Atomnummer bis und mit 355, wie Chlor oder Brom, darstellt und $R_6$ Wasserstoff, Niederalkyl mit bis und mit 4 C-Atomen, wie Methyl, Halogen der Atomnummer bis und mit 35, wie Chlor oder Brom, Niederalkoxycarbonyl mit bis und mit 5 C-Atomen, wie Aethoxycarbonyl, Carboxy oder Niederalkanoyl mit bis und mit 7 C-Atomen, wie Formyl, Acetyl oder Pivaloyl, bedeutet und alk für, insbesondere geradkettiges, terminal gebundenes Niederalkylen mit bis und mit 4 C-Atomen, wie 1,3-Propylen, steht, und ihre Salze, insbesondere pharmazeutisch verwendbaren Salze.

Die Erfindung betrifft in erster Linie Verbindungen der Formel I, worin $R_1$ Niederalkyl mit bis und mit 4 C-Atomen, wie Methyl, bedeutet, $R_2$ geradkettiges Niederalkyl mit bis und mit 4 C-Atomen, wie Propyl,

bedeutet, $R_3$ und $R_7$ für Wasserstoff stehen, einer der Reste $R_4$ und $R_6$ Niederalkyl mit bis und mit 4 C-Atomen, wie Methyl, und der andere Halogen der Atomnummer bis und mit 35, wie Fluor, Chlor oder Brom, bedeutet, $R_5$ Oxaloamino oder 5-Tetrazolylcarbonylamino ist und alk geradkettiges, terminal gebundenes Niederalkylen mit bis und mit 7 C-Atomen, wie 1,3-Propylen, darstellt, und ihre Salze, insbesondere pharmazeutisch verwendbare Salze, mit Basen.

Die Erfindung betrifft insbesondere Verbindungen der Formel I, worin $R_1$ Niederalkyl mit bis und mit 4 C-Atomen, wie Methyl, bedeutet, $R_2$ geradkettiges Niederalkyl mit 2 bis und mit 4 C-Atomen, wie Propyl, bedeutet, $R_3$ Wasserstoff bedeutet und $R_5$ Oxaloamino oder 5-Tetrazolylcarbonylamino darstellt, und worin entweder $R_4$ für Wasserstoff steht und $R_6$ und $R_7$ unabhängig voneinander Wasserstoff oder Niederalkyl mit bis und mit 4 C-Atomen bedeuten bzw. $R_4$ für Niederalkyl mit bis und mit 4 C-Atomen, wie Methyl, oder Trifluormethyl steht, $R_6$ Wasserstoff oder Halogen der Atomnummer bis und mit 35, wie Chlor oder Brom, darstellt und $R_7$ Wasserstoff ist bzw. $R_4$, $R_6$ und $R_7$ gleiche oder verschiedene Halogenatome der Atomnummer bis und mit 35, wie Chlor oder Brom, bedeuten, und alk geradkettiges, terminal gebundenes Niederalkylen mit 2 bis und mit 4 C-Atomen, wie 1,3-Propylen, darstellt, und ihre Salze, insbesondere pharmazeutisch verwendbaren Salze mit Basen.

Die Erfindung betrifft namentlich die in den Beispielen genannten Verbindungen der Formel I und ihre Salze, insbesondere pharmazeutisch verwendbaren Salze, mit Basen.

Die Erfindung betrifft ferner ein auf an sich bekannten Methoden beruhendes Verfahren zur Herstellung von Verbindungen der Formel I und ihre Salze. Dieses ist dadurch gekennzeichnet, dass man

a) in einer Verbindung der Formel

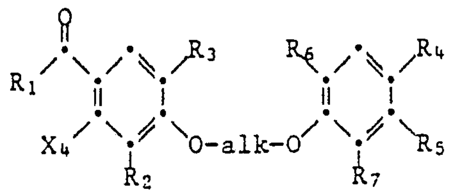

(VI)

worin $X_4$ einen in Hydroxy überführbaren Rest bedeutet, $X_4$ in Hydroxy überführt oder

b) Verbindungen der Formeln

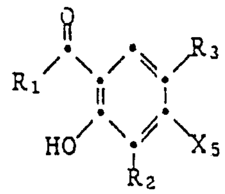

(VII) und

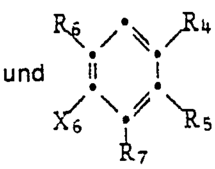

(VIII)

miteinander umsetzt, worin einer der Reste $X_5$ und $X_6$ gegebenenfalls in Salzform vorliegendes Hydroxy und der andere einen durch reaktionsfähiges verestertes Hydroxy substituierten Rest —O-alkH, d. h. einen durch reaktionsfähiges verestertes Hydroxy substituierten Alkoxyrest, darstellt, oder

c) eine Verbindung der Formel

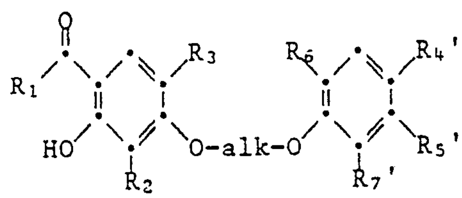

(X)

worin einer der Reste $R_4'$ $R_5'$ und $R_7'$ die Aminogruppe, ein davon verschiedener Rest $R_4'$ bzw. $R_5'$ einen Rest $R_9$ und ein davon verschiedener Rest $R_7'$ einen Rest $R_{10}$ darstellt, oder ein Salz davon mit einer Verbindung der Formel

$$X_7—R_8' \quad (XI)$$

umsetzt, worin $R_8'$ eine gegebenenfalls veresterte oder amidierte Carboxygruppe oder gegebenenfalls in 1-Stellung geschütztes 5-Tetrazolyl und $X_7$ eine gegebenenfalls veresterte, amidierte, anhydridisierte oder, sofern $R_8'$ für in 1-Stellung geschütztes 5-Tetrazolyl steht, in Salzform vorliegende Carboxygruppe bedeutet, und gegebenenfalls die Schutzgruppe in 1-Stellung einer Tetrazolylgruppe $R_8$ abspaltet oder

d) in einer Verbindung der Formel

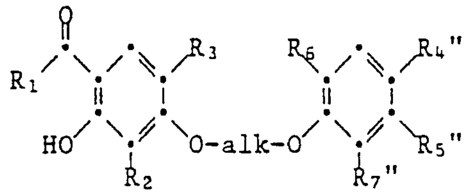

(XII)

worin einer der Reste $R_4''$, $R_5''$ und $R_7''$ einen Rest $X_8$, ein davon verschiedener Rest $R_4''$ bzw. $R_5''$ einen Rest $R_9$ und ein davon verschiedener Rest $R_7''$ einen Rest $R_{10}$ darstellt und $X_8$ einen in die gewünschte Gruppe der Formel —NH—C(=O)—$R_8$ überführbaren Rest bedeutet, $X_8$ in diese überführt und gewünschtenfalls jeweils eine verfahrensgemäss erhältliche Verbindung in eine andere Verbindung der Formel I überführt, ein verfahrensgemäss erhältliches Isomerengemisch auftrennt und das oder die gewünschte(n) Isomere(n) isoliert und/oder eine verfahrensgemäss erhältliche freie Verbindung in ein Salz oder ein verfahrensgemäss erhältliches Salz in die freie Verbindung oder in ein anderes Salz umwandelt.

In Hydroxy überführbare Reste $X_4$ in Formel VI sind beispielsweise verätherte oder veresterte Hydroxygruppen. Als veräthertes Hydroxy $X_4$ komment beispielsweise aliphatisch veräthertes Hydroxy, z. B. Niederalkoxy, wie Methoxy, oder Niederalkenyloxy, insbesondere Niederalk-2-enyloxy, z. B. Allyloxy, Phenylniederalkoxy, insbesondere gegebenenfalls substituiertes Phenylniederalkoxy, wie Benzyloxy, ferner Tetrahydropyran-2-yloxy oder Silyloxy, insbesondere Triniederalkylsilyloxy, z. B. Trimethylsilyloxy, in Betracht. Verestertes Hydroxy $X_4$ ist beispielsweise mit einer Carbonsäure, wie einer aliphatischen oder aromatischen Carbonsäure oder mit einem aliphatischen oder aromatischen Halbester der Kohlensäure verestertes Hydroxy, wie Niederalkanoyloxy, z. B. Acetoxy, gegebenenfalls substituiertes Benzoyloxy, z. B. der Formel $R_1$—C(=O)—O—, gegebenenfalls halogeniertes Niederalkoxycarbonyl, z. B. Methoxy-, Aethoxy- oder Tertiärbutyloxycarbonyl, 2,2,2-Trijodäthoxy- oder 2,2,2-Trichloräthoxycarbonyl, gegebenenfalls substituiertes Phenylniederalkoxycarbonyl, insbesondere 1-Phenylniederalkoxycarbonyl, z. B. Benzyloxycarbonyl, oder gegebenenfalls substituiertes Phenoxycarbonyl.

In Salzform vorliegendes Hydroxy $X_5$ in Formel VII bzw. $X_6$ in Formel VIII bzw. Carboxy in Formel XI liegt insbesondere in einer Alkalimetallsalzform, z. B. als Natrium- oder Kaliumsalz, vor.

Durch reaktionsfähiges verestertes Hydroxy substituierte Alkoxyreste $X_5$ in Formel VII bzw. $X_6$ in Formel VIII sind bespielsweise reaktionsfähige veresterte Hydroxyalkoxyreste, insbesondere der Formel $—O—(CH_2)_m—X$, worin X reaktionsfähiges verestertes.

Hydroxy und m eine ganze Zahl von 2 bis und mit 9 ist. Reaktionsfähiges verestertes Hydroxy ist dabei beispielsweise Halogen, wie Chlor, Brom oder Jod, oder organisches Sulfonyloxy, wie Niederalkansulfonyloxy, z. B. Methansulfonyloxy, oder gegebenenfalls substituiertes Benzolsulfonloxy, z. B. Benzol-, p-Brombenzol- oder p-Toluolsulfonyloxy.

Gegebenenfalls verestertes, amidiertes oder anhydridisiertes Carboxy $X_7$ in Formel XI ist beispielsweise freies Carboxy, verestertes Carboxy $R_8$ oder mit einem gegebenenfalls substituierten Phenol verestertes Carboxy, wie Phenoxy-, 4-Nitrophenoxy- oder 2,4-Dinitrophenoxycarbonyl, amidiertes Carboxy $R_8$ oder aktiviertes Carbamyl, wie 1-Imidazolyl- bzw. 1-(2,5-Dimethylimidazolyl)-carbonyl, oder mit einer Halogenwasserstoffsäure anhydridisiertes Carboxy, wie Halogencarbonyl, z. B. der Formel Hal—C(=O)—, worin Hal Chlor, Brom oder Jod, vor allem Chlor, ist.

Als Ausgangsstoffe XI kommen insbesondere solche der Formeln $R_8''$—$R_8''$ (XIa), worin $R_8''$ gegebenenfalls verestertes oder amidiertes Carboxy bedeutet, oder Hal—C(=O)—$R_8''$ (XIb') in Betracht. In geschützter Form vorliegende 5-Tetrazolylreste $R_8'$ sind beispielsweise gegebenenfalls im Arylteil substituierte 1-(α-Aralkyl)-tetrazolyl-(5)-reste, wie 1-Benzyltetrazolyl-(5) oder 1-(p-Methoxybenzyl)-tetrazolyl-(5).

Ein in die Gruppe der Formel —NH—C(=O)—$R_8$ überführbarer Rest $X_8$ in Formel XII ist beispielsweise ein durch Solvolyse, d. h. Hydrolyse, Alkoholyse (Umsetzung mit dem der gewünschten veresterten Carboxygruppe $R_8$ entsprechenden Alkohol) und/oder Aminolyse (Umsetzung mit Ammoniak oder einem der gewünschten amidierten Carboxygruppe $R_8$ entsprechenden Amin) in diese überführbarer Rest, beispielsweise eine Gruppe der Formel —NH—$X_A$, in der $X_A$ eine von einer gegebenenfalls veresterten oder amidierten Oxalogruppe verschiedene und in diese überführbare funktionell abgewandelte Oxalogruppe bedeutet. Derartige funktionell abgewandelte Oxalogruppen sind vorzugsweise solche, die als funktionell abgewandelte α-Carbonylgruppierung Thioxomethylen, Iminomethylen oder eine veresterte und/oder verätherte Dihydroxymethylengruppierung und/oder als funktionell abgewandelte Carboxygruppe eine von einer veresterten oder amidierten Carboxygruppe verschiedene funktionell abgewandelte Carboxygruppe aufweisen. Veresterte und/oder verätherte Dihydroxymethylengruppierungen sind beispielsweise mit einer Halogenwasserstoffsäure, wie Chlorwasserstoffsäure, veresterte und/oder mit einem Niederalkanol, wie Methanol oder Aethanol verätherte Dihydroxymethylengruppen. Als Beispiele seien vor allem Dihalogenmethylengruppierungen, wie Dichlormethylen, Niederalkoxyhalogenmethylengruppierungen, wie Methoxy- oder Aethoxychlormethylen oder Diniederalkoxymethylengruppierungen, wie Dimethoxy- oder Diäthoxymethylen, genannt. Von veresterten oder amidierten Carboxylgruppen

verschiedene funktionell abgewandelte Carboxygruppierungen sind beispielsweise die Cyanogruppe, anhydridisierte Carboxygruppen, wie Halogen-, z. B. Chlorcarbonyl, Iminoester-, wie Imid- bzw. Aminhalogenidgruppierungen, z. B. Iminochlor- oder Aminodichlormethyl, Iminoäthergruppierungen, wie Niederalkyl- oder Niederalkyleniminoäthergruppierungen, z. B. Methoxy- oder Aethoxyiminomethylen, 4,4- oder 5,5-Dimethyloxazolinyl (2) oder 4,4,6-Trimethyl-dihydro-oxazinyl-(2), Amidinogruppen, wie Amidino- oder Niederalkyl-, z. B. Methylamidino, mit einer Halogenwasserstoffsäure, wie Chlorwasserstoffsäure, veresterte und/oder mit einem Niederalkanol verätherte Orthosäuregruppierungen, wie Triniederalkoxy-, Niederalkoxyhalogen- oder Trihalogenmethylgruppen, vor allem Trimethoxy- oder Triäthoxymethyl, Aethoxydichlormethyl oder Trichlormethyl, oder gegebenenfalls veresterte Thiocarboxylgruppen, wie Niederalkylthiocarbonylgruppen, z. B. Aethylthiocarbonyl.

Ein in die Gruppe der Formel —NH—C(=O)—$R_8$, worin $R_8$ 5-Tetrazolyl bedeutet, überführbarer Rest ist beispielsweise die Gruppe der Formel —NH—C(=O)—CN oder eine Gruppe der Formel —NH—C(=O)—$R_8'$, worin $R_8'$ in 1-Stellung geschütztes 5-Tetrazolyl ist. In geschützter Form vorliegende 5-Tetrazolylreste $R_8'$ sind beispielsweise gegebenenfalls im Arylteil substituierte 1-(α-Aralkyl)-tetrazolyl-(5)-reste, wie 1-Benzyltetrazolyl-(5) oder 1-(p-Methoxybenzyl)-tetrazolyl-(5).

Weitere in Gruppen der Formel —NH—C(=O)—$R_8$ überführbare Reste $X_8$ sind beispielsweise oxydativ in diese überführbare Gruppen der Formel —NH—$X_B$, worin $X_B$ die oxydativ in die Oxalogruppe der Formel —C(=O)—$R_8$ worin $R_8$ Carboxy darstellt, überführbare gegebenenfalls hydratisierte Glyoxylgruppe bedeutet. Diese kann vorteilhaft im Verlaufe der Oxydationsreaktion, z. B. aus der Acylgruppe einer gegebenenfalls α,β-ungesättigten oder α,β-dihydroxylierten aliphatischen oder araliphatischen Carbonsäure, einer gegebenenfalls an der Hydroxygruppe veresterten Glykoloylgruppe oder der Glycylgruppe, in situ gebildet oder aus einem ihrer funktionellen Derivate, z. B. einem ihrer Acetale oder Imine, in Freiheit gesetzt werden. Acylgruppen von gegebenenfalls α,β-ungesättigten oder α,β-dihydroxylierten Carbonsäuren sind beispielsweise Alkanoylgruppen, wie Niederalkanoyl, z. B. Acetyl, Acylgruppen von α,β-ungesättigten aliphatischen Mono- oder Dicarbonsäuren, z. B. Acryloyl, Crotonyl oder die Acylgruppe der gegebenenfalls funktionell abgewandelten Fumar- oder Maleinsäure, Acylgruppen von α,β-ungesättigten araliphatischen Carbonsäuren, z. B. gegebenenfalls substituiertes Cinnamoyl, oder Acylgruppen von aliphatischen, α,β-Dihydroxydicarbonsäuren, wie der Weinsäure, oder monofunktioneller Carboxyderivate, wie Estern oder Amiden, derselben. Veresterte Glykoloylgruppen sind beispielsweise an der Hydroxygruppe mit einer Mineralsäure, wie einer Halogenwasserstoffsäure, z. B. mit Chlor- oder Bromwasserstoffsäure, oder mit einer Carbonsäure, z. B. mit Essigsäure oder der gegebenenfalls substituierten Benzoesäure, veresterte Glykoloylgruppen. Acetalisierte Glyoxyloylgruppen sind beispielsweise mit Niederalkanolen oder einem Niederalkandiol acetalisierte Glyoxyloylgruppen, wie Dimethoxy-, Diäthoxy- oder Aethylendioxyacetyl. Imine von Glyoxyloylgruppen sind beispielsweise gegebenenfalls substituierte N-Benzylimine oder N-(2-Benzothiazolyl)-imine derselben oder Imine mit 3,4-Di-tert.-butyl-o-chinon. Weitere oxydativ in die Oxalogruppe überführbare Reste sind z. B. gegebenenfalls substituierte, wie in 5-Stellung eine acetalisierte Formylgruppe, wie Diäthoxymethyl, aufweisende 2-Furoylgruppen. Zu veresterten Oxalogruppen der Formel —C(=O)—$R_8$, worin $R_8$ für verestertes Carboxy steht, oxydierbare Gruppen sind verätherte Glykoloylgruppen, wie Niederalkoxyacetyl. Zu gegebenenfalls veresterten oder amidierten Oxaloaminogruppen oxydierbare Reste $X_B$ sind ferner gegebenenfalls hydratisierte oder acetalisierte Formylmethylaminogruppen oder gegebenenfalls funktionell abgewandelte Carboxymethylaminogruppen bzw. Carboxymethyleniminogruppen, z. B. der Formel —NH—$CH_2$—CH=O, —NH—$CH_2$—$R_8$ bzw. —N=CH—$R_8$.

Die Durchführung der verfahrensgemässen Reaktionen sowie die Herstellung neuen Ausgangsstoffe bzw. Zwischenprodukte erfolgt in Analogie zur Reaktions- und Bildungsweise bekannter Ausgangsstoffe bzw. Zwischenprodukte. Dabei werden, auch wenn nachstehend nicht ausdrücklich erwähnt, die jeweils üblichen Hilfsmittel, wie Katalysatoren, Kondensations- sowie Solvolysemittel und/oder Lösungs- bzw. Verdünnungsmittel, und Reaktions-, wie Temperatur- und Druckbedingungen, sowie gegebenenfalls Schutzgase verwendet.

Die Ueberführung von Gruppen $X_4$ in Hydroxy gemäss der Verfahrensvariante a) erfolgt in üblicher Weise, beispielsweise durch Behandeln mit einem komplexen Metallhalogenid der Formel $M^nYn$ (XIX), worin M ein n-wertiges, koordinativ ungesättigtes Metallkation der Gruppe IIa, IIb, IIIa, IIIb, IVb, Va oder VIIb des periodischen Systems der Elemente, z. B. Magnesium-, $Zink^{II}$-, $Bor^{III}$-, $Aluminium^{III}$-, $Gallium^{III}$-, $Zinn^{IV}$-, $Titan^{IV}$-, $Antimon^{V}$- oder $Eisen^{III}$- bzw. Eisen VI-ion, Y ein Halogenatom der Atomnummer bis und mit 35, wie Fluor oder Chlor bedeutet, z. B. Aluminiumtrichlorid oder mit einem tertiären organischen Ammoniumsalz, wie einem Pyridinium- oder Triniederalkylammoniumhalogenid, z. B. mit Pyridiniumchlorid oder -bromid oder Triäthylammoniumchlorid, kann aber auch durch Solvolyse, insbesondere durch Hydrolyse, erforderlichenfalls in Gegenwart eines, vorzugsweise sauren, Hydrolysemittels, erfolgen. Hydrolysemittel sind neben üblichen basischen Hydrolysemitteln, wie Alkalimetallhydroxiden, als saure Hydrolysemittel beispielsweise Mineralsäuren, z. B. Chlor-, Brom- oder Jodwasserstoffsäure, Schwefelsäure, Phosphorsäure oder Polyphosphorsäure, ebenso komplexe Metallsäuren, z. B. Hexachloroantimonsäure, Tetrafluorborsäure und dergleichen, im Falle von mit organischen Carbonsäuren veresterten Hydroxygruppen $X_4$ ferner Niederalkansäuren, wie Essigsäure. Lösungsmittel sind bei der Hydrolyse beispielsweise mit Wasser mischbare organische Lösungsmittel. Vorzugsweise arbeitet man jeweils in Gegenwart eines Lösungs- oder Verdünnungsmittels bzw. eines Lösungsvermittlers, unter Kühlen oder

Erwärmen, z. B. im Temperaturbereich von etwa 0 bis 120 °C, und/oder unter Inertgas.

So kann man verätherte Hydroxygruppen beispielsweise durch Behandeln mit wässriger Jodwasserstoffsäure, Pyridiniumhydrochlorid, z. B. in Methylenchlorid, mit Bromwasserstoffsäure in hochkonzentrierter, z. B. 98 %iger Essigsäure oder durch Behandeln mit Bortribromid oder Aluminiumtrichlorid zu Hydroxy spalten. In einer Abwandlung dieses Verfahrens kann man eine Verbindung VI, worin $X_4$ Niederalk-2-enyloxy bedeutet und $R_2$ Wasserstoff ist, durch Erwärmen auf etwa 150 bis 250 °C, vorzugsweise auf etwa 190 bis 220 °C, vorteilhaft in einem Lösungsmittel, wie Diphenyläther oder N,N-Dimethyl- oder N,N-Diäthylanilin, zu einer Verbindung I umlagern, worin $R_2$ Niederalk-2-enyl substituiert ist.

In als Gruppe $X_4$ eine gegebenenfalls substituierte α-Phenylniederalkoxygruppe oder eine andere übliche durch Reduktion spaltbare geschützte Hydroxygruppe aufweisenden Verbindungen VI kann die Hydroxygruppe vorteilhaft reduktiv freigesetzt werden. So kann man beispielsweise hydrieren, d. h. mit Wasserstoff in Gegenwart eines Hydrierungskatalysators, z. B. eines Palladium-, Platin-, Nickel- oder Rhodiumkatalysators, z. B. von Palladium auf Kohle oder von Raney-Nickel, reduzieren.

Ferner kann man ausgehend von Verbindungen VI, worin $X_4$ mit einer organischen Carbonsäure verestertes Hydroxy ist, die Hydroxygruppe durch Umesterung, d. h. durch Behandlung mit einem Alkohol, z. B. Niederalkanol, in Gegenwart eines sauren oder basischen Mittels, wie einer Mineralsäure, z. B. von Schwefelsäure oder eines Alkalimetallhydroxides oder -alkoholates, z. B. von Natriumhydroxid oder eines Natriumniederalkanolates, freisetzen.

Ausgangsstoffe VI werden beispielsweise hergestellt, indem man Verbindungen der Formeln

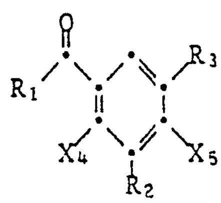 (XVIII) 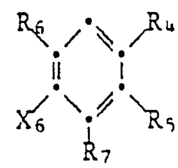 (VIII)

miteinander umsetzt, worin einer der Reste $X_5$ und $X_6$ gegebenenfalls in Salzform vorliegendes Hydroxy und der andere einen durch reaktionsfähiges verestertes Hydroxy substituierten Rest —O-alkH, z. B. eine Gruppe der Formeln $—O—(CH_2)_m—X$, in der X reaktionsfähiges verestertes Hydroxy, z. B. Halogen, bedeutet, darstellt.

Die Umsetzung von Verbindungen VII und VIII gemäss Verfahrensvariante b) erfolgt in üblicher Weise, beispielsweise in Gegenwart eines basischen Kondensationsmittels, wie eines Hydroxides oder Carbonates eines Alkali- oder Erdalkalimetalles, wie von Natrium- oder Kaliumhydroxid, Kaliumcarbonat oder Calciumcarbonat, vorteilhaft in einem Niederalkanol, z. B. Methanol oder Amylalkohol, Diniederalkylketon, z. B. in Aceton oder Diäthylketon, oder N,N-Diniederalkylniederalkansäureamid oder N-Niederalkylniederalkansäurelactam, z. B. in Dimethylformamid oder N-Methylpyrrolidinon.

Die Ausgangsstoffe VII werden beispielsweise hergestellt, indem man eine Verbindung der Formel

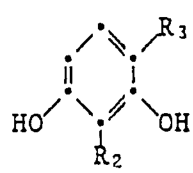 (XIII)

in Gegenwart einer Lewissäure, z. B. von Aluminiumtrichlorid oder Zinkchlorid, mit einer Verbindung der Formel $R_1—X_2$ [IV ; $X_2$ = Halogencarbonyl oder $—O—C(=O)—R_1$] umsetzt und gewünschtenfalls in der erhaltenen Verbindung VII, worin $X_5$ Hydroxy ist, die Hydroxygruppe in p-Stellung zu $R_1—C(=O)—$ durch Umsetzung mit einem Dihalogenalkan, Epoxyalkan oder Halogenalkanol in einen durch Halogen oder Hydroxy substituierten Alkoxyrest überführt und gegebenenfalls Hydroxyalkoxy reaktionsfähig verestert, z. B. durch Behandeln mit Thionylchlorid, Phosphortribromid oder einem Sulfonsäurechlorid.

Verbindungen VIII können biespielsweise erhalten werden, indem man in einer Verbindung der Formel

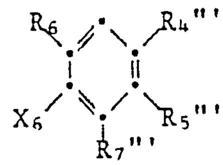 (XX)

worin einer der Reste $R_4'''$, $R_5'''$ und $R_7'''$ die Nitrogruppe, ein davon verschiedener Rest $R_4'''$ oder $R_5'''$

einen Rest $R_9$ und ein davon verschiedener Rest $R_7'''$ einen Rest $R_{10}$ darstellt, die Nitrogruppe zu Amino reduziert, z. B. mit Wasserstoff in Gegenwart von Raney-Nickel, und die Verbindung der Formel

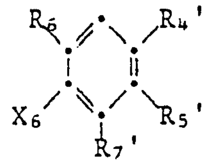
(XXI)

worin einer der Reste $R_4'$, $R_5'$ und $R_7'$ die Aminogruppe, ein davon verschiedener Rest $R_4'$ oder $R_5'$ eine Gruppe $R_9$ und ein davon verschiedener Rest $R_7'$ einen Rest $R_{10}$ bedeutet, in Gegenwart einer Base, z. B. von Triäthylamin oder Pyridin, mit einer Verbindung der Formel Hal—C(=O)—$R_8$ (XIb ; Hal = Halogen) umsetzt und gewünschtenfalls in der erhaltenen Verbindung VIII, worin $X_6$ Hydroxy ist, die Hydroxygruppe durch Umsetzung mit einem Dihalogenalkan, Epoxyalkan oder Halogenalkanol in einen durch Halogen oder Hydroxy substituierten Alkoxyrest überführt und gegebenenfalls Hydroxyalkoxy reaktionsfähig verestert, z. B. durch Behandeln mit Thionylchlorid, Phosphortriromid oder einem Sulfonsäurechlorid.

Die Umsetzung von Verbindungen X und XI gemäss Verfahrensvariante c) kann in üblicher, insbesondere in der aus der Literatur für analoge Umsetzungen, bekannten Weise erfolgen, erforderlichenfalls in Gegenwart eines Kondensationsmittels, bei der Umsetzung mit einem Esterhalogenid oder Amidhalogenid der Oxalsäure beispielsweis eines basischen Kondensationsmittels, wie einer tertiären organischen Stickstoffbase, z. B. von Triäthylamin oder Pyridin, oder eines Alkalimetallhydroxides oder -carbonates, z. B. von Natrium- oder Kaliumhydroxid, oder bei der Umsetzung mit Oxalsäure beispielsweise eines die Dehydratisierung des primär gebildeten Ammoniumsalzes bewirkenden Kondensationsmittels, wie eines wasserbindenden Mittels, z. B. von Dicyclohexylcarbodiimid oder eines Isonitrils, wie von tert.-Butylisonitril, oder einer Mineralsäure, z. B. von Chlorwasserstoffsäure, oder eines Säureanhydrids, z. B. von Phosphorpentoxid, jeweils in einem inerten Lösungsmittel, wie einem Halogenalkan, z. B. in Methylenchlorid, oder einem N,N-Dialkylamid, z. B. in N,N-Dimethylformamid oder -acetamid.

Die 1-Schutzgruppe von 5-Tetrazolylresten $R_8'$ kann anschliessend z. B. durch Acidolyse, d. h. Behandeln mit einer Säure, z. B. mit Trifluoressigsäure/Anisol, oder hydrogenolytisch, insbesondere mittels Wasserstoff und Palladium auf Kohle, abgespalten werden.

Ausgangsstoffe X können beispielsweise hergestellt werden, indem man Verbindungen der Formeln

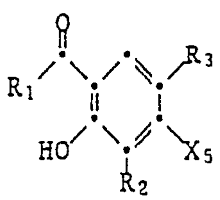
(VII) und

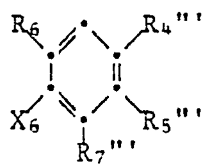
(XX)

worin einer der Reste $R_4'''$, $R_5'''$ und $R_7'''$ die Nitrogruppe, ein davon verschiedener Rest $R_4'''$ oder $R_5'''$ einen Rest $R_9$ und ein davon verschiedener Rest $R_7'''$ einen Rest $R_{10}$ darstellt, miteinander umsetzt, worin einer der Reste $X_5$ und $X_6$ gegebenenfalls in Salzform vorliegendes Hydroxy und der andere einen durch reaktionsfähiges verestertes Hydroxy substituierten Rest —O-alkH, z. B. eine Gruppe der Formeln —O—$(CH_2)_m$—X, in der X reaktionsfähiges verestertes Hydroxy, z. B. Halogen, bedeutet, darstellt, und in der erhaltenen Verbindung der Formel

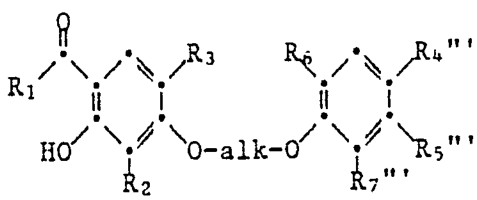

die Nitrogruppe zu Amino reduziert, beispielsweise durch Umsetzung mit Wasserstoff in Gegenwart eines Hydrierungskatalysators, wie von Palladium auf Kohle oder vor allem Raney-Nickel, beispielsweise in Tetrahydrofuran.

Die Ueberführung der Gruppe $X_8$ in Verbindungen XII in solche der Formel —NH—C(=O)—$R_8$ gemäss Verfahrensvariante d) erfolgt beispielsweise durch Solvolyse oder Oxidation oder ausgehend von Gruppen $X_8$ der Formel NH—C(=O)—CN durch Umsetzung mit Stickstoffwasserstoffsäure bzw. ausgehend von Gruppen $X_8$ der Formel —NHC(=O)—$R_8'$, worin $R_8'$ in 1-Stellung geschütztes 5-Tetrazolyl bedeutet, durch Abspaltung der Schutzgruppe. So können die genannten Gruppen $X_A$ in Resten $X_8$ der Formel —NH—$X_A$ hydrolytisch in die Oxalogruppe überführt werden. Als funktionell abgewandelte

Carboxygruppe eine Iminoäther, Orthoester- oder Esterhalogenidgruppierung und/oder als funktionell abgewandelte α-Carbonylgruppe Thioxo- oder Iminomethylen oder eine veresterte oder verätherte Dihydroxymethylengruppe aufweisende Gruppe $X_A$ können ferner zu veresterten Oxalogruppen —C(=O)—$R_8$ hydrolysiert werden. Ebenso können als funktionell abgewandelte Carboxygruppe die Cyanogruppe, eine Amidino- oder Imid- bzw. Amidhalogenidgruppierung und/oder als funktionell abgewandelte α-Carbonylgruppe Thioxo- oder Iminomethylen oder eine verätherte oder veresterte Dihydroxymethylengruppe aufweisende Gruppe $X_A$ zu amidierten Oxalogruppen —C(=O)—$R_8$ hydrolysiert werden. Die Hydrolyse kann in üblicher Weise durchgeführt werden, erforderlichenfalls in Gegenwart eines basischen oder vorzugsweise sauren Hydrolysemittels, wie eines Alkalimetallhydroxides, wie Natrium- oder Kaliumhydroxid, oder vorzugsweise einer Protonensäure, vorzugsweise einer Mineralsäure, z. B. einer Halogenwasserstoffsäure, wie Salzsäure oder einer organischen Carbon- oder Sulfonsäure, z. B. von Essigsäure oder p-Toluolsulfonsäure.

Als funktionell abgewandelte Carboxygruppe eine anhydridisierte Carboxygruppe, wie Halogencarbonyl, z. B. Chlorcarbonyl, Cyanocarbonyl oder eine Niederalkyleniminäthergruppierung, z. B. 4,4- oder 5,5-Dimethyl-oxazolinyl-(2), oder 4,4,6-Trimethyl-dihydrooxazinyl-(2), aufweisende funktionell abgewandelte Oxalogruppe $X_A$, können ferner durch übliche Alkoholyse, d. h. Umsetzung mit dem entsprechenden Alkohol, in veresterte Oxalogruppen —C(=O)—$R_8$ überführt werden. Bei der Alkoholyse von anhydridisierten Carboxygruppen arbeitet man vorteilhaft in Gegenwart eines basischen Kondensationsmittels, z. B. von Pyridin oder Triäthylamin, während man die Alkoholyse von Carboxy oder einer Niederalkyleniminoäthergruppierung vorzugsweise sauer, z. B. in Gegenwart von Chlorwasserstoffsäure, p-Toluolsulfonsäure oder Essigsäure, durchführt. In analoger Weise kann man eine anhydridisierte Carboxygruppe aufweisende funktionell abgewandelte Oxalogruppe auch durch Ammono- bzw. Aminolyse, d. h. Umsetzung mit Ammoniak oder eines entsprechenden primären oder sekundären Amins, vorzugsweise in Gegenwart eines basischen Kondensationsmittels, z. B. von Natriumhydroxid, Pyridin oder Triäthylamin, in eine amidierte Oxalogruppe —C(=O)—$R_8$ überführen.

Die Ueberführung der genannten Gruppen $X_B$ in solche der Formel —NH—C(=O)—$R_8$ erfolgt beispielsweise oxidativ. Die Oxidation kann in üblicher Weise durch Umsetzung mit einem geeigneten Oxydationsmittel erfolgen. Geeignete Oxydationsmittel sind insbesondere oxydierende Schwermetallverbindungen, wie Silberverbindungen, z. B. Silbernitrat oder Silberpicolinat, Sauerstoffsäuren von Schwermetallen, z. B. von Mangan-IV, Mangan-VII, Chrom-VI und Eisen-III, oder von Halogenen bzw. deren Anhydride oder Salze, wie Chromsäure, Chromdioxid, Kaliumdichromat, Kaliumpermanganat Mangandioxid, Kaliumhexacyanoferat, Natriumchlorit in Gegenwart von Sulfaminsäure, Natriumhypochlorit in Gegenwart von Nickelchlorid oder Natriumjodat, Natriumperjodat oder Bleiteteraacetat. Die Umsetzung mit diesen Oxydationsmitteln erfolgt in üblicher Weise, beispielsweise in einem inerten Lösungsmittel, wie Aceton, Essigsäure, Pyridin oder Wasser, oder einem, vorzugsweise wässrigen, inerten Lösungsmittelgemisch, bei Normaltemperatur oder erforderlichenfalls unter Kühlen oder Erwärmen, z. B. bei etwa 0° bis etwa 100 °C. Die Oxydation von gegebenenfalls verätherten Glykoloylgruppen zu gegebenenfalls veresterten Oxalogruppen wird z. B. vorteilhaft mit Kaliumpermanganat in wässrigen Pyridin oder Aceton bei Raumtemperatur vorgenommen. Acetalisierte Glyoxylgruppen und Iminoacetylgruppen werden vorzugsweise sauer oxydiert, z. B. mit Kaliumdichromat in Schwefelsäure, Acylgruppen von α,β-dihydroxylierten aliphatischen Carbonsäuren, wie der Acylrest der Weinsäure, werden vorteilhaft mit Perjodsäure oxydiert, während man für die Oxydation der Glycylgruppe vorzugsweise Kaliumferat in alkalischem Milieu, z. B. bei pH 10-13, z. B. 11,5, oder organische Silbersalze, wie Silberpicolinat, verwendet. Gruppen der Formel —N=CH—$R_8$ werden vorzugsweise mit einer organischen Persäure, z. B. mit Peressigsäure oder m-Chlorperbenzoesäure, in einem inerten Lösungsmittel, z. B. Methylenchlorid, Chloroform oder Benzol, oxydiert.

Die Umsetzung von Gruppen $X_8$ der Formel —NH—C(=O)—CN mit Stickstoffwasserstoffsäure erfolgt vorzugsweise unter Bildung derselben in situ durch Behandeln eines Alkalimetallazides mit einer Säure, wie Chlorwasserstoffsäure, vorzugsweise in Toluol oder ähnlichen Lösungsmitteln.

Die Abspaltung der Schutzgruppe aus Gruppen $X_8$ der Formel —NH—C(=O)—$R_8'$, worin $R_8'$ in 1-Stellung geschütztes 5-Tetrazolyl ist, erfolgt in üblicher Weise, insbesondere durch Acidolyse, d. h. Behandeln mit einer Säure, z. B. mit Trifluoressigsäure in einem Aether, wie Anisol, oder durch katalytische Hydrierung, z. B. in Gegenwart von Palladium.

Die Ausgangsstoffe XII werden beispielsweise hergestellt, indem man eine Verbindung der Formel

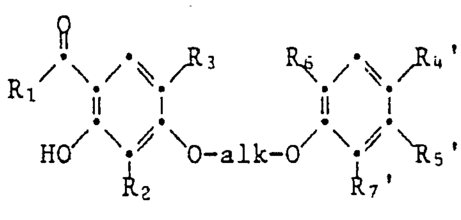

(X)

worin einer der Rest $R_4'$, $R_5'$ und $R_7'$ die Aminogruppe, ein davon verschiedener Rest $R_4'$ bzw. $R_5'$ einen Rest $R_9$ und ein davon verschiedener Rest $R_7'$ einen Rest $R_{10}$ darstellt oder ein Säureadditionssalz davon

mit einer entsprechenden Säure, z. B. der Formel $X_A$—OH (XXVIIa) bzw. $X_B$—OH (XXVIIb) oder $R_8'$—COOH (XXVIIc) oder einem funktionellen Derivat davon umsetzt. Funktionelle Derivate von Säuren XXVIIa bis XXVIIc sind vor allem eine veresterte, amidierte oder anhydridisierte Carboxygruppe, wie Niederalkoxycarbonyl, gegebenenfalls substituiertes Carbamyl, z. B. Carbamyl oder Imidazolyl-1-carbonyl, oder Halogencarbonyl, z. B. Chlor- oder Bromcarbonyl, oder eine Gruppe der Formel —$CON_3$ oder —$CON_2^+$ $Hal^-$ enthaltende Säurederivate. Als Beispiele für Säuren XXVIIa bzw. XXVIIb und deren funktionelle Derivate seien insbesondere genannt: Als funktionelle Derivate von Säuren XXVIIa Oxalylhalogenide, wie Oxalylchlorid oder Oxalylbromid, Triniederalkoxy- und Dihalogenniederalkoxyessigsäureniederalkylester, wie Oxalsäuretetraäthylester oder Dichloroxalsäurediäthylester, Oxalsäureiminodialkylester, wie Oxalsäuremono- oder -diiminodiäthylester, Oxalsäureamidine, wie N-Niederalkyloxalsäureesteramidine, Oxalsäuredithioniederalkyl-, wie -dimethylester, Cyanoformylchlorid oder Cyanogen und als Säuren XXVIIb und deren funktionelle Derivate Glykolsäuren und ihre Niederalkylester bzw. das entsprechende Lactid, Mono- oder Diniederalkoxyessigsäureniederalkyl-, wie -äthylester, z. B. Aethoxy- oder Diäthoxyessigsäureäthylester, Halogenacetanhydride, wie Chloracetanhydrid oder Chloracetylchlorid und Weinsäure, bzw. 2,3-Diacetoxybernsteinsäureanhydrid, ferner Cinnamoylchlorid, Acetylchlorid und Glycin. Funktionelle Derivate von Säuren XXVIIc sind insbesondere deren Chloride.

Die Umsetzung von Verbindungen X und XXVIIa bis XXVIIc oder ihrer Derivate kann in üblicher Weise erfolgen, beispielsweise in Gegenwart eines wasserbindenden Mittels, wie eines Säureanhydrides, z. B. von Phosphorpentoxid, oder von Dicyclohexylcarbodiimid, oder eines z. B. sauren oder basischen Kondensationsmittels, wie einer Mineralsäure, z. B. von Chlorwasserstoffsäure, oder eines Alkalimetallhydroxides oder -carbonates, z. B. von Natrium- oder Kaliumhydroxid, oder einer organischen Stickstoffbase, z. B. von Triäthylamin oder Pyridin. Bei der Umsetzung mit einem Säureanhydrid, wie Säurechlorid, verwendet man vorzugsweise eine organische Stickstoffbase als Kondensationsmittel 1. Die Umsetzung mit Carbonsäuren führt man vorzugsweise in Gegenwart eines wasserbindenden Mittels durch. Erforderlichenfalls arbeitet man jeweils in einem inerten Lösungsmittel, bei normaler Temperatur oder unter Kühlen oder Erwärmen, z. B. in einem Temperaturbereich von etwa 0° bis etwa 100 °C, in einem geschlossenen Gefäss und/oder unter Inertgas, z. B. Stickstoff.

Analog kann man Verbindungen XII, in denen $X_8$ eine Gruppe $R_8$—CH=N-bedeutet, durch Kondensation von Verbindungen X mit der gegebenenfalls veresterten oder amidierten Glyoxylsäure herstellen.

Verbindungen XII, in denen $X_8$ für eine Gruppe —NH—$X_B$ und $X_B$ für Glyoxyloyl steht, können ferner hergestellt werden, indem man eine entsprechende Halogen-, wie Bromacetylverbindung mit Hexamethylentetramin, vorzugsweise in einem wässrigen Alkohol, erhitzt oder mit Silbertetrafluoroborat in Dimethylsulfoxid oxydiert. Analog kann man auch eine Chloracetylverbindung mit Kaliumdichromat in Hexamethylphosphorsäuretriamid in Gegenwart von Dicyclohexyl-18-crown-6-äther oxydieren. Verbindungen XII, in denen $X_8$ eine Gruppe —NH—$X_B$ und $X_B$ eine Iminoacetylgruppe, z. B. gegebenenfalls substituiertes Benzyliminoacetyl, bedeutet, können ausgehend von den entsprechenden Glycylverbindungen hergestellt werden, indem man diese mit der entsprechenden Carbonylverbindung, z. B. mit Benzaldehyd, umsetzt und das so erhältliche Zwischenprodukt, z. B. eine N-Benzylidenglycylverbindung, vorzugsweise unter den Reaktionsbedingungen, umlagert.

Als funktionell abgewandelte Carboxygruppe eine Iminoäthergruppierung aufweisende funktionell abgewandelte Oxalogruppen können ausgehend von der entsprechenden Cyanocarbonylverbindung durch Umsetzung mit dem entsprechenden Alkohol, z. B. Niederalkan(di)ol oder Aminoniederalkanol, hergestellt werden.

Eine erfindungsgemäss erhältliche Verbindung der allgemeinen Formel I kann in an sich bekannter Weise in eine andere Verbindung der allgemeinen Formel I umgewandelt werden.

So kann man beispielsweise eine freie Carboxylgruppe $R_6$, $R_7$ und/oder $R_8$ in üblicher Weise, z. B. durch Behandeln mit einem Diazoniederalkan oder Triniederalkyloxonium-, Triniederalkylcarboxonium- oder Diniederalkylcarboniumsalz, wie -hexachloroantimonat oder -hexafluorophosphat, oder vor allem durch Umsetzung mit dem entsprechenden Alkohol oder einem reaktionsfähigen Derivat, wie einem Carbon-, Phosphorig-, Schweflig- oder Kohlensäureester davon, z. B. einem Niederalkanol, N,N-Diniederalkylaminoniederalkanol, N,N-Niederalkylenaminoniederalkanol, gegebenenfalls substituiertem N,N-(Aza) niederalkylenaminoniederalkanol, N,N-(Oxa) niederalkylenaminoniederalkanol oder N,N-(Thia) niederalkylenaminoniederalkanol bzw. mit einem Niederalkancarbonsäureester, Triniederalkylphosphit oder Diniederalkylsulfit, zu Verbindungen der allgemeinen Formel I, worin $R_6$, $R_7$ und/oder $R_8$ verestertes Carboxy ist, umsetzen. Die Umsetzung mit dem entsprechenden Alkohol selbst kann vorteilhaft in Gegenwart eines sauren Katalysators erfolgen, wie einer Protonensäure, z. B. von Chlor- oder Bromwasserstoff-, Schwefel-, Phosphor-, Bor-, Benzolsulfon- und/oder Toluolsulfonsäure, in einem inerten Lösungsmittel, insbesondere einem Ueberschuss des eingesetzten Alkohols und erforderlichenfalls in Gegenwart eines wasserbildenden Mittels und/oder unter destillativer, z. B. azeotroper, Entfernung des Reaktionswassers und/oder bei erhöhter Temperatur. Die Umsetzung mit einem reaktionsfähigen Derivat des entsprechenden Alkohols kann in üblicher Weise durchgeführt werden, ausgehend von einem Carbon-, Phosphorig-, Schweflig- oder Kohlensäureester beispielsweise in Gegenwart eines sauren Katalysators, wie eines der vorstehend genannten, in einem inerten Lösungsmittel, z. B. in Toluol, oder einem Ueberschuss des eingesetzten Alkoholderivates oder des entsprechenden Alkohols, erforderlichenfalls unter, z. B. azeotroper Abdestillation des Reaktionswassers. Ausgehend von einem Mineralsäure- oder Sulfonsäureester setzt man die zu veresternde Säure vorteilhaft in Form eines Salzes, z. B. des Natrium- oder Kaliumsalzes, ein und arbeitet erforderlichenfalls in Gegenwart eines basischen Kondensationsmittels, wie einer anorganischen Base, z. B. von Natrium-, Kalium- oder Calciumhydroxid oder -carbonat, oder einer tertiären organischen Stickstoffbase, z. B. von Triäthylamin oder Pyridin, und/oder in einem inerten Lösungsmittel, wie einer der vorstehenden tertiären Stickstoffbasen oder eines polaren Lösungsmittels, z. B. in Dimethylformamid und/oder bei erhöhter Temperatur. Die Umsetzung mit einem Olefin kann beispielsweise in Gegenwart eines sauren Katalysators, z. B. einer Lewissäure, z. B. von Bortrifluorid, einer Sulfonsäure, z. B. von p-Toluolsulfonsäure, oder vor allem eines basischen Katalysators, z. B. von Natrium- oder Kaliumhydroxid, vorteilhaft in einem inerten Lösungsmittel, wie einem Aether, z. B. in Diäthyläther oder Tetrahydrofuran, erfolgen.

Eine freie Carboxylgruppe $R_6$, $R_7$ und/oder $R_8$ kann ferner durch Umsetzung mit Ammoniak oder einem mindestens ein Wasserstoffatom aufweisenden Amin in üblicher Weise, unter Dehydratisieren des intermediär gebildeten Ammoniumsalzes, z. B. durch azeotrope Destillation mit Benzol oder Toluol oder trockenes Erhitzen, in eine amidierte Carboxylgruppe überführt werden.

Die vorstehend beschriebenen Umwandlungen von Carboxy in veresterte oder amidierte Carboxylgruppen können aber auch so durchgeführt werden, dass man eine Verbindung der Formel I, worin $R_6$, $R_7$ und/oder $R_8$ Carboxy ist, zunächst in üblicher Weise in ein reaktionsfähiges Derivat, beispielsweise mittels eines Halogenides des Phosphors oder Schwefels, z. B. mittels Phosphortrichlorid oder -bromid, Phosphorpentachlorid oder Thionylchlorid, in ein Säurehalogenid oder durch Umsetzung mit einem entsprechenden Alkohol oder Amin in einen reaktiven Ester, d. h. Ester mit elektronenanziehenden Strukturen, wie den Ester mit Phenol, Thiophenol, p-Nitrophenol oder Cyanmethylalkohol, oder ein reaktivs Amid, z. B. das von Imidazol oder 3,5-Dimethylpyrazol abgeleitete Amid, überführt und das erhaltene reaktionsfähige Derivat dann in üblicher Weise, z. B. wie nachstehend für die Umesterung, Umamidierung bzw. gegenseitige Umwandlung veresterter und amidierter Carboxylgruppen beschrieben, mit einem entsprechenden Alkohol, Ammoniak oder dem entsprechenden mindestens ein Wasserstoffatom aufweisenden Amin zu der gewünschten Gruppe $R_4$, $R_6$ und/oder $R_8$ umsetzt.

Eine veresterte Carboxylgruppe $R_6$, $R_7$ und/oder $R_8$ sowie Cyano $R_6$ und/oder $R_7$ kann in üblicher Weise, z. B. durch Hydrolyse in Gegenwart eines Katalysators, beispielsweise eines basischen oder sauren Mittels, wie einer starken Base, z. B. von Natrium- oder Kaliumhydroxid, oder einer Mineralsäure, z. B. von Salzsäure, Schwefelsäure oder Phosphorsäure, zur freien Carboxylgruppe, Cyano $R_6$ und/oder $R_7$ ferner zu Carbamyl hydrolysiert werden. Ebenso kann man verestertes Carboxy $R_6$, $R_7$ und/oder $R_8$ z. B. durch Umsetzung mit Ammoniak oder dem entsprechenden, mindestens ein Wasserstoffatom aufweisenden Amin, in eine amidierte Carboxylgruppe überführt.

Eine veresterte Carboxylgruppe $R_6$, $R_7$ und/oder $R_8$ kann ferner in üblicher Weise, z. B. durch Umsetzung mit einem Metallsalz, wie dem Natrium- oder Kaliumsalz, eines entsprechenden Alkohols oder mit diesem selbst in Gegenwart eines Katalysators, beispielsweise einer starken Base, z. B. von Natrium- oder Kaliumhydroxid, oder einer starken Säure, wie einer Mineralsäure, z. B. von Salzsäure, Schwefelsäure oder Phosphorsäure, oder einer organischen Sulfonsäure, z. B. von p-Toluolsulfonsäure, oder einer Lewissäure, z. B. von Bortrifluorid-Aetherat, zu einer anderen veresterten Carboxylgruppe umgeestert werden.

Eine amidierte Carboxylgruppe $R_6$, $R_7$ und/oder $R_8$ kann in üblicher Weise, z. B. durch Hydrolyse in Gegenwart eines Katalysators, beispielsweise einer starken Base, wie eines Alkalimetall- oder Erdalkalimetallhydroxids oder -carbonats, z. B. von Natrium- oder Kalilumhydroxid oder -carbonat, oder einer starken Säure, wie einer Mineralsäure, z. B. von Salzsäure, Schwefelsäure oder Phosphorsäure, in die freie Carboxylgruppe umgewandelt werden.

In einer erfindungsgemäss erhältlichen Verbindung kann man weiterhin in einen oder beide Phenylringe Substituenten einführen und/oder vorhandene Substituenten in andere Substituenten umwandeln. So kann man durch Umsetzung mit einem Niederalkylhalogenid oder Niederalken bzw. einem Niederalkansäurehalogenid oder -anhydrid, jeweils in Gegenwart einer Lewissäure, wie Aluminiumtrichlorid, Niederalkyl einführen. Ferner kann man Halogen einführen, beispielsweise durch Behandeln mit einem Halogen in Gegenwart einer Lewissäure, wie Eisen-III-chlorid, oder durch Umsetzung mit N-Chlorsuccinimid. Ferner kann man Niederalkenyl- bzw. Niederalkinylreste zu Niederalkyl reduzieren, beispielsweise durch Behandeln mit Wasserstoff in Gegenwart eines Hydrierungskatalysators, z. B. von Palladium auf Kohle. Weiterhin kann man Halogen, insbesondere Jod, durch Umsetzung mit Trifluorjodmethan in Gegenwart von Kupfer durch Trifluormethyl ersetzen.

Die neuen Verbindungen können, wie erwähnt, je nach der Wahl der Ausgangsstoffe und Arbeitsweisen, in Form eines der möglichen Isomeren oder als Gemisch derselben, z. B. je nach der Anzahl der asymmetrischen Kohlenstoffatome, als reine optische Isomere, wie Antipoden oder als Isomerengemische, wie Racemate, Diastereoisomerengemische oder Racematgemische, vorliegen.

Erhaltene Diastereomerengemische und Racematgemische können aufgrund der physikalischchemischen Unterschiede der Bestandteile in bekannter Weise in die reinen Isomeren, Diastereomeren oder Racemate aufgetrennt werden, beispielsweise durch Chromatographie und/oder fraktionierte Kristallisation.

Erhaltene Racemate lassen sich ferner nach bekannten Methoden in die optischen Antipoden

zerlegen, beispielsweise durch Umkristallisation aus einem optisch aktiven Lösungsmittel, mit Hilfe von Mikroorganismen, oder durch Umsetzen eines sauren Endstoffes mit einer mit der racemischen Säure Salze bildenden optisch aktiven Base und Trennung der auf diese Weise erhaltenen Salze, z. B. aufgrund ihrer verschiedenen Löslichkeiten, in die Diastereomeren, aus denen die Antipoden durch Einwirkung geeigneter Mittel freigesetzt werden können, zerlegen. Vorteilhaft isoliert man den wirksameren der beiden Antipoden.

Erhaltene freie Verbindungen der Formel I, z. B. solche, worin $R_6$ und/oder $R_7$ für Carboxy und/oder $R_8$ für Carboxy oder 5-Tetrazolyl steht, können in an sich bekannter Weise in Salze überführt werden, z. B. durch Behandeln mit einer Base oder mit einem geeigneten Salz einer Carbonsäure, üblicherweise in Gegenwart eines Lösungs- oder Verdünnungsmittels.

Erhaltene Salze können in an sich bekannter Weise in die freien Verbindungen bzw. erhaltene freie Verbindungen, worin $R_8$ zur Bildung von Säureadditionssalzen befähigt ist, in deren Säureadditionssalze umgewandelt werden, z. B. durch Behandeln mit einem sauren Reagens, wie einer Mineralsäure bzw. einer der genannten salzbildenden Säuren.

Die Verbindungen einschliesslich ihrer Salze können auch in Form ihrer Hydrate erhalten werden oder das zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes und/oder Racemates bzw. Antipoden verwendet oder insbesondere unter den Reaktionsbedingungen bildet.

Vorzugsweise werden solche Ausgangsstoffe verwendet und die Reaktionsbedingungen so gewählt, dass man zu den vorstehend als besonders bevorzugt aufgeführten Verbindungen gelangt.

Die vorliegende Erfindung betrifft ebenfalls pharmazeutische Präparate, welche eine der erfindungsgemässen Verbindungen der Formel I oder ein pharmazeutisch verwendbares Salz davon enthalten. Bei den erfindungsgemässen pharmazeutischen Präparaten handelt es sich um solche, die zur topischen und lokalen sowie enteralen, wie oralen oder rektalen, sowie parenteralen Verabreichung an und zur Inhalation durch Warmblüter, bestimmt sind und den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab.

Die neuen pharmazeutische Präparate enthalten z. B. von etwa 10 % bis etwa 95 %, vorzugsweise von etwa 20 % bis etwa 90 % des Wirkstoffs. Erfindungsgemässe pharmazeutische Präparate sind z. B. solche in Aerosol- oder Sprayform oder in Dosiseinheitsformen, wie Dragées, Tabletten, Kapseln oder Suppositorien, ferner Ampullen.

Die pharmazeutischen Präparate der vorliegenden Erfindung werden in an sich bekannter Weise, z. B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z. B. Lactose, Saccharose, Mannit oder Sorbit, Cellulosepräparate und/oder Calciumphosphate, z. B. Tricalciumphosphat oder Calciumhydrogenphosphat, ferner Bindemittel, wie Stärkekleister, z. B. Mais-, Weizen-, Reis- oder Kartoffelstärkekleister, Gelatine, Tragaganth, Methylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z. B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzügen versehen, wobei man u. a. konzentrierte Zuckerlösungen, welche gegebenenfalls arabischen Gummi, Talk, Polyvinylpyrrolidone, Polyäthylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z. B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z. B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert,

wobei ebenfalls Stabilisatoren zugefügt sein können.

Als rektal anwendbare pharmazeutische Präparate kommen z. B. Suppositorien in Betracht, welche aus einer Kombination des Wirkstoffs mit einer Suppositoriengrundmasse bestehen. Als Suppositoriengrundmasse eignen sich z. B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einer Grundmasse enthalten ; als Grundmassenstoffe kommen z. B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z. B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z. B. Sesamöl, oder synthetische Fettsäureester, z. B. Aethyloleat oder Triglyceride, verwendet, oder wässrige Injektionssuspensionen, welche viskositätserhöhende Stoffe, z. B. Natriumcarboxymethylcellulose, Sorbit und/oder Dextran und gegebenenfalls auch Stabilisatoren, enthalten.

Inhalationspräparate für die Behandlung der Atemwege durch nasale oder buccale Verabreichung sind z. B. Aerosole oder Sprays, welche den pharmakologische Wirkstoff in Form eines Puders oder in Form von Tropfen einer Lösung oder Suspension verteilen können. Präparate mit Puder-verteilenden Eigenschaften enthalten ausser dem Wirkstoff üblicherweise ein flüssiges Treibgas mit einem Siedepunkt unter der Raumtemperatur, sowie, wenn erwünscht, Trägerstoffe, wie flüssige oder feste nicht-ionische oder anionische oberflächenaktive Mittel und/oder Verdünnungsmittel. Präparate, in welchen der pharmakologische Wirkstoff in Lösung vorliegt, enthalten ausser diesem ein geeignetes Treibmittel, ferner, falls notwendig, ein zusätzliches Lösungsmittel und/oder einen Stabilisator. Anstelle des Treibgases kann auch Druckluft verwendet werden, wobei diese mittels einer geeigneten Verdichtungs- und Entspannungsvorrichtung nach Bedarf erzeugt werden kann.

Pharmazeutische Präparate für topische und lokale Verwendung sind z. B. für die Behandlung der Haut Lotionen und Cremen, die eine flüssige oder semifeste Oel-in-Wasser- oder Wasser-in-Oel-Emulsion enthalten, und Salben (wobei solche vorzugsweise ein Konservierungsmittel enthalten), für die Behandlung der Augen Augentropfen, welche die aktive Verbindung in wässriger oder öliger Lösung enthalten und Augensalben, die vorzugsweise in steriler Form hergestellt werden, für die Behandlung der Nase Puder, Aerosole und Sprays (ähnlich den oben beschriebenen für die Behandlung der Atemwege), sowie grobe Puder, die durch schnelles Inhalieren durch die Nasenlöcher verabreicht werden, und Nasentropfen, welche die aktive Verbindung in wässriger oder öliger Lösung enthalten, oder für die lokale Behandlung des Mundes Lutschbonbons, welche die aktive Verbindung in einer im allgemeinen aus Zucker und Gummiarabikum oder Tragaganth gebildeten Masse enthalten, welcher Geschmacksstoffe beigegeben sein können, sowie Pastillen, die den Aktivstoff in einer inerten Masse, z. B. aus Gelatine und Glycerin oder Zucker und Gummiarabikum, enthalten.

Die nachfolgenden Beispiele illustrieren die oben beschriebene Erfindung. Temperaturen sind in Celsiusgraden angegeben.

## Beispiel 1

Zu einer auf 0° gekühlten Lösung von 7,5 g (21 mMol) 3-[3-(4-Acetyl-3-hydroxy-2-n-propyl-phenoxy)-propyloxy]-6-methylanilin und 3,0 g (21 mMol) Triäthylamin in 60 ml Methylenchlorid wird innerhalb von etwa 5 Minuten eine Lösung von 1,9 ml (21 mMol) Chloroxalsäuremethylester in 8 ml Methylenchlorid zugetropft. Man lässt 90 Minuten bei Raumtemperatur nachrühren, giesst das Reaktionsgemisch auf Eiswasser und trennt die organische Phase ab. Die Methylenchlorid-Phase wird mit Wasser gewaschen, über Natriumsulfat getrocknet und unter vermindertem Druck eingeengt. Kristallisation des Rückstandes aus Methylenchlorid/Aether ergibt den N-{3-[3-(4-Acetyl-3-hydroxy-2-n-propyl-phenoxy)-propyloxy]-6-methyl-phenyl}-oxaminsäuremethylester vom Smp. 125-127°.

In analoger Weise erhält man ausgehend von 3-[3-(4-Acetyl-3-hydroxy-2-n-propyl-phenoxy)-propyloxy]-anilin N-{3-[3-(4-Acetyl-3-hydroxy-2-n-propyl-phenoxy)-propyloxy]-phenyl}-oxaminsäuremethylester vom Smp. 124-125°.

Das Ausgangsmaterial kann man z. B. folgendermassen herstellen.

Eine Suspension von 5,1 g (33 mMol) 4-Methyl-3-nitro-phenol und 4,6 g (33 mMol) geglühten Kaliumcarbonates in 100 ml Aethylmethylketon wird mit einer Spatelspitze Kaliumjodid und 9,5 g (30 mMol) 4-(3-Brompropoxy)-2-hydroxy-3-propyl-acetophenon versetzt und 14 Stunden zum Rückfluss erhitzt. Das Reaktionsgemisch wird abgekühlt, auf Wasser gegossen und dreimal mit Aether extrahiert. Die organischen Phasen werden mit Wasser gewaschen, vereinigt, über Natriumsulfat getrocknet und unter vermindertem Druck zur Trockne eingeengt. Kristallisation des Rückstandes liefert das 3-[3-(4-Acetyl-3-hydroxy-2-n-propyl-phenoxy)-propyloxy]-6-methyl-nitrobenzol vom Smp. 90-91°.

In analoger Weise erhält man ausgehend von m-Nitrophenol 3-[3-(4-Acetyl-3-hydroxy-2-n-propyl-phenoxy)-propyloxy]-nitrobenzol vom Smp. 71-72°.

Eine Lösung von 9,1 g (23,5 mMol) 3-[3-(4-Acetyl-3-hydroxy-2-n-propyl-phenoxy)-propyloxy]-6-methyl-nitrobenzol in 90 ml Tetrahydrofuran wird mit 1,0 g Raney-Nickel versetzt und bei Raumtemperatur hydriert. Der Katalysator wird abfiltriert und mit Tetrahydrofuran gewaschen. Das Filtrat wird unter vermindertem Druck zur Trockne eingedampft. Man erhält so das 3-[3-(4-Acetyl-3-hydroxy-2-n-propylphe-

noxy)-propyloxy]-6-methyl-anilin mit dem Rf-Wert = 0,12 (Kieselgel/Methylenchlorid).

In analoger Weise erhält man ausgehend von 3-[3-(4-Acetyl-3-hydroxy-2-n-propyl-phenoxy)-propyloxy]-nitrobenzol 3-[3-(4-Acetyl-3-hydroxy-2-n-propyl-phenoxy)-propyloxy]-anilin vom Smp. 76-77° (Aether/Petroläther).

Beispiel 2

Eine Suspension von 8,60 g (19,4 mMol) N-{3-[3-(4-Acetyl-3-hydroxy-2-n-propyl-phenoxy)-propyloxy]-6-methyl-phenyl}-oxaminsäuremethylester in 60 ml Methanol und 20 ml Wasser wird mit 20 ml 1n-Natronlauge versetzt und 10 Minuten zum Rückfluss erhitzt. Das Reaktionsgemisch wird abgekühlt und das ausgefallene Produkt abfiltriert. Man erhält so das Natriumsalz der N-{3-[3-(4-Acetyl-3-hydroxy-2-n-propyl-phenoxy)-propyloxy]-6-methyl-phenyl}-oxaminsäure vom Smp. 200-203°.

In analoger Weise kann das Natriumsalz der N-{3-[3-(4-Acetyl-3-hydroxy-2-n-propyl-phenoxy)-propyloxy]-phenyl}-oxaminsäure vom Smp. über 200° (Zers.) hergestellt werden.

Beispiel 3

Zu einer einer auf 0° gekühlten Lösung von 14,0 g (32 mMol) 3-[3-(4-Acetyl-3-hydroxy-2-n-propyl-phenoxy)-propyloxy]-4-brom-6-methyl-anilin und 4,9 ml Triäthylamin in 140 ml Methylenchlorid wird innerhalb von etwa 5 Minuten eine Lösung von 3,2 ml Chloroxalsäuremethylester in 10 ml Methylenchlorid zugetropft und anschliessend noch 90 Minuten bei Raumtemperatur gerührt. Das Reaktionsgemisch wird auf Eiswasser gegossen und die organische Phase abgetrennt, mit Wasser gewaschen, über Natriumsulfat getrocknet und unter vermindertem Druck eingeengt. Kristallisation aus Essigester/Aether/Petroläther liefert den N-{3-[3-(4-Acetyl-3-hydroxy-2-n-propyl-phenoxy)-propyloxy]-4-brom-6-methyl-phenyl}-oxaminsäuremethylester vom Smp. 92-93°.

In analoger Weise erhält man :

N-{3-[3-(4-Acetyl-3-hydroxy-2-n-propyl-phenoxy)-propyloxy]-2-methyl-phenyl}-oxaminsäuremethylester vom Smp. 108-110° (Essigester/Petroläther) ;

N-{3-[5-(4-Acetyl-3-hydroxy-2-n-propyl-phenoxy)-pentyloxy]-phenyl}-oxaminsäuremethylester vom Smp. 108-109° (Methylenchlorid/Aether) ;

N-{3-[3-(4-Acetyl-3-hydroxy-2-n-propyl-phenoxy)-propyloxy]-4-chlor-6-trifluormethyl-phenyl}-oxaminsäuremethylester vom Smp. 109-110° (Aether/Petroläther) ;

N-{3-[3-(4-Acetyl-3-hydroxy-2-n-propyl-phenoxy)-propyloxy]-2,4,6-trichlor-phenyl}-oxaminsäuremethylester als Oel mit dem RF-Wert = 0,17 (Kieselgel/Methylenchlorid) und

N-{3-[3-(4-Acetyl-3-hydroxy-2-n-propyl-phenoxy)-propyloxy]-4,6-dimethyl-phenyl}-oxaminsäuremethylester vom Smp. 111-112° (Essigester/Petroläther).

Die Ausgangsmaterialien können z. B. folgendermassen hergestellt werden.

Eine Suspension von 9,8 g (42 mMol) 2-Brom-4-methyl-5-nitrophenol und 6,4 g (46,6 mMol) geglühtem Kaliumcarbonat in 100 ml Aethylmethylketon wird mit einer Spatelspitze Kaliumjodid und 14,7 g (46,6 mMol) 4-(3-Brompropyloxy)-2-hydroxy-3-n-propyl-acetophenon versetzt und 7 Stunden zum Rückfluss erhitzt. Das Reaktionsgemisch wird abgekühlt, auf Wasser gegossen und dreimal mit Aether extrahiert. Die organischen Phasen werden mit Wasser gewaschen, vereinigt, über Natriumsulfat getrocknet und unter vermindertem Druck eingeengt. Kristallisation des Rückstandes liefert das 3-[3-(4-Acetyl-3-hydroxy-2-n-propylphenoxy)-propyloxy)-4-brom-6-methyl-nitrobenzol vom Smp. 100-101° (Aether).

In analoger Weise erhält man :

3-[3-(4-Acetyl-3-hydroxy-2-n-propyl-phenoxy)-propyloxy]-2-methyl-nitrobenzol vom Smp. 79-80° (Aether) ;

3-[5-(4-Acetyl-3-hydroxy-2-n-propyl-phenoxy)-pentyloxy]-nitrobenzol vom Smp. 52-54° ;

3-[3-(4-Acetyl-3-hydroxy-2-n-propyl-phenoxy)-propyloxy]-4-chlor-6-trifluormethyl-nitrobenzol vom Smp. 112-113° (Aethanol) ;

3-[3-(4-Acetyl-3-hydroxy-2-n-propyl-phenoxy)-propyloxy]-2,4,6-trichlor-nitrobenzol vom Smp. 91-92° (Aether/Petroläther) und

3-[3-(4-Acetyl-3-hydroxy-2-n-propyl-phenoxy)-propyloxy]-4,6-dimethyl-nitrobenzol vom Smp. 89-91° (Aether/Petroläther).

Eine Lösung von 15,9 g (34,1 mMol) 3-[3-(4-Acetyl-3-hydroxy-2-n-propyl-phenoxy)-propyloxy]-4-brom-6-methyl-nitrobenzol in 160 ml Tetrahydrofuran wird mit 3,0 g Raney-Nickel versetzt und bei Raumtemperatur hydriert. Der Katalysator wird abfiltriert und mit Tetrahydrofuran gewaschen. Das Filtrat wird unter vermindertem Druck zur Trockne eingedampft. Man erhält so das 3-[3-(4-Acetyl-3-hydroxy-2-n-propyl-phenoxy)-propyloxy]-4-brom-6-methyl-anilin vom Smp. 108-109° (Aether/Petroläther).

In analoger Weise erhält man :

3-[3-(4-Acetyl-3-hydroxy-2-n-propyl-phenoxy)-propyloxy]-2-methyl-anilin vom Smp. 99-100° (Aether/Petroläther) ;

3-[5-(4-Acetyl-3-hydroxy-2-n-propyl-phenoxy)-pentyloxy]-anilin als Oel mit dem Rf-Wert = 0,44 (Kieselgel ; Methylenchlorid/Essigester = 10 : 1) ;

3-[3-(4-Acetyl-3-hydroxy-2-n-propyl-phenoxy)-propyloxy]-4-chlor-6-trifluormethyl-anilin vom Smp. 107-108° (Methanol) ;

3-[3-(4-Acetyl-3-hydroxy-2-n-propyl-phenoxy)-propyloxy]-2,4,5-trichlor-anilin vom Smp. 57-58° und

3-[3-(4-Acetyl-3-hydroxy-2-n-propyl-phenoxy)-propyloxy]-4,6-dimethyl-anilin vom Smp. 72-73° (Aether-Petroläther).

Beispiel 4

Eine Suspension von 15,1 g (29 mMol) N-{3-[3-(4-Acetyl-3-hydroxy-2-n-propyl-phenoxy)-propyloxy]-4-brom-6-methyl-phenyl}-oxaminsäuremethylester in 120 ml Methanol und 30,4 ml n-Natronlauge wird 90 Minuten zum Rückfluss erhitzt. Das Reaktionsgemisch wird unter vermindertem Druck eingeengt, in Aceton und verdünnter Natronlauge gelöst und mit verdünnter Salzsäure angesäuert. Das ausgefallene Produkt wird abfiltriert, mit Aceton/Wasser gewaschen und aus Isopropanol umkristallisiert. Man erhält so die N-{3-[3-(4-Acetyl-3-hydroxy-2-n-propyl-phenoxy)-propyloxy]-4-brom-6-methyl-phenyl}-oxaminsäure vom Smp. 191-192°. 10,9 g (21,5 mMol) dieses Produkts werden in 450 ml Isopropanol unter Rückfluss gelöst und dann mit einer Lösung von 3,20 g (21,5 mMol) Triäthanolamin in 30 ml Isopropanol versetzt. Die Lösung wird abgekühlt und unter vermindertem Druck auf etwa ein Drittel eingeengt. Nach Zugabe von Aether beginnt die Kristallisation. Das ausgefallene Produkt wird abfiltriert und mit Aether gewaschen. Man erhält so das Triäthanolammoniumsalz der N-{3-[3-(4-Acetyl-3-hydroxy-2-n-propyl-phenoxy)-propyloxy]-4-brom-6-methyl-phenyl}-oxaminsäure vom Smp. 85-86°.

In analoger Weise erhält man ausgehend von N-{3-[3-(4-Acetyl-3-hydroxy-2-n-propyl-phenoxy)-propyloxy]-2-methyl-phenyl}-oxaminsäuremethylester N-{3-[3-(4-Acetyl-3-hydroxy-2-n-propyl-phenoxy)-propyloxy]-2-methyl-phenyl}-oxaminsäure vom Smp. 146-148° (Toluol) und deren Triäthanolammoniumsalz vom Smp. 114-115° sowie

ausgehend vom N-[3-(5-[4-Acetyl-3-hydroxy-2-n-propyl-phenoxy]-pentyloxy)-phenyl]-oxaminsäuremethylester N-{3-[5-(4-Acetyl-3-hydroxy-2-n-propyl-phenoxy)-pentyloxy]-phenyl}-oxaminsäure vom Smp. 117-118° (Toluol) sowie deren Triäthanolammoniumsalz ;

ausgehend von N-{3-[3-(4-Acetyl-3-hydroxy-2-n-propyl-phenoxy)-propyloxy]-4-chlor-6-trifluormethyl-phenyl}-oxaminsäuremethylester N-{3-[3-(4-Acetyl-3-hydroxy-2-n-propyl-phenoxy)-propyloxy]-4-chlor-6-trifluormethyl-phenyl}-oxaminsäure vom Smp. 173-174° und deren Triäthanolammoniumsalz vom Smp. 94-95° ;

ausgehend vom N-{3-[3-(4-Acetyl-3-hydroxy-2-n-propyl-phenoxy)-propyloxy]-2,4,6-trichlor-phenyl}-oxaminsäuremethylester N-{3-[3-(4-Acetyl-3-hydroxy-2-n-propyl-phenoxy)-propyloxy]-2,4,6-trichlorphenyl}-oxaminsäure vom Smp. 151-152° (Aether/Petroläther) und deren Triäthanolammoniumsalz und ;

ausgehend vom N-{3-[3-(4-Acetyl-3-hydroxy-2-n-propyl-phenoxy)-propyloxy]-4,6-dimethyl-phenyl}-oxaminsäuremethylester N-{3-[3-(4-Acetyl-3-hydroxy-2-n-propyl-phenoxy)-propyloxy]-4,6-dimethyl-phenyl}-oxaminsäure vom Smp. 197-198° (Essigester/Petroläther) und deren Triäthanolammoniumsalz.

Beispiel 5

Eine Lösung von 8,45 g (50 mMol) 2-Methoxy-4-nitrophenol in 100 ml N,N-Dimethylformamid wird mit 2,25 g (50 mMol) 55 %iger Natriumhydrid-Suspension in Mineralöl versetzt. Anschliessend wird auf 40° erwärmt und innerhalb von etwa 10 Minuten eine Lösung von 15,7 g (50 mMol) 4-(3-Bromopropoxy)-2-hydroxy-3-n-propylacetophenon in 30 ml N,N-Dimethylformamid zugetropft und anschliessend noch 6 Stunden bei dieser Temperatur gehalten. Das Reaktionsgemisch wird unter vermindertem Druck eingeengt, mit Wasser verdünnt und dreimal mit Methylenchlorid extrahiert. Die organischen Phasen werden mit Wasser gewaschen, vereinigt, über Natriumsulfat getrocknet und unter vermindertem Druck zur Trockene eingedampft. Der Rückstand wird an Kieselgel mit Methylenchlorid als Laufmittel chromatographiert. Kristallisation der dünnschichtchromatographisch reinen Fraktionen ergibt das 4-[3-(4-Acetyl-3-hydroxy-2-n-propyl-phenoxy)-propyloxy]-3-methoxy-nitrobenzol vom Smp. 109-111°.

In analoger Weise erhält man :

4-[3-(4-Acetyl-3-hydroxy-2-n-propyl-phenoxy)-propyloxy]-2-methyl-nitrobenzol vom Smp. 76-77° ;

2-[3-(4-Acetyl-3-hydroxy-2-n-propyl-phenoxy)-propyloxy]-nitrobenzol vom Smp. 103-104° (Aether) und

4-[3-(4-Acetyl-3-hydroxy-2-n-propyl-phenoxy)-propyloxy]-nitrobenzol vom Smp. 98-99° (Essigester/Aether/Petroläther).

Die Reduktion der Nitrogruppe zur Aminogruppe mit Wasserstoff und Raney-Nickel in Tetrahydrofuran wird analog der im Beispiel 1 beschriebenen Methode durchgeführt. Man erhält so aus den oben beschriebenen Nitro-Verbindungen :

4-[3-(4-Acetyl-3-hydroxy-2-n-propyl-phenoxy)-propyloxy]-3-methoxy-anilin vom Smp. 68-69° (Aether/Petroläther) ;

4-[3-(4-Acetyl-3-hydroxy-2-n-propyl-phenoxy)-propyloxy]-2-methyl-anilin vom Smp. 81-82° (Aether/Petroläther) ;

2-[3-(4-Acetyl-3-hydroxy-2-n-propyl-phenoxy)-propyloxy]-anilin als Oel und

4-[3-(4-Acetyl-3-hydroxy-2-n-propyl-phenoxy)-propyloxy]-anilin vom Smp. 76-77° (Essigester/Petroläther).

Die Umsetzung dieser Anilinverbindungen mit Chloroxalsäuremethylester und Triäthylamin in Methylenchlorid zu den Oxaminsäureestern erfolgt wie in Beispiel 1 beschrieben. Man erhält so aus den oben beschriebenen Anilinen die folgenden Verbindungen :

N-{4-[3-(4-Acetyl-3-hydroxy-2-n-propyl-phenoxy)-propyloxy]-3-methoxy-phenyl}-oxaminsäuremethylester vom Smp. 135-136° (Methylenchlorid/Aether) ;

N-{4-[3-(4-Acetyl-3-hydroxy-2-n-propyl-phenoxy)-propyloxy]-2-methyl-phenyl}-oxaminsäuremethylester vom Smp. 116-117° (Aether/Petroläther) ;

N-{2-[3-(4-Acetyl-3-hydroxy-2-n-propyl-phenoxy)-propyloxy]-phenyl}-oxaminsäuremethylester vom Smp. 110-111° (Essigester/Aether) und

N-{4-[3-(4-Acetyl-3-hydroxy-2-n-propyl-phenoxy)-propyloxy]-phenyl}-oxaminsäuremethylester vom Smp. 135-136° (Essigester/Aether/Petroläther).

## Beispiel 6

Eine Lösung von 7,95 g (17,3 mMol) N-{4-[3-(4-Acetyl-3-hydroxy-2-n-propyl-phenoxy)-propyloxy]-3-methoxy-phenyl}-oxaminsäuremethylester in 100 ml Methanol wird mit 18 ml einer n-Natronlauge versetzt und 1 Stunde zum Rückfluss erhitzt. Die heisse Lösung wird anschliessend in 200 ml 0,1 n-Salzsäure gegossen. Das ausgefallene Produkt wird abfiltriert, mit Wasser gewaschen und im Trockenschrank über Phosphorpentoxid getrocknet. Die so erhaltene N-{4-[3-(4-Acetyl-3-hydroxy-2-n-propyl-phenoxy)-propyloxy}-3-methoxy-phenyl}-oxaminsäure vom Smp. 156-157° wird erneut in 100 ml Methanol heiss gelöst und mit einem Aequivalent Triäthanolamin in 10 ml Methanol versetzt. Nach Zugabe von 400 ml Aether setzt Kristallisation ein. Man erhält so das Triäthanolammoniumsalz der N-{4-[3-(4-Acetyl-3-hydroxy-2-n-propyl-phenoxy)-propyloxy]-3-methoxy-phenyl}-oxaminsäure vom Smp. 125-127°.

In analoger Weise erhält man :

ausgehend von N-{4-[3-(4-Acetyl-3-hydroxy-2-n-propyl-phenoxy)-propyloxy}-2-methyl-phenyl}-oxaminsäuremethylester N-{4-[3-(4-Acetyl-3-hydroxy-2-n-propyl-phenoxy)-propyloxy}-2-methyl-phenyl}-oxaminsäure vom Smp. 164-165° (Essigester/Petroläther) und deren Triäthanolammoniumsalz vom Smp. 103-104° ;

ausgehend von N-{2-[3-(4-Acetyl-3-hydroxy-2-n-propyl-phenoxy)-propoxy]-phenyl}-oxaminsäuremethylester N-{2-[3-(4-Acetyl-3-hydroxy-2-n-propyl-phenoxy)-propyloxy]-phenyl}-oxaminsäure vom Smp. 164-165° (Essigester/Petroläther) und deren Triäthanolammoniumsalz ;

ausgehend von N-[4-(3-[4-Acetyl-3-hydroxy-2-n-propyl-phenoxy]-propoxy)-phenyl]-oxaminsäuremethylester N-{4-[3-(4-Acetyl-3-hydroxy-2-n-propyl-phenoxy)-propyloxy]-phenyl}-oxaminsäure vom Smp. 145-146° (Aceton/Aether/Petroläther) und deren Triäthanolammoniumsalz von Smp. 111-112°.

## Beispiel 7

Eine Lösung von 5,6 g (8,58 mMol) N-{3-[3-(4-Acetyl-3-hydroxy-2-n-propyl-phenoxy)-propyloxy]-4-brom-6-methyl-phenyl}-1-(4-methoxybenzyl)-tetrazol-5-carboxamid in 150 ml Trifluoressigsäure und 15 ml Anisol wird 30 Minuten zum Rückfluss erhitzt. Das Reaktionsgemisch wird unter vermindertem Druck eingeengt, mit etwa 200 ml Aether sowie 300 ml Petroläther versetzt und die Kristalle abfiltriert. Das so erhaltene N-{3-[3-(4-Acetyl-3-hydroxy-2-n-propyl-phenoxy)-propyloxy]-4-brom-6-methyl-phenyl}-1H-tetrazol-5-carboxamid vom Smp. 236-238° wird in 150 ml Tetrahydrofuran heiss gelöst und mit einem Aequivalent Triäthanolamin in 20 ml Tetrahydrofuran versetzt. Nach Zugabe von Aether setzt Kristallisation ein. Man erhält so das Triäthanolammoniumsalz von N-{3-[3-(4-Acetyl-3-hydroxy-2-n-propyl-phenoxy)-propyloxy]-4-brom-6-methyl-phenyl}-1H-tetrazol-5-carboxamid vom Smp. 63-65°.

In analoger Weise erhält man :

ausgehend von N-{3-[3-(4-Acetyl-3-hydroxy-2-n-propyl-phenoxy)-propyloxy]-phenyl}-1-(4-methoxybenzyl)-tetrazol-5-carboxamid N-{3-[3-(4-Acetyl-3-hydroxy-2-n-propyl-phenoxy)-propyloxy]-phenyl}-1H-tetrazol-5-carboxamid vom Smp. 176-178° (Essigester/Petroläther) und dessen Triäthanolammoniumsalz vom Smp. 118-119° (Aceton/Aether).

Die Ausgangsmaterialien erhält man beispielsweise wie folgt :

Zu einer Suspension von 5,8 g (21,5 mMol) Kalium-{1-(4-methoxy-benzyl)-tetrazol}-5-carboxylat in 110 ml Benzol und 1,0 ml Pyridin gibt man bei 0-5° 1,84 ml (21,5 mMol) Oxalylchlorid und lässt 30 Minuten bei Raumtemperatur rühren. Das Reaktionsgemisch wird unter vermindertem Druck eingeengt, der Rückstand in Benzol aufgenommen und erneut unter vermindertem Druck eingedampft. Der Rückstand wird in 80 ml Methylenchlorid gelöst und bei 0-5° innerhalb von etwa 10 Minuten zu einer Lösung von 7,5 g (17,2 mMol) 3-[3-(4-Acetyl-3-hydroxy-2-n-propyl-phenoxy)-propyloxy]-4-brom-6-methyl-anilin (Beispiel 3) und 1,72 ml (21,5 mMol) Pyridin in 40 ml Methylenchlorid zugetropft. Anschliessend wird 3 Stunden bei Raumtemperatur berührt. Das Reaktionsgemisch wird mit Methylenchlorid verdünnt und dreimal mit Wasser gewaschen. Die organischen Phasen werden vereinigt, über Natriumsulfat getrocknet und unter vermindertem Druck eingedampft. Kristallisation des Rückstandes aus Methylenchlorid/Aether liefert das N-{3-[3-(4-Acetyl-3-hydroxy-2-n-propyl-phenoxy)-propyloxy]-4-brom-6-methyl-phenyl}-1-(4-methoxy-benzyl)-tetrazol-5-carboxamid vom Smp. 146-147°.

In analoger Weise erhält man :
ausgehend von 3-[3-(4-Acetyl-3-hydroxy-2-n-propyl-phenoxy)-propyloxy]-anilin (Beispiel 3) und 1-(4-Methoxybenzyl)-tetrazolyl-5-carbonsäure-Kaliumsalz N-{3-[3-(4-Acetyl-3-hydroxy-2-n-propyl-phenoxy)-propyloxy]-phenyl}-1-(4-methoxybenzyl)-tetrazol-5-carboxamid vom Smp. 141-142 (Aether/Petroläther).

Beispiel 8

Zu einer auf 0 °C gekühlten Lösung von 43,6 g (100 mMol) 3-[3-(4-Acetyl-3-hydroxy-2-n-propyl-phenoxy)-propyloxy]-4-brom-6-methyl-anilin und 15,3 ml Triäthylamin in 400 ml Methylenchlorid wird innerhalb von etwa 5 Minuten eine Lösung von 12,3 ml Chloroxalsäureäthylester in 30 ml Methylenchlorid zugetropft und anschliessend noch 90 Minuten bei Raumtemperatur gerührt. Das Reaktionsgemisch wird auf Eiswasser gegossen und die organische Phase abgetrennt, mit Wasser gewaschen, über Natriumsulfat getrocknet und unter vermindertem Druck eingeengt. Kristallisation aus Essigester/Aether/Petroläther liefert den N-{3-[3-(4-Acetyl-3-hydroxy-2-n-propyl-phenoxy)-propyloxy]-4-brom-6-methyl-phenyl}-oxaminsäureäthylester vom Smp. 100-102°.

Beispiel 9

Eine Suspension von 4,0 g (7,46 mMol) N-{3-[3-(3-(4-Acetyl-3-hydroxy-2-n-propyl-phenoxy)-propyloxy]-4-brom-6-methyl-phenyl}-oxaminsäureäthylester in 400 ml Aethanol werden mit 448 mg (7,5 mMol) Kaliumhydroxid versetzt und 4 Stunden am Rückfluss erhitzt. Das Reaktionsgemisch wird abgekühlt und das ausgefallene Produkt abfiltriert. Man erhält so das Kaliumsalz der N-{3-[3-(4-Acetyl-3-hydroxy-2-n-propyl-phenoxy)-propyloxy]-4-brom-6-methyl-phenyl}-3-oxaminsäure von Smp. 117-119°.

In analoger Weise kann man auch das Natriumsalz der N-{3-[3-(4-Acetyl-3-hydroxy-2-n-propyl-phenoxy)-propyloxy]-4-brom-6-methyl-phenyl}-oxaminsäure vom Smp. 215° (Zers.) herstellen.

Beispiel 10

4,06 g (8 mMol) N-{3-[3-(4-Acetyl-3-hydroxy-2-n-propyl-phenoxy)-propyloxy]-4-brom-6-methyl-phenyl}-oxaminsäure werden in 40 ml Aceton gelöst und mit einer Lösung von 840 mg (8 mMol) Diäthanolamin in 5 ml Aceton versetzt. Nach Zugabe von Aether beginnt die Kristallisation. Das ausgefallene Produkt wird abfiltriert und mit Aether gewaschen. Man erhält so das Diäthanolammoniumsalz der N-{3-[3-(4-Acetyl-3-hydroxy-2-n-propyl-phenoxy)-propyloxy]-4-brom-6-methyl-phenyl}-oxaminsäure vom Smp. 101-103°.

In analoger Weise kann man auch das Tris-(hydroxymethyl) methyl-ammoniumsalz der N-{3-[3-(4-Acetyl-3-hydroxy-2-n-propyl-phenoxy)-propyloxy]-4-brom-6-ethyl-phenyl}-oxaminsäure vom Smp. 88-90° herstellen.

Beispiel 11

Eine Lösung von 7.05 g (12,06 mMol) N-{3-[3-(4-Acetyl-3-hydroxy-2-n-propyl-phenoxy)-propyloxy]-2-cyano-phenyl}-1-(4-methoxybenzyl)-tetrazol-5-carboxamid in 150 ml Trifluoressigsäure und 15 ml Anisol wird 30 Minuten zum Rückfluss erhitzt. Das Reaktionsgemisch wird unter vermindertem Druck eingeengt, mit etwa 200 ml Aether sowie 300 ml Petroläther versetzt und die Kristalle abfiltriert. Das so erhaltene N-{3-[3-(4-Acetyl-3-hydroxy-2-n-propyl-phenoxy)-propyloxy]-2-cyano-phenyl}-1H-tetrazol-5-carboxamid vom Smp. 206-208° wird in 50 ml Aceton heiss gelöst und mit der berechneten Menge Triäthanolamin in 30 ml Aceton versetzt. Nach Zugabe von Aether setzt Kristallisation ein. Man erhält so das Triäthanolammoniumsalz von N-{3-[3-(4-Acetyl-3-hydroxy-2-n-propyl-phenoxy)-propyloxy]-2-cyano-phenyl}-1H-tetrazol-5-carboxamid vom Smp. 81° (Zersetzung).

Das Ausgangsmaterial kann man z. B. folgendermassen herstellen :

Eine Suspension von 13.1 g (80 mMol) 2-Cyano-3-nitro-phenol und 13.8 g (100 mMol) geglühten Kaliumcarbonates in 100 ml Aethylmethylketon wird mit einer Spatelspitze Kaliumjodid und 31.5 g (100 mMol) 4-(3-Brompropoxy)-2-hydroxy-3-propyl-acetophenon versetzt und 20 Stunden zum Rückfluss erhitzt. Das Reaktionsgemisch wird abgekühlt, auf Wasser gegossen und dreimal mit Methylenchlorid extrahiert. Die organischen Phasen werden mit Wasser gewaschen, vereinigt, über Natriumsulfat getrocknet und unter vermindertem Druck zur Trockne eingeengt. Kristallisation des Rückstandes aus Aether/Hexan liefert das 2-[3-(4-Acetyl-3-hydroxy-2-propyl-phenoxy)-propyloxy]-6-nitro-benzonitril vom Smp. 86-88°.

Zur Lösung von 10 g (25.1 mMol) 2-[3-(4-Acetyl-3-hydroxy-2-propyl-phenoxy)-propyloxy]-6-nitro-benzonitril und 10 g Cyclohexan in 500 ml Aethanol gibt man 2,5 g 10 % Pd auf Kohle und erhitzt 30 Minuten zum Rückfluss. Nach Abkühlen auf Raumtemperatur wird filtriert und vom Lösungsmittel befreit. Der Rückstand wird mit Aether versetzt und die ausgeschiedenen Kristalle werden abfiltriert. Man erhält 2-Amino-6-[3-(4-Acetyl-3-hydroxy-2-n-propyl-phenoxy)-propyloxy]-benzonitril vom Smp. 120-122 °C.

Zu einer Suspension von 5,8 g (21,5 mMol) Kalium-{1-(4-methoxy-benzyl)-tetrazol}-5-carboxylat in 110 ml Benzol und 1,0 ml Pyridin gibt man bei 0-5° 1,84 ml (21,5 mMol) Oxalylchlorid und lässt 30

Minuten bei Raumtemperatur rühren. Das Reaktionsgemisch wird unter vermindertem Druck eingeengt, der Rückstand in Benzol aufgenommen und erneut unter vermindertem Druck eingedampft. Der Rückstand wird in 80 ml Methylenchlorid gelöst und bei 0-5° innerhalb von etwa 10 Minuten zu einer Lösung von 6,3 g (17,2 mMol) 2-Amino-6-[3-(4-Acetyl-3-hydroxy-2-n-propyl-phenoxy)-propyloxy]-benzonitril und 1,72 ml (21,5 mMol) Pyridin in 40 ml Methylenchlorid zugetropft. Anschliessend wird 3 Stunden bei Raumtemperatur gerührt. Das Reaktionsgemisch wird mit Methylenchlorid verdünnt und dreimal mit Wasser gewaschen. Die organischen Phasen werden vereinigt, über Natriumsulfat getrocknet und unter vermindertem Druck eingedampft. Kristallisation des Rückstandes aus Aethylacetat/Hexan liefert das N-{3-[3-(4-Acetyl-3-hydroxy-2-n-propyl-phenoxy)-propyloxy]-2-cyano-phenyl}-1-(4-methoxybenzyl)-tetrazol-5-carboxamid vom Smp. 137-139°.

## Beispiel 12

Zu einer auf 0° gekühlten Lösung von 7,0 g (17.8 mMol) 3-[3-(4-Acetyl-3-hydroxy-2-n-propyl-phenoxy)-propyloxy]-4-methyl-6-chlor-anilin und 22.2 g (21.4 mMol) Triäthylamin in 100 ml Methylenchlorid wird innerhalb von etwa 5 Minuten eine Lösung von 1,9 ml (21 mMol) Chloroxalsäuremethylester in 8 ml Methylenchlorid zugetropft. Man lässt 90 Minuten bei Raumtemperatur nachrühren, giesst das Reaktionsgemisch auf Eiswasser und trennt die organische Phase ab. Die Methylenchlorid-Phase wird mit Wasser gewaschen, über Natriumsulfat getrocknet und unter vermindertem Druck eingeengt.

Kristallisation des Rückstandes aus Methylenchlorid/Aether ergibt den N-{3-[3-(4-Acetyl-3-hydroxy-2-n-propyl-phenoxy)-propyloxy]-4-methyl-6-chlor-phenyl}-oxaminsäuremethylester vom Smp. 101-102°.

In analoger Weise erhält man ausgehend von 3-[3-(4-Acetyl-3-hydroxy-2-n-propyl-phenoxy)-propyloxy]-4-chlor-6-methyl-anilin den N-{3-[3-(4-Acetyl-3-hydroxy-2-n-propyl-phenoxy)-propyloxy]-4-chlor-6-methyl-phenyl}-oxaminsäuremethylester vom Smp. 92-93 °C, sowie ausgehend von 3-[3-(4-Acetyl-3-hydroxy-2-n-propyl-phenoxy)-propyloxy]-4-methyl-6-brom-anilin den N-{3-[3-(4-Acetyl-3-hydroxy-2-n-propyl-phenoxy)-propyloxy]-4-methyl-6-brom-phenyl}-oxaminsäuremethylester vom Smp. 100-101°.

Die Ausgangsmaterialien erhält man beispielsweise wie folgt :

Eine Suspension von 9,7 g (51 mMol) 2-Methyl-4-chlor-5-nitro-phenol und 10 g (72 mMol) geglühten Kaliumcarbonates in 50 ml Aethylmethylketon wird mit einer Spatelspitze Kaliumjodid und 30 g (95 mMol) 4-(3-Brompropoxy)-2-hydroxy-3-propyl-acetophenon versetzt und 4 Stunden zum Rückfluss erhitzt. Das Reaktionsgemisch wird abgekühlt, auf Wasser gegossen und dreimal mit Methylenchlorid extrahiert. Die organischen Phasen werden mit Wasser gewaschen, vereinigt, über Natriumsulfat getrocknet und unter vermindertem Druck zur Trockne eingeengt. Kristallisation des Rückstandes aus Methylenchlorid/Hexan liefert das 3-[3-(4-Acetyl-3-hydroxy-2-n-propyl-phenoxy)-propyloxy]-4-methyl-6-chlor-nitrobenzol vom Smp. 113-115 °C.

In analoger Weise erhält man ausgehend von 2-Chlor-4-methyl-5-nitro-phenol 3-[3-(4-Acetyl-3-hydroxy-2-n-propyl)-propyloxy]-4-chlor-6-methyl-nitrobenzol vom Smp. 96-98 °C, sowie ausgehend von 2-Methyl-4-brom-5-nitro-phenol 3-[3-(4-Acetyl-3-hydroxy-2-n-propyl)-propyloxy]-4-methyl-6-brom-nitrobenzol vom Smp. 111-113°.

Eine Lösung von 19 g (45 mMol) 3-[3-(4-Acetyl-3-hydroxy-2-n-propyl-phenoxy)-propyloxy]-4-methyl-6-chlor-nitrobenzol in 200 ml Tetrahydrofuran with mit 4.0 g Raney-Nickel versetzt und bei Raumtemperatur hydriert. Der Katalysator wird abfiltriert und mit Tetrahydrofuran gewaschen. Das Filtrat wird unter vermindertem Druck zur Trockne eingedampft und der Rückstand aus Aether/Hexan umkristallisiert. Man erhält so das 3-[3-(4-Acetyl-3-hydroxy-2-n-propyl-phenoxy)-propyloxy]-4-methyl-6-chlor-anilin vom Smp. 81 °C.

In analoger Weise erhält man ausgehend von 3-[3-(4-Acetyl-3-hydroxy-2-n-propyl-phenoxy)-propyloxy]-4-chlor-6-methyl-nitrobenzol das 3-[3-(4-Acetyl-3-hydroxy-2-n-propyl-phenoxy)-propyloxy]-4-chlor-6-methyl-anilin vom Smp. 119 °C sowie ausgehend von 3-[3-(4-Acetyl-3-hydroxy-2-n-propyl-phenoxy)-propyloxy]-4-methyl-6-brom-nitrobenzol das 3-[3-(4-Acetyl-3-hydroxy-2-n-propyl-phenoxy)-propyloxy]-4-methyl-6-brom-anilin vom Smp. 97-99°.

## Beispiel 13

Eine Suspension von 6,1 g (12.8 mMol) N-{3-[3-(4-Acetyl-3-hydroxy-2-n-propyl-phenoxy)-propyloxy]-4-methyl-6-chlor-phenyl}-oxaminsäuremethylester in 200 ml Methanol und 14.1 ml n-Natronlauge wird 90 Minuten am Rückfluss erhitzt. Das Reaktionsgemisch wird unter vermindertem Druck eingeengt, in heissem Wasser gelöst und nach Abkühlen mit verdünnter Salzsäure angesäuert. Das ausgefallene Produkt wird abfiltriert, mit Wasser gewaschen und aus Aceton umkristallisiert. Man erhält so die N-{3-[3-(4-Acetyl-3-hydroxy-2-n-propyl-phenoxy)-propyloxy]-4-methyl-6-chlor-phenyl}-oxaminsäure vom Smp. 183-184° (Zersetzung). Die Säure wird in 350 ml Aceton gelöst und mit einem Aequivalent Triethanolamin versetzt. Die Lösung wird unter vermindertem Druck auf etwa ein Drittel eingeengt und mit Aether versetzt. Das ausgefallene Produkt wird abfiltriert und mit Aether gewaschen. Man erhält so das Triäthanolammoniumsalz der N-{3-[3-(4-Acetyl-3-hydroxy-2-n-propyl-phenoxy)-propyloxy]-4-methyl-6-chlor-phenyl}-oxaminsäure vom Smp. 70° (Zersetzung).

In analoger Weise erhält man ausgehend von N-{3-[3-(4-Acetyl-3-hydroxy-2-n-propyl-phenoxy)-propyloxy]-4-chlor-6-methyl-phenyl}-oxaminsäuremethylester N-{3-[3-(4-Acetyl-3-hydroxy-2-n-propyl-phenoxy)-propyloxy]-4-chlor-6-methyl-phenyl}-oxaminsäure vom Smp. 188-189 °C und deren Triäthanolammoniumsalz vom Smp. 86-88°.

Beispiel 14

Eine Lösung von 7.2 g (11.9 mMol) N-{3-[3-(4-Acetyl-3-hydroxy-2-n-propyl-phenoxy)-propyloxy]-4-methyl-6-chlor-phenyl}-1-(4-methoxybenzyl)-tetrazol-5-carboxamid in 150 ml Trifluoressigsäure und 15 ml Anisol wird 30 Minuten zum Rückfluss erhitzt. Das Reaktionsgemisch wird unter vermindertem Druck eingeengt, mit etwa 200 ml Aether sowie 300 ml Petroläther versetzt und die Kristalle abfiltriert. Das so erhaltene N-{3-[3-(4-Acetyl-3-hydroxy-2-n-propyl-phenoxy)-propyloxy]-4-methyl-6-chlor-phenyl}-1H-tetrazol-5-carboxamid vom Smp. 213-215° wird in 70 ml Aceton heiss gelöst und mit einem Aequivalent Triäthanolamin versetzt. Nach Zugabe von Aether setzt Kristallisation ein. Man erhält so das Triäthanolammoniumsalz vom Smp. 107-109°.

In analoger Weise erhält man ausgehend von N-{3-[3-(4-Acetyl-3-hydroxy-2-n-propyl-phenoxy)-propyloxy]-4-chlor-6-methyl-phenyl}-1-(4-methoxybenzyl)-tetrazol-5-carboxamid das N-{3-[3-(4-Acetyl-3-hydroxy-2-n-propyl-phenoxy)-propyloxy]-4-chlor-6-methyl-phenyl}-1H-tetrazol-5-carboxamid vom Smp. 234-235° (Zersetzung) und dessen Triäthanolammoniumsalz vom Smp. 108-110°, sowie ausgehend von N-{3-[3-(4-Acetyl-3-hydroxy-2-n-propyl-phenoxy)-propyloxy]-4-methyl-6-brom-phenyl}-1-(4-methoxybenzyl)-tetrazol-5-carboxamid das N-{3-[3-(4-Acetyl-3-hydroxy-2-n-propyl-phenoxy)-propyloxy]-4-methyl-6-brom-phenyl}-1H-tetrazol-5-carboxamid vom Smp. 212-214° und dessen Triäthanolammoniumsalz vom Smp. 129-130°.

Die Ausgangsmaterialien erhält man beispielsweise wie folgt :

Zu einer Suspension von 6.3 g (23,2 mMol) Kalium-{1-(4-methoxybenzyl)-tetrazol}-5-carboxylat in 70 ml Benzol und 1,0 ml Pyridin gibt man bei 0-5° 2.03 ml (23.2 mMol) Oxalylchlorid und lässt 30 Minuten bei Raumtemperatur rühren. Das Reaktionsgemisch wird unter vermindertem Druck eingeengt, der Rückstand in Benzol aufgenommen und erneut unter vermindertem Druck eingedampft. Der Rückstand wird in 80 ml Methylenchlorid gelöst und bei 0-5° innerhalb von etwa 10 Minuten zu einer Lösung von 7,5 g (17,2 mMol) 3-[3-(4-Acetyl-3-hydroxy-2-n-propyl-phenoxy)-propyloxy]-4-methyl-6-chlor-anilin (Beispiel 12) und 1,87 ml (23.2 mMol) Pyridin in 40 ml Methylenchlorid zugetropft. Anschliessend wird 3 Stunden bei Raumtemperatur gerührt. Das Reaktionsgemisch wird mit Methylenchlorid verdünnt und dreimal mit Wasser gewaschen. Die organischen Phasen werden vereinigt, über Natriumsulfat getrocknet und unter vermindertem Druck eingedampft. Kristallisation des Rückstandes aus Methylenchlorid/Aether liefert das N-{3-[3-(4-Acetyl-3-hydroxy-2-n-propyl-phenoxy)-propyloxy]-4-methyl-6-chlor-phenyl}-1-(4-methoxybenzyl)-tetrazol-5-carboxamid vom Smp. 132°.

In analoger Weise erhält man ausgehend von 3-[3-(4-Acetyl-3-hydroxy-2-n-propyl-phenoxy)-propyloxy]-4-chlor-6-methyl-anilin (Beispiel 12) das N-{3-[3-(4-Acetyl-3-hydroxy-2-n-propyl-phenoxy)-propyloxy]-4-chlor-6-methyl-phenyl}-1-(4-methoxybenzyl)-tetrazol-5-carboxyamid vom Smp. 137-139°, sowie ausgehend von 3-[3-(4-Acetyl-3-hydroxy-2-n-propyl-phenoxy)-propyloxy]-4-methyl-6-brom-anilin (Beispiel 12) das N-{3-[3-(4-Acetyl-3-hydroxy-2-n-propyl-phenoxy)-propyloxy]-4-methyl-6-brom-phenyl}-1-(4-methoxybenzyl)-tetrazol-5-carboxamid vom Smp. 122-123°.

Beispiel 15

In analoger Weise wie in Beispiel 8 und 9 beschrieben erhält man ausgehend von 3-[3-(4-Acetyl-3-hydroxy-2-n-propyl-phenoxy)-propyloxy]-2-cyano-anilin durch Umsetzung mit Chloroxalsäureäthylester den N-{3-[3-(4-Acetyl-3-hydroxy-2-n-propyl-phenoxy)-propyloxy]-2-cyano-phenyl}-oxaminsäureäthylester vom Smp. 158-159° und durch Hydrolyse derselben die N-{3-[3-(4-Acetyl-3-hydroxy-2-n-propyl-phenoxy)-propyloxy]-2-cyano-phenyl}-oxaminsäure in Form des Triäthanolammoniumsalzes vom Smp. 79-81°.

Beispiel 16

Zu einer auf —78 °C gekühlten Lösung von 2.2 g N-{3-[3-(4-Acetyl-3-methoxy-2-n-propyl-phenoxy)-propoxy]-phenyl}-oxaminsäureäthylester in 20 ml Methylenchlorid tropft man innerhalb von 5 Minuten 3.0 g Bortribromid hinzu. Dann wird 6 Stunden bei Raumtemperatur gerührt. Unter Kühlung gibt man 5 ml Wasser zu, trennt, die organische Phase ab und dampft unter vermindertem Druck ein. Nach Umkristallisieren aus Methylenchlorid/Aether erhält man den N-{3-[3-(4-Acetyl-3-hydroxy-2-n-propyl-phenoxy)-propoxy]-phenyl}-oxaminsäureäthylester, der durch Hydrolyse zur freien Säure vom Smp. 159-160° charakterisiert werden kann.

Das Ausgangsmaterial kann man z. B. wie folgt herstellen :

Zu einer Lösung von 7.46 g 3-[3-(4-Acetyl-3-hydroxy-2n-propyl-phenoxy)-propyloxy]-nitrobenzol in 75 ml Dimethylformamid gibt man 5 530 mg Natriumhydrid und erwärmt das Reaktionsgemisch auf 40°. Innert 15 Minuten werden 5.7 g Methylidoid zugetropft. Danach hält man die Reaktionsmischung noch 1 Stunde bei 40 °C. Nach Abkühlen giesst man auf verdünnte Salzsäure und extrahiert mit Methylenchlorid

und dampft ein. Nach Umkristallisieren aus Aether/Hexan erhält man das 3-[3-(4-Acetyl-3-methoxy-2-n-propyl-phenoxy)-propyloxy]-nitrobenzol vom Smp. 52-57°.

Eine Lösung von 10 g 3-[3-(4-Acetyl-3-methoxy-2-n-propyl-phenoxy)-propyloxy]-nitrobenzol in 100 ml Tetrahydrofuran wird mit 2.0 g Raney-Nickel versetzt und bei Raumtemperatur hydriert. Der Katalysator wird abfiltriert und mit Tetrahydrofuran gewaschen. Nach Eindampfen des Filtrats unter vermindertem Druck erhält man das 3-[3-(4-Acetyl-3-methoxy-2-n-propyl-phenoxy)-propyloxy]-anilin als farbloses Oel.

Zu einer Lösung von 9.2 g 3-[3-(4-Acetyl-3-methoxy-2-n-propyl-phenoxy)-propyloxy]-anilin und 4.3 ml Triäthanolamin in 90 ml Methylenchlorid tropft man innert 10 min die Lösung von 3.45 ml Chloroxalsäureäthylester in 10 ml Methylenchlorid. Nach 5-stündigem Rühren bei Raumtemperatur giesst man auf Wasser und extrahiert mit Methylenchlorid. Eindampfen der Extrakte und Umkristallisieren des Rückstandes aus Aether liefert den N-{3-[3-(4-Acetyl-3-methoxy-2-n-propyl-phenoxy)-propoxy]-phenyl}-oxaminsäureäthylester vom Smp. 91-92°.

Beispiel 17

Eine Suspension von 2.3 g 2,4-Dihydroxy-3-n-propyl-acetophenon und 1.6 g geglühten Kaliumcarbonates in 40 ml Aethylmethylketon wird mit einer Spatelspitze Kaliumiodid und 3.2 g N-[3-(3-Brompropoxy)-phenyl]-oxaminsäuremethylester versetzt und 8 Stunden zum Rückfluss erhitzt. Das Reaktionsgemisch wird abgekühlt, aus Wasser gegossen und dreimal mit Methylenchlorid extrahiert. Die vereinigten Extrakte werden mit Wasser gewaschen, über Natriumsulfat getrocknet und unter vermindertem Druck zur Trockne eingeengt. Kristallisation des Rückstandes aus Aether liefert den N-{3-[3-(4-Acetyl-3-hydroxy-2-n-propyl-phenoxy)-propoxy]-phenyl}-oxaminsäuremethylester vom Smp. 124-125°.

Das Ausgangsmaterial kann man z. B. wie folgt herstellen :

Zu einer Suspension von 29 g Kaliumcarbonat und 0.5 g Kaliumiodid in 170 ml Aceton gibt man 43 ml 1,3-Dibrompropan und erwärmt zum Rückfluss. Dann tropft man innert 2 Stunden eine Lösung von 19.4 g 3-Nitrophenol zu und erhitzt noch 15 Stunden am Rückfluss. Das Reaktionsgemisch wird heiss filtriert und eingedampft. Nach Chromatographie des Rückstandes an Kieselgel mit Toluol erhält man das 3-(3-Brom-propoxy)-nitrobenzol als hellgelbes Oel.

Eine Lösung von 4 g 3-(3-Brompropoxy)-nitrobenzol in 40 ml Tetrahydrofuran wird mit 1 g Raney-Nickel versetzt und bei Raumtemperatur hydriert. Der Katalysator wird abfiltriert und mit Tetrahydrofuran gewaschen. Nach Eindampfen des Filtrats erhält man das 3-(3-Brompropoxy)-anilin als farbloses Oel.

Zu einer Lösung von 3.5 g 3-(3-Brompropoxy)-anilin und 1,3 ml Pyridin in 40 ml Methylenchlorid tropft man innert 10 Minuten die Lösung von 1.5 ml Oxalsäuremonomethylesterchlorid in 10 ml Methylenchlorid. Nach 2-stündigem Rühren bei Raumtemperatur giesst man auf Wasser und extrahiert mit Methylenchlorid. Die Vereinigten Extrakte werden eingedampft und der Rückstand mit Methylenchlorid/Aethylacetat (10 : 1) an Kieselgel chromatographiert. Das Eluat wird eingedampft und der Rückstand aus Aether/Hexan kristallisiert. Man erhält den N-[3-(3-Brom-propoxy)-phenyl]-oxaminsäuremethylester vom Smp. 90-91°.

Beispiel 18

Eine Suspension von 1.8 g N-(3-Hydroxyphenyl)-oxaminsäuremethylester und 1.38 g geglühtem Kaliumcarbonat in 20 ml Aethylmethylketon wird mit einer Spatelspitze Kaliumiodid und 3.15 g 4-(3-Brompropoxy)-2-hydroxy-3-propyl-acetophenon versetzt und 12 Stunden zum Rückfluss erhitzt. Das Reaktionsgemisch wird abgekühlt, auf Wasser gegossen und dreimal mit Methylenchlorid extrahiert. Die vereinigten Extrakte werden mit Wasser gewaschen, über Natriumsulfat getrocknet und unter vermindertem Druck zur Trockne eingeengt. Kristallisation des Rückstandes aux Methylenchlorid/Aether liefert den N-{3-[3-(4-Acetyl-3-hydroxy-2-n-propyl-phenoxy)-propoxy]-phenyl}-oxaminsäuremethylester vom Smp. 124-125°.

Das Ausgangsmaterial erhält man z. B. wie folgt :

Zu einer Lösung von 2.5 g 3-Aminoanisol und 1,5 ml Pyridin in 40 ml Methylenchlorid tropft man innert 10 Minuten die Lösung von 1.9 ml Chloroxalsäuremethylester in 10 ml Methylenchlorid. Nach 3 stündigem Rühren bei Raumtemperatur giesst man auf Wasser und extrahiert mit Methylenchlorid. Die Extrakte werden über Natriumsulfat getrocknet, eingedampft und aus Aether/Hexan kristallisiert. Man erhält den N-(3-Methoxy-phenyl)-oxaminsäuremethylester.

Zu einer auf —78 °C gekühlten Lösung von 2 g N-(3-Methoxyphenyl)-oxaminsäuremethylester in 20 ml Methylenchlorid tropft man innert 5 Minuten 5 g Bortribromid. Man rührt 5 Stunden bei Raumtemperatur, gibt unter Kühlung 5 ml Wasser zu und trennt die organischen Phase ab. Nach Trocknen über Natriumsulfat und Eindampfen erhält man N-(3-Hydroxyphenyl)-oxaminsäuremethylester.

Beispiel 19

In analoger Weise wie in den Beispielen 1 bis 18 beschrieben erhält man ferner :

N-{3-[3-(4-Acetyl-3-hydroxy-2-n-propyl-phenoxy)-propyloxy]-6-methyl-phenyl}-oxaminsäure, Smp. 178-179°, und deren Triäthanolammoniumsalz ;

N-{3-[3-(4-Acetyl-3-hydroxy-2-n-propyl-phenoxy)-propyloxy]-phenyl}-oxaminsäure, Smp. 159-160°, und deren Triäthanolammoniumsalz ;

N-{3-[3-(4-Acetyl-3-hydroxy-2-propyl-phenoxy)-propyloxy]-4-methoxy-carbonyl-phenyl}-oxaminsäure und deren Triäthanolammoniumsalz vom Smp. 90-100 sowie

N-{3-[3-(4-Acetyl-3-hydroxy-2-propyl-phenoxy)-propyloxy]-4-carboxy-phenyl}-oxaminsäure und deren Mono- und Dinatriumsalz.

Beispiel 20

Tabletten, enthaltend 25 mg Wirkstoff, z. B. Triäthanolammoniumsalz von N-{3-[3-(4-Acetyl-3-hydroxy-2-propyl-phenoxy)-propyloxy]-6-methyl-phenyl}-oxaminsäure, können folgendermassen hergestellt werden :

| Bestandteile (für 1 000 Tabletten) : | |
|---|---|
| Wirkstoff | 25,0 g |
| Lactose | 100,7 g |
| Weizstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung

Sämtliche festen Ingredienzien werden zunächst durch ein Sieb mit 0,6 mm Maschenweite getrieben. Dann wird der Wirkstoff, die Lactose, das Talkum, das Magnesiumsteart und die Hälfte der Stärke vermischt. Die andere Hälfte der stärke wird in 40 ml Wasser suspendiert und diese Suspension zu einer siedenden Lösung des Polyäthylenglykols in 100 ml Wasser hinzugegeben und das Gemisch, wenn nötig unter Hinzufügen von Wasser, granuliert. Das Granulat wird über Nacht bei 35° getrocknet durch ein Sieb mit 1,2 mm Maschenweite getrieben und zu beidseitig konkaven Tabletten von etwa 6 mm Durchmesser verpresst.

In analoger Weise können auch Tabletten, enthaltend jeweils 25 mg einer anderen der in den Beispielen 1 bis 19 genannten Verbindungen der Formel I hergestellt werden, wobei Verbindungen, worin $R_2$ Carboxy oder 5-Tetrazolyl ist, in Form von Salzen mit Basen, z. B. als Natriumsalz oder Triäthanolammoniumsalz, oder in freier Form vorliegen können.

Beispiel 21

Kautabletten, enthaltend 30 mg Wirkstoff, z. B. Triäthanolammoniumsalz von N-{3-[3-(4-Acetyl-3-hydroxy-2-propyl-phenoxy)-propyloxy]-6-methyl-phenyl}-oxaminsäure, können z. B. folgendermassen hergestellt werden :

| Zusammensetzung (für 1 000 Tabletten) : | |
|---|---|
| Wirkstoff | 30,0 g |
| Mannit | 267,0 g |
| Lactose | 179,5 g |
| Talkum | 20,0 g |
| Glycin | 12,5 g |
| Stearinsäure | 10,0 g |
| Saccharin | 1,0 g |
| 5 %-ige Gelatinelösung | q.s. |

Herstellung

Alle festen Ingredienzien werden zunächst durch ein Sieb mit 0,25 mm Maschenweite getrieben. Der Mannit und die Lactose werden gemischt, unter Hinzufügen von Gelatinelösung granuliert, durch ein Sieb mit 2 mm Maschenweite getrieben, bei 50° getrocknet und nochmals durch ein Sieb mit 1,7 mm Maschenweite getrieben. Der Wirkstoff, das Glycin und das Saccharin werden sorgfältig vermischt, der Manitt, das Lactosegranulat, die Stearinsäure und das Talkum hinzugegeben, das Ganze gründlich vermisch und zu beidseitig konkaven Tabletten von etwa 10 mm Durchmesser mit Bruchrille auf der Oberseite verpresst.

In analoger Weise können auch Tabletten enthaltend jeweils 30 mg einer anderen der in den Beispielen 1 bis 19 genannten Verbindungen der Formel I hergestellt werden, wobei Verbindungen, worin $R_2$ Carboxy oder 5-Tetrazolyl ist, in freier from oder in Form von Salzen mit Basen, z. B. als Natriumsalz oder Triäthanolammoniumsalz, vorliegen können.

Beispiel 22

Tabletten enthaltend 100 mg Wirkstoff, z. B. Triäthanolammoniumsalz von N-{3-[3-(4-Acetyl-3-hydroxy-2-propyl-phenoxy)-propyloxy]-6-methyl-phenyl}-oxaminsäure, können folgendermassen hergestellt werden :

| Zusammensetzung (für 1000 Tabletten) : | |
|---|---|
| Wirkstoff | 100,0 g |
| Lactose | 248,5 g |
| Maisstärke | 17,5 g |
| Polyäthylenglykol 6000 | 5,0 g |
| Talkum | 15,0 g |
| Magnesiumstearat | 4,0 g |
| entmineralisiertes Wasser | q.s. |

Herstellung

Die festen Ingredienzien werden zunächst durch ein Sieb mit 0,6 mm Maschenweite getrieben. Dann werden Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyäthylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von etwa 10 mm Durchmesser mit Bruchkerbe auf der Oberseite verpresst.

In analoger Weise können auch Tabletten, enthaltend 100 mg einer anderen Verbindung der Formel I gemäss den Beispielen 1 bis 19 hergestellt werden, wobei Verbindungen, worin $R_2$ Carboxy oder 5-Tetrazolyl ist, in freier Form oder in Form von Salzen mit Basen, z. B. als Natriumsalz oder Triäthanolammoniumsalz, vorliegen können.

Beispiel 23

Eine treibmittelhaltige, feststoffaerosolbildende Inhalationssuspension enthaltend 0,1 Gew.-% N-{3-[3-(4-Acetyl-3-hydroxy-2-propyl-phenoxy)-propyloxy]-6-methyl-phenyl}-oxaminsäure (Wirkstoff) kann z. B. folgendermassen hergestellt werden :

| Zusammensetzung : | |
|---|---|
| Wirkstoff, mikronisiert | 0,1 Gew.-% |
| « Sorbitantrioleate » | 0,5 Gew.-% |
| Treibmittel A (Trichlortrifluoräthan) | 4,4 Gew.-% |
| Treibmittel B (Gemisch aus 15 Teilen Dichlordifluormethan und 80 Teilen symm. Dichlortetrafluoräthan) | q.s. |

Herstellung

Der Wirkstoff wird unter Feuchtigkeitsausschluss mit Hilfe eines üblichen Homogenisators unter Zusatz des Sorbitantrioleats in dem Trichlortrifluoräthan suspendiert, die Suspension in einen Dosieraerosolbehälter abgefüllt, dieser verschlossen und unter Druck mit dem Dichlordifluormethan-Dichlortetrafluoräthan-Gemisch aufgefüllt.

In analoger Weise können auch Inhalationssuspensionen enthaltend eine andere Verbindung der Formel I gemäss den Beispielen 1 bis 19 hergestellt werden, wobei Verbindungen, worin $R_2$ Carboxy oder 5-Tetrazolyl ist, in freier Form oder in Form von Salzen mit Basen, z. B. als Natriumsalz oder Triäthanolammoniumsalz, vorliegen können.

Beispiel 24

Eine zur Inhalation geeignete, etwa 2 %-ige wässrige Lösung eines Triäthanolammoniumsalzes von N-{3-[3-(4-Acetyl-3-hydroxy-2-propyl-phenoxy)-propyloxy]-6-methylphenyl}-oxaminsäure als Wirkstoff kann z. B. in folgender Zusammensetzung hergestellt werden :

| Zusammensetzung | |
|---|---|
| Wirkstoff | 2 000 mg |
| Stabilisator, z. B. Ethylendiamintetra- | |

| | |
|---|---|
| essigsäure-dinatriumsalz | 10 mg |
| Konservierungsmittel, z. B. Benzalkoniumchlorid | 10 mg |
| Wasser, frisch destilliert | ad 100 ml |

Herstellung

Der Wirkstoff wird in frisch destilliertem Wasser gelöst. Dann wird der Stabilisator und das Konservierungsmittel hinzugegeben. Nach vollständiger Auflösung aller Komponenten wird die erhaltene Lösung auf 100 ml aufgefüllt, in Fläschchen abgefüllt und diese gasdicht verschlossen.

In analoger Weise können auch 2 %-ige Inhalationslösungen enthaltend einen anderen Wirkstoff eines der Beispiele 1 bis 19 hergestellt werden.

## Beispiel 25

Zur Insufflation geeignete, etwa 25 mg Triäthanolammoniumsalz von N-{3-[3-(4-Acetyl-3-hydroxy-2-propyl-phenoxy)-propyloxy]-6-methylphenyl}-oxaminsäure als Wirkstoff enthaltende Kapseln können z. B. in folgender Zusammensetzung hergestellt werden :

Zusammensetzung

| | |
|---|---|
| Wirkstoff | 25 g |
| Lactose, fein gemahlen | 25 g |

Herstellung

Der Wirkstoff und die Lactose werden innig vermischt. Das erhaltene Pulver wird sodann gesiebt und in Portionen zu je 50 mg in 1 000 Gelatinekapseln abgefüllt.

In analoger Weise können auch Insufflationskapseln enthaltend jeweils einen Wirkstoff gemäss einem der Beispiele 1 bis 19 hergestellt werden.

**Patentansprüche** (für die Vertragsstaaten : BE, CH, DE, FR, GB, IT, LI, LU, NL, SE)

1. Neue 4-Acylresorcinäther der Formel

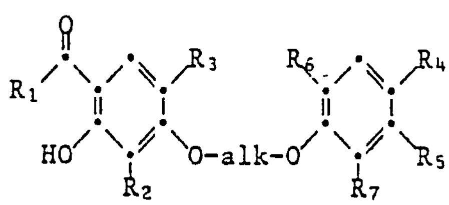

(I)

worin $R_1$ Niederalkyl bedeutet, $R_2$ Niederalkyl, Niederalkenyl oder Niederalkinyl darstellt, $R_3$ Wasserstoff, Niederalkoxy, Trifluormethyl oder Halogen bedeutet, alk einen Alkylenrest mit 2 bis 9 Kettengliedern darstellt, einer der Reste $R_4$, $R_5$ und $R_7$ für eine Gruppe der Formel $—NH—C(=O)—R_8$, ein davon verschiedener Rest $R_4$ oder $R_5$ für einen Rest $R_9$ und ein davon verschiedener Rest $R_7$ für einen Rest $R_{10}$ steht, $R_6$ Wasserstoff, Niederalkyl, Halogen, Trifluormehtyl, gegebenenfalls verestertes oder amidiertes Carboxy, Cyano oder Niederalkanoyl darstellt, $R_8$ gegebenenfalls verestertes oder amidiertes Carboxy oder 5-Tetrazolyl bedeutet, $R_9$ Wasserstoff, Niederalkyl, Niederalkoxy, Halogen oder Trifluormethyl bedeutet und $R_{10}$ für Wasserstoff, Niederalkyl, Niederalkoxy, Halogen, Trifluormethyl, Cyano oder gegebenenfalls verestertes oder amidiertes Carboxy steht und « niedere » organische Gruppen bis und mit 7 Kohlenstoffatome aufweisen, mit der Massgabe, dass in Verbindungen der Formel I, worin $R_4$ eine Gruppe $—NH—(C=O)—R_8$ und $R_8$ 5-Tetrazolyl bedeutet, mindestens einer der Reste $R_3$, $R_6$, $R_9$ und $R_{10}$ von Wasserstoff verschieden ist, und ihre Salze.

2. Verbindungen gemäss Anspruch 1 der Formeln

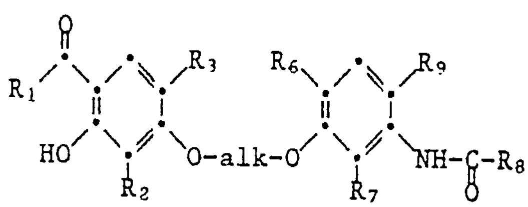

(I')

bzw.

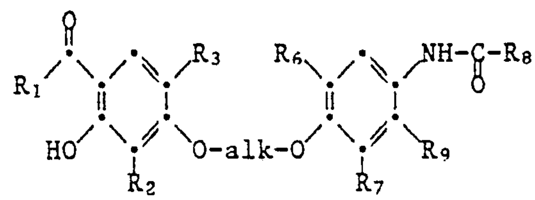

(I'')

worin $R_1$ für Niederalkyl mit bis und mit 4 C-Atomen steht, $R_8$ einerseits Carboxy, Niederalkoxycarbonyl mit bis und mit 4 C-Atomen im Niederalkoxyteil, Diniederalkylaminoniederalkoxycarbonyl mit jeweils bis und mit 4 C-Atomen im Diniederalkylamino- und Niederalkoxyteil, Carbamyl, N-Mono- oder N,N-Diniederalkylcarbamyl, worin Niederalkyl bis und mit 4 C-Atome aufweist, N-(N', N'-Diniederalkylamino-niederalkyl)-carbamyl mit jeweils bis und mit 4 C-Atomen im Diniederalkylamino- und Niederalkylteil, oder andrerseits 5-Tetrazolyl bedeutet, $R_2$ Niederalkyl mit bis und mit 4 C-Atomen darstellt, $R_3$ für Wasserstoff steht, $R_9$ für Wasserstoff, Niederalkyl mit bis und mit 4 C-Atomen, Halogen der Atomnummer 17 bis und mit 53 oder Trifluormethyl steht $R_7$ Wasserstoff, Niederalkyl mit bis und mit 4 C-Atomen, Niederalkoxy mit bis und mit 4 C-Atomen, oder Halogen der Atomnummer bis und mit 35 darstellt und $R_6$ Wasserstoff, Niederalkyl mit bis und mit 4 C-Atomen, Halogen der Atomnummer bis und mit 35, Niederalkoxycarbonyl mit bis und mit 4 C-Atomen im Niederalkoxyteil, Carboxy oder Niederalkanoyl mit bis und mit 7 C-Atomen bedeutet und alk für Niederalkylen mit bis und mit 4 C-Atomen, steht, und ihre Salze, insbesondere pharmazeutisch verwendbaren Salze.

3. Verbindungen gemäss Anspruch 1 der Formel I, worin $R_1$ Niederalkyl mit bis und mit 4 C-Atomen bedeutet, $R_2$ geradkettiges Niederalkyl mit 2 bis und mit 4 C-Atomen bedeutet, $R_3$ Wasserstoff bedeutet und $R_5$ Oxaloamino oder 5-Tetrazolylcarbonylamino darstellt, und worin entweder $R_4$ für Wasserstoff steht und $R_6$ und $R_7$ unabhängig voneinander Wasserstoff oder Niederalkyl mit bis und mit 4 C-Atomen bedeuten bzw. $R_4$ für Niederalkyl mit bis und mit 4 C-Atomen oder Trifluormethyl steht, $R_6$ Wasserstoff oder Halogen der Atomnummer bis und mit 35 darstellt und $R_7$ Wasserstoff ist bzw. $R_4$, $R_6$ und $R_7$ gleiche oder verschiedene Halogenatome der Atomnummer bis und mit 35 bedeuten, und alk geradkettiges, terminal gebundenes Niederalkylen mit 2 bis und mit 4 C-Atomen darstellt, und ihre Salze, insbesondere pharmazeutisch verwendbaren Salze mit Basen.

4. Verbindungen gemäss Anspruch 1 der Formel I, worin $R_1$ Niederalkyl mit bis und mit 4 C-Atomen bedeutet, $R_2$ geradkettiges Niederalkyl mit bis und mit 4 C-Atomen bedeutet, $R_3$ und $R_7$ Wasserstoff stehen, einer der Reste $R_4$ und $R_6$ Niederalkyl mit bis und mit 4 C-Atomen und der andere Halogen der Atomnummer bis und mit 35 bedeutet, $R_5$ Oxaloamino oder 5-Tetrazolylcarbonylamino ist und alk geradkettiges, terminal gebundenes Niederalkylen mit bis und mit 7 C-Atomen darstellt, und ihre Salze, insbesondere pharmazeutisch verwendbaren Salze, mit Basen.

5. N-{3-[3-(4-Acetyl-3-hydroxy-2-n-propyl-phenoxy)-propyloxy]-6-methyl-phenyl}-oxaminsäure oder ein Salz davon, N-{3-[3-(4-Acetyl-3-hydroxy-2-n-propyl-phenoxy)-propyloxy]-4-brom-6-methyl-phenyl}-oxaminsäure oder ein Salz davon, N-{3-[3-(4-Acetyl-3-hydroxy-2-n-propyl-phenoxy)-propyloxy]-2,4,6-trichlor-phenyl}-oxaminsäure oder ein Salz davon oder N-{3-[3-(4-Acetyl-3-hydroxy-2-n-propyl-phenoxy)-propyloxy]-phenyl}-tetrazol-5-carboxamid oder ein Salz davon gemäss Anspruch 1.

6. N-{3-[3-(4-Acetyl-3-hydroxy-2-n-propyl-phenoxy)-propyloxy]-phenyl}-oxaminsäuremethylester, N-{3-[3-(4-Acetyl-3-hydroxy-2-n-propyl-phenoxy)-propyloxy]-phenyl}-oxaminsäure oder ein Salz davon, N-{3-[3-(4-Acetyl-3-hydroxy-2-n-propyl-phenoxy)-propyloxy]-4-brom-6-methyl-phenyl}-oxaminsäuremethylester, N-{3-[3-(4-Acetyl-3-hydroxy-2-n-propyl-phenoxy)-propyloxy]-6-methyl-phenyl}-oxaminsäuremethylester, N-{3-[3-(4-Acetyl-3-hydroxy-2-n-propyl-phenoxy)-propyloxy]-2-methyl-phenyl}-oxaminsäuremethylester, N-{3-[3-(4-Acetyl-3-hydroxy-2-n-propyl-phenoxy)-propyloxy]-2-methyl-phenyl}-oxaminsäure oder ein Salz davon, N-{3-[5-(4-Acetyl-3-hydroxy-2-n-propyl-phenoxy)-pentyloxy]-phenyl}-oxaminsäuremethylester, N-{3-[3-(4-Acetyl-3-hydroxy-2-n-propyl-phenoxy)-propyloxy]-4-chlor-6-trifluormethyl-phenyl}-oxaminsäuremethylester, N-{3-[3-(4-Acetyl-3-hydroxy-2-n-propyl-phenoxy)-propyloxy-4-chlor-6-trifluormethyl-phenyl}-oxaminsäure oder ein Salz davon, N-{3-[3-(4-Acetyl-3-hydroxy-2-n-propyl-phenoxy)-propyloxy]-2,4,6-trichlor-phenyl}-oxaminsäuremethylester,N-{3-[3-(4-Acetyl-3-hydroxy-2-n-propyl-phenoxy)-propyloxy]-4,6-dimethyl-phenyl}-oxaminsäuremethylester, N-{3-[3-(4-Acetyl-3-hydroxy-2-n-propyl-phenoxy)-propyloxy]-4,6-dimethyl-phenyl}-oxaminsäure oder ein Salz davon, N-{4-[3-(4-Acetyl-3-hydroxy-2-n-propyl-phenoxy)-propyloxy]-3-methoxy-phenyl}-oxaminsäuremethylester, N-{4-[3-(4-Acetyl-3-hydroxy-2-n-propyl-phenoxy)-propyloxy]-2-methyl-phenyl}-oxaminsäuremethylester, N-{4-[3-(4-Acetyl-3-hydroxy-2-n-propyl-phenoxy)-propyloxy]-2-methyl-phenyl}-oxaminsäure oder ein Salz davon, N-{4-[3-(4-Acetyl-3-hydroxy-2-n-propyl-phenoxy)-propyloxy]-phenyl}-oxaminsäuremethylester, N-{4-[3-(4-Acetyl-3-hydroxy-2-n-propyl-phenoxy)-propyloxy-phenyl}-oxaminsäure oder ein Salz davon, N-{3-[5-(4-Acetyl-3-hydroxy-2-n-propyl-phenoxy)-pentyloxy]-phenyl} oxaminsäure oder ein Salz davon, N-(3-[3-(4-Acetyl-3-hydroxy-2-n-propyl-phenoxy)-propyloxy]-4-brom-6-methylphenyl}-tetrazol-5-carboxamid oder ein Salz davon, N-{3-[5-(4-Acetyl-3-hydroxy-2-n-propyl-phenoxy)-pentyloxy]-phenyl}-oxaminsäure oder ein Salz davon, oder N-{3-[3-(4-Acetyl-3-hydroxy-2-n-propyl-phenoxy)-propyloxy]-4-carboxy-phenyl}-oxaminsäure oder ein saures oder neutrales Salz davon gemäss Anspruch 1.

7. N-{3-[3-(4-Acetyl-3-hydroxy-2-n-propyl-phenoxy)-propyloxy]-4-brom-6-methyl-phenyl}-oxaminsäureäthylester gemäss Anspruch 1.

8. N-{3-[3-(4-Acetyl-3-hydroxy-2-n-propyl-phenoxy)-propyloxy]-6-brom-4-methyl-phenyl}-1H-tetrazol-5-carboxamid oder ein Salz davon, N-{3-[3-(4-Acetyl-3-hydroxy-2-n-propyl-phenoxy)-propyloxy]-2-cyano-phenyl}-oxaminsäure oder ein Salz davon, N-{3-[3-(4-Acetyl-3-hydroxy-2-n-propyl-phenoxy)-propyloxy]-4-chlor-6-methyl-phenyl}-oxaminsäure oder ein Salz davon, N-{3-[3-(4-Acetyl-3-hydroxy-2-n-propyl-phenoxy)-propyloxy]-6-chlor-4-methyl-phenyl}-tetrazol-5-carboxamid oder ein Salz davon oder N-{3-[3-(4-Acetyl-3-hydroxy-2-n-propyl-phenoxy)-propyloxy]-4-chlor-6-methyl-phenyl}-1H-tetrazol-5-carboxamid oder ein Salz davon gemäss Anspruch 1.

9. N-{3-[3-(4-Acetyl-3-hydroxy-2-n-propyl-phenoxy)-propyloxy-2-cyano-phenyl}-1H-tetrazol-5-carboxamid oder ein Salz davon, N-{3-[3-(4-Acetyl-3-hydroxy-2-n-propyl-phenoxy)-propyloxy]-6-chlor-4-methyl-phenyl}-oxaminsäuremethylester, N-{3-[3-(4-Acetyl-3-hydroxy-2-n-propyl-phenoxy)-propyloxy]-4-chlor-6-methyl-phenyl}-oxaminsäuremethylester, N-{3-[3-(4-Acetyl-3-hydroxy-2-n-propyl-phenoxy)-propyloxy]-6-brom-4-methyl-phenyl}-oxaminsäuremethylester, N-{3-[3-(4-Acetyl-3-hydroxy-2-n-propyl-phenoxy)-propyloxy]-6-chlor-4-methyl-phenyl}-oxaminsäure oder ein Salz davon oder N-{-3-[3-(4-Acetyl-3-hydroxy-2-n-propyl-phenoxy)-propyloxy]-2-cyano-phenyl}-oxyaminsäureäthylester gemäss Anspruch 1.

10. Eine salzbildende Verbindung gemäss einem der Ansprüche 4-6 in Form des Triäthanolammoniumsalzes.

11. Eine salzbildende Verbindung gemäss einem der Ansprüche 1-3 und 7-9 in Form des Triäthanolammoniumsalzes.

12. Eine salzbildende Verbindung gemäss Anspruch 4 oder N-{3-[3-(4-Acetyl-3-hydroxy-2-n-propyl-phenoxy)-propyloxy]-6-methyl-phenyl}-oxaminsäure, N-{3-[3-(4-Acetyl-3-hydroxy-2-n-propyl-phenoxy)-propyloxy]-4-brom-6-methyl-phenyl}-oxaminsäure, N-{3-[5-(4-Acetyl-3-hydroxy-2-n-propyl-phenoxy)-pentyloxy]-phenyl}oxaminsäure oder N-{3-[3-(4-Acetyl-3-hydroxy-2-n-propyl-phenoxy)-propyloxy]-phenyl}-oxaminsäure in Form des Natriumsalzes.

13. Eine Verbindung gemäss einem der Ansprüche 1 bis 12 zur Anwendung in einem Verfahren zur therapeutischen Behandlung des menschlichen oder tierischen Körpers.

14. Eine Verbindung gemäss einem der Ansprüche 1 bis 12 zur Anwendung gemäss Anspruch 12 als Antiallergikum, Antiasthmatikum, Antiinflammatorikum und/oder Antiphogistikum.

15. Pharmazeutische Präparate enthaltend eine Verbindung gemäss einem der Ansprüche 1, 2, 8, 9 und 11-14 neben üblichen Hilfs- und Trägerstoffen.

16. Pharmazeutische Präparate enthaltend eine Verbindung gemäss einem der Ansprüche 3 und 7 neben üblichen Hilfs- und Trägerstoffen.

17. Pharmazeutische Präparate enthaltend eine Verbindung gemäss einem der Ansprüche 4-6 und 10 neben üblichen Hilfs- und Trägerstoffen.

18. Verwendung einer Verbindung gemäss einem der Ansprüche 1 bis 12 zur Herstellung allergischer und/oder entzündungshemmender Arzneimittel.

19. Verfahren zur Herstellung neuer 4-Acylresorcinäther der Formel

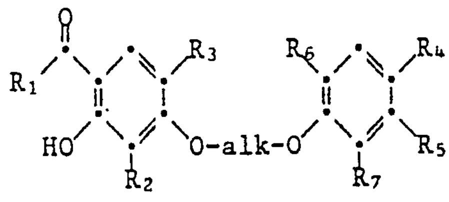

(I)

worin $R_1$ Niederalkyl bedeutet, $R_2$ Niederalkyl, Niederalkenyl oder Niederalkinyl darstellt, $R_3$ Wasserstoff, Niederalkoxy, Trifluormethyl oder Halogen bedeutet, alk einen Alkylenrest mit 2 bis 9 Kettengliedern darstellt, einer der Reste $R_4$, $R_5$ und $R_7$ für eine Gruppe der Formel —NH—C(=O)—$R_8$, ein davon verschiedener Rest $R_4$ oder $R_5$ für einen $R_9$ und ein davon verschiedener Rest $R_7$ für einen Rest $R_{10}$ steht, $R_6$ Wasserstoff, Niederalkyl, Trifluormethyl, Halogen, gegebenenfalls verestertes oder amidiertes Carboxy, Cyano oder Niederalkanoyl darstellt $R_8$ gegebenenfalls verestertes oder amidiertes Carboxy oder 5-Tetrazolyl bedeutet, $R_9$ Wasserstoff, Niederalkyl, Niederalkoxy, Halogen oder Trifluormethyl bedeutet, und $R_{10}$ für Wasserstoff, Niederalkyl, Niederalkoxy, Halogen, Trifluormethyl, Cyano oder gegebenenfalls verestertes oder amidiertes Carboxy steht, mit der Massgabe, dass in Verbindungen der Formel I, worin $R_4$ eine Gruppe —NH—(C=O)—$R_8$ 5-Tetrazolyl bedeutet, mindestens einer der Reste $R_3$, $R_6$, $R_9$ und $R_{10}$ von Wasserstoff verschieden ist, und ihre Salze, dadurch gekennzeichnet, dass man

a) in einer Verbindung der Formel

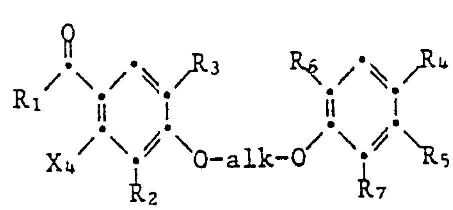

(VI)

worin $X_4$ einen in Hydroxy überführbaren Rest bedeutet, $X_4$ in Hydroxy überführt oder

b) Verbindungen der Formeln

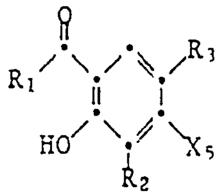 (VII) und 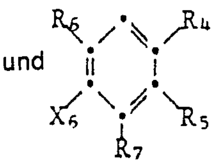 (VIII)

miteinander umsetzt, worin einer der Reste $X_5$ und $X_6$ gegebenenfalls in Salzform vorliegendes Hydroxy und der andere einen durch reaktionsfähiges verestertes Hydroxy substituierten Rest —O-alkH, d. h. einen durch reaktionsfähiges verestertes Hydroxy substituierten Alkoxyrest, darstellt, oder

c) eine Verbindung der Formel

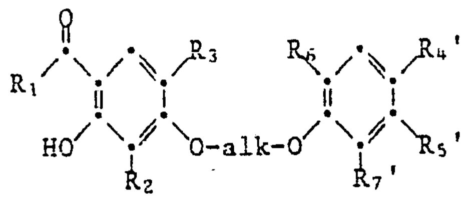 (X)

worin einer der Reste $R_4'$ $R_5'$ und $R_7'$ die Aminogruppe, ein davon verschiedener Rest $R_4'$ bzw. $R_5'$ einen Rest $R_9$ und ein davon verschiedener Rest $R_7'$ einen Rest $R_{10}$ darstellt, oder ein Salz davon mit einer Verbindung der Formel

$$X_7—R_8' \qquad (XI)$$

umsetzt, worin $R_8'$ eine gegebenenfalls veresterte oder amidierte Carboxygruppe oder gegebenenfalls in 1-Stellung geschütztes 5-Tetrazolyl und $X_7$ eine gegebenenfalls veresterte, amidierte, anhydridisierte oder, sofern $R_8'$ für in 1-Stellung geschütztes 5-Tetrazolyl steht, in Salzform vorliegende Carboxygruppe bedeutet, und gegebenenfalls die Schutzgruppe in 1-Stellung einer Tetrazolylgruppe $R_8$ abspaltet oder

d) in einer Verbindung der Formel

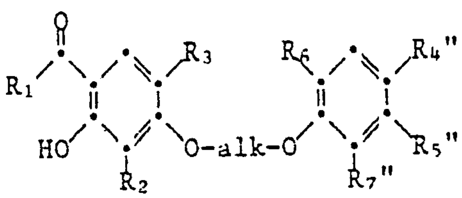 (XII)

worin einer der Reste $R_4''$, $R_5''$ und $R_7''$ einen Rest $X_8$, ein davon verschiedener Rest $R_4''$ bzw. $R_5''$ einen Rest $R_9$ und ein davon verschiedner Rest $R_7''$ einen Rest $R_{10}$ darstellt und $X_8$ einen in die gewünschte Gruppe der Formel —NH—C(=O)—$R_8$ überführbaren Rest bedeutet, $X_8$ in diese überführt und gewünschtenfalls jeweils eine verfahrensgemäss erhältliche Verbindung in eine andere Verbindung der Formel I überführt, ein verfahrensgemäss erhältliches Isomerengemisch auftrennt und das oder die gewünschte(n) Isomere(n) isoliert und/oder eine verfahrensgemäss erhältliche freie Verbindung in ein Salz oder ein verfahrensgemäss erhältliches Salz in die freie Verbindung oder in ein anderes Salz umwandelt.

**Patentansprüche** (für den Vertragsstaat AT)

1. Verfahren zur Herstellung neuer 4-Acylresorcinäther der Formel

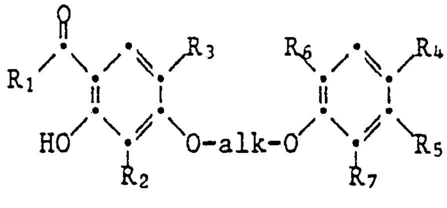 (I)

worin $R_1$ Niederalkyl bedeutet, $R_2$ Niederalkyl, Niederalkenyl oder Niederalkinyl darstellt, $R_3$ Wasserstoff, Niederalkoxy, Trifluormethyl oder Halogen bedeutet, alk einen Alkylenrest mit 2 bis 9 Kettengliedern darstellt, einer der Reste $R_4$, $R_5$ und $R_7$ für eine Gruppe der Formel —NH—C(=O)—$R_8$, ein davon verschiedener Rest $R_4$ oder $R_5$ für einen Rest $R_9$ und ein davon verschiedener Rest $R_7$ für einen Rest $R_{10}$ steht, $R_6$ Wasserstoff, Niederalkyl, Halogen, Trifluormethyl, gegebenenfalls verestertes oder amidiertes Carboxy, Cyano oder Niederalkanoyl darstellt $R_8$ gegebenenfalls verestertes oder amidiertes Carboxy oder 5-Tetrazolyl bedeutet, $R_9$ Wasserstoff, Niederalkyl, Niederalkoxy, Halogen oder Trifluormethyl bedeutet, und $R_{10}$ für Wasserstoff, Niederalkyl, Niederalkoxy, Halogen, Trifluormethyl, Cyano oder gegebenenfalls verestertes oder amidiertes Carboxy steht und « niedere » organische Gruppen bis und mit 7 Kohlenstoffatome aufweisen, mit der Massgabe, dass in Verbindungen der Formel I, worin $R_4$ eine Gruppe —NH(C=O)—$R_8$ und $R_8$ 5-Tetrazolyl bedeutet, mindestens einer der Reste $R_3$, $R_6$, $R_9$ und $R_{10}$ von Wasserstoff verschieden ist, und ihre Salze, dadurch gekennzeichnet, dass man

a) in einer Verbindung der Formel

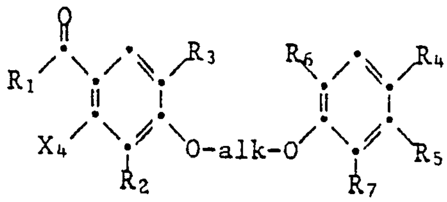

(VI)

worin $X_4$ einen in Hydroxy überführbaren Rest bedeutet, $X_4$ in Hydroxy überführt oder

b) Verbindungen der Formeln

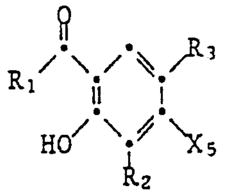

(VII) und

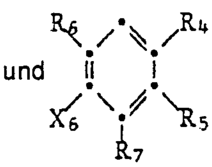

(VIII)

miteinander umsetzt, worin einer der Reste $X_5$ und $X_6$ gegebenenfalls in Salzform vorliegendes Hydroxy und der andere einen durch reaktionsfähiges verestertes Hydroxy substituierten Rest —O-alkH, d. h. einen durch reaktionsfähiges verestertes Hydroxy substituierten Alkoxyrest, darstellt, oder

c) eine Verbindung der Formel

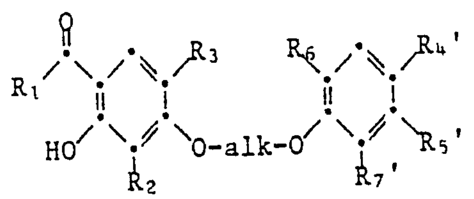

(X)

worin einer der Reste $R_4'$ $R_5'$ und $R_7'$ die Aminogruppe, ein davon verschiedener Rest $R_4'$ bzw. $R_5'$ einen Rest $R_9$ und ein davon verschiedener Rest $R_7'$ einen Rest $R_{10}$ darstellt, oder ein Salz davon mit einer Verbindung der Formel

$$X_7—R_8' \quad (XI)$$

umsetzt, worin $R_8'$ eine gegebenenfalls veresterte oder amidierte Carboxygruppe oder gegebenenfalls in 1-Stellung geschütztes 5-Tetrazolyl und $X_7$ eine gegebenenfalls veresterte, amidierte, anhydridisierte oder, sofern $R_8'$ für in 1-Stellung geschütztes 5-Tetrazolyl steht, in Salzform vorliegende Carboxygruppe bedeutet, und gegebenenfalls die Schutzgruppe in 1-Stellung einer Tetrazolylgruppe $R_8$ abspaltet oder

d) in einer Verbindung der Formel

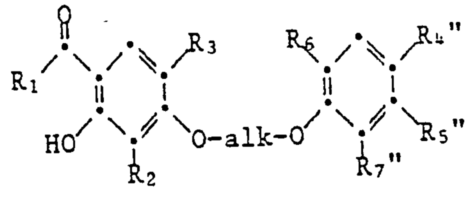

(XII)

worin einer der Reste $R_4''$, $R_5''$ und $R_7''$ einen Rest $X_8$, ein davon verschiedener Rest $R_4''$ bzw. $R_5''$ einen Rest $R_9$ und ein davon verschiedner Rest $R_7''$ einen Rest $R_{10}$ darstellt und $X_8$ einen in die gewünschte Gruppe der Formel —NH—C(=O)—$R_8$ überführbaren Rest bedeutet, $X_8$ in diese überführt und gewünschtenfalls jeweils eine verfahrensgemäss erhältliche Verbindung in eine andere Verbindung der Formel I überführt, ein verfahrensgemäss erhältliches Isomerengemisch auftrennt und das oder die gewünschte(n) Isomere(n) isoliert und/oder eine verfahrensgemäss erhältliche freie Verbindung in ein Salz oder ein verfahrensgemäss erhältliches Salz in die freie Verbindung oder in ein anderes Salz umwandelt.

2. Verfahren gemäss Anspruch 1 zur Herstellung von Verbindungen gemäss Anspruch 1 der Formeln

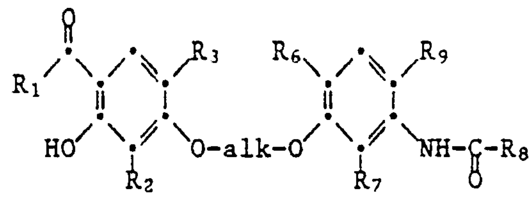

(I')

bzw.

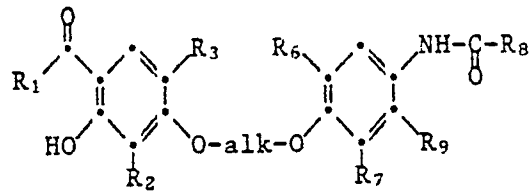

(I'')

worin $R_1$ für Niederalkyl mit bis und mit 4 C-Atomen, wie Methyl, steht, $R_8$ einerseits Carboxy, Niederalkoxycarbonyl mit bis und mit 4 C-Atomen im Niederalkoxyteil, Diniederalkylaminoniederalkoxycarbonyl mit jeweils bis und mit 4 C-Atomen im Diniederalkylamino- und Niederalkoxyteil, Carbamyl, N-Mono- oder N,N-Diniederalkylcarbamyl, worin Niederalkyl bis und mit 4 C-Atome aufweist, N-(N',N'-Diniederalkylaminoniederalkyl)-carbamyl mit jeweils bis und mit 4 C-Atomen im Diniederalkylamino- und Niederalkylteil, oder andrerseits 5-Tetrazolyl bedeutet, $R_2$ Wasserstoff oder Niederalkyl mit bis und mit 4 C-Atomen darstellt, $R_3$ für Wasserstoff, Niederalkyl mit bis und mit 4 C-Atomen, Niederalkoxy mit bis und mit 4 C-Atomen oder Halogen der Atomnummer 17 bis und mit 53 steht, $R_9$ für Wasserstoff, Niederalkyl mit bis und mit 4 C-Atomen, Halogen der Atomnummer 17 bis und mit 53 oder Trifluormethyl steht, $R_7$ Wasserstoff, Niederalkyl mit bis und mit 4 C-Atomen, Niederalkoxy mit bis und mit 4 C-Atomen, oder Halogen der Atomnummer bis und mit 35 darstellt und $R_6$ Wasserstoff, Niederalkyl mit bis und mit 4 C-Atomen, Halogen der Atomnummer bis und mit 35, Niederalkoxycarbonyl mit bis und mit 4 C-Atomen im Niederalkoxyteil, Carboxy oder Niederalkanoyl mit bis und mit 7 C-Atomen bedeutet und alk für Niederalkylen mit bis und mit 4 C-Atomen, steht, und ihrer Salze, insbesondere pharmazeutisch verwendbaren Salze.

3. Verfahren gemäss Anspruch 1 zur Herstellung von Verbindungen gemäss Anspruch 1 der Formel I, worin $R_1$ Niederalkyl mit bis und mit 4 C-Atomen bedeutet, $R_2$ geradkettiges Niederalkylen mit 2 bis und mit 4 C-Atomen bedeutet, $R_3$ Wasserstoff bedeutet und $R_5$ Oxaloamino oder 5-Tetrazolylcarbonylamino darstellt, und worin entweder $R_4$ für Wasserstoff steht und $R_6$ und $R_7$ unabhängig voneinander Wasserstoff oder Niederalkyl mit bis und mit 4 C-Atomen bedeuten bzw. $R_4$ für Niederalkyl mit bis und mit 4 C-Atomen oder Trifluormethyl steht, $R_6$ Wassertoff oder Halogen der Atomnummer bis und mit 35 darstellt und $R_7$ Wasserstoff ist bzw. $R_4$, $R_6$ und $R_7$ gleiche oder verschiedene Halogenatome der Atomnummer bis und mit 35 bedeuten, und alk geradkettiges, terminal gebundenes Niederalkylen mit 2 bis und mit 4 C-Atomen darstellt, und ihre Salze, insbesondere pharmazeutisch verwendbaren Salze mit Basen.

4. Verfahren gemäss Anspruch 1 zur Herstellung von Verbindungen gemäss Anspruch 1 der Formel I, worin $R_1$ Niederalkyl mit bis und mit 4 C-Atomen bedeutet, $R_2$ geradkettiges Niederalkyl mit bis und mit 4 C-Atomen bedeutet, $R_3$ und $R_7$ für Wasserstoff stehen, einer der Reste $R_4$ und $R_6$ Niederalkyl mit bis und mit 4 C-Atomen und der andere Halogen der Atomnummer bis und mit 35 bedeutet, $R_5$ Oxaloamino oder 5-Tetrazolylcarbonylamino ist und alk geradkettiges, terminal gebundenes Niederalkylen mit bis und mit 7 C-Atomen darstellt, und ihre Salze, insbesondere pharmazeutisch verwendbaren Salze, mit Basen.

5. Verfahren gemäss Anspruch 1 zur Herstellung von N-{3-[3-(4-Acetyl-3-hydroxy-2-n-propyl-phenoxy)-propyloxy]-6-methyl-phenyl}-oxaminsäure oder eine Salzes davon, N-{3-[3-(4-Acetyl-3-hydroxy-2-n-propyl-phenoxy)-propyloxy]-4-brom-6-methyl-phenyl}-oxaminsäure oder eines Salzes davon, N-{3-[3-(4-Acetyl-3-hydroxy-2-n-propyl-phenoxy)-propyloxy]-2-cyano-phenyl}-1H-tetrazol-5-carboxamid oder eines Salzes davon, N-{3-[3-(4-Acetyl-3-hydroxy-2-n-propyl-phenoxy)-propyloxy]-6-brom-4-methyl-phenyl}-1H-tetrazol-5-carboxamid oder eines Salzes davon, N-{3-[3-(4-Acetyl-3-hydroxy-2-n-propyl-phenoxy)-propyloxy]-2-cyano-phenyl}-oxaminsäure oder eines Salzes davon, N-{3-[3-(4-Acetyl-3-hydroxy-2-n-propyl-phenoxy)-propyloxy]-2,4,6-trichlor-phenyl}-oxaminsäure oder eines Salzes davon, N-{3-[3-(4-Acetyl-3-hydroxy-2-n-propyl-phenoxy)-propyloxy]-phenyl}-tetrazol-5-carboxamid oder eines Salzes davon, N-{3-[3-(4-Acetyl-3-hydroxy-2-n-propyl-phenoxy)-propyloxy]-6-chlor-4-methyl-phenyl}-oxaminsäure oder eines Salzes davon, N-{3-[3-(4-Acetyl-3-hydroxy-2-n-propyl-phenoxy)-propyloxy]-6-chlor-4-methyl-phenyl}-1H-tetrazol-5-carboxamid oder eines Salzes davon oder N-{3-[3-(4-Acetyl-3-hydroxy-2-n-propyl-phenoxy)-propyloxy]-4-chlor-6-methyl-phenyl}-1H-tetrazol-5-carboxamid oder eines Salzes davon.

6. Verfahren gemäss Anspruch 1 zur Herstellung von N-{3-[5-(4-Acetyl-3-hydroxy-2-n-propylphenoxy)-pentyloxy]-phenyl}-oxaminsäure oder eines Salzes davon, N-{3-[3-(4-Acetyl-3-hydroxy-2-n-propylphenoxy)-propyloxy]-phenyl}-oxaminsäuremethylester, N-{3-[3-(4-Acetyl-3-hydroxy-2-n-propyl-phenoxy)-propyloxy]-phenyl}-oxaminsäure oder eines Salzes davon, N-{3-[3-(4-Acetyl-3-hydroxy-2-n-propyl-phenoxy)-propyloxy]-4-brom-6-methyl-phenyl}-oxaminsäuremethylester, N-{3-[3-(4-Acetyl-3-hydroxy-2-n-propyl-phenoxy)-propyloxy]-6-methyl-phenyl}-oxaminsäuremethylester, N-{3-[3-(4-Acetyl-3-hydroxy-2-n-propyl-phenoxy)-propyloxy]-2-methyl-phenyl}-oxaminsäuremethylester, N-{3-[3-(4-Acetyl-3-hydroxy-2-n-propyl-phenoxy)-propyloxy]-2-methyl-phenyl}-oxaminsäure oder eines Salzes davon, N-{3-[5-(4-Acetyl-3-hydroxy-2-n-propyl-phenoxy)-pentyloxy]-phenyl}-oxaminsäuremethyl-ester, N-{3-[3-(4-Acetyl-3-hydroxy-2-n-propyl-phenoxy)-propyloxy]-4-chlor-6-trifluormethyl-phenyl}-oxaminsäuremethylester, N-{3-[3-(4-Acetyl-3-hydroxy-2-n-propyl-phenoxy)-propyloxy]-4-chlor-6-trifluor-methyl-phenyl}-oxaminsäure oder eines Salzes davon, N-{3-[3-(4-Acetyl-3-hydroxy-2-n-propyl-phenoxy)-propyloxy]-2,4,6-trichlorphenyl}-oxaminsäuremethylester, N-{3-[3-(4-Acetyl-3-hydroxy-2-n-propyl-phenoxy)-propyloxy]-4,6-dimethyl-phenyl}-oxaminsäuremethyl-ester, N-{3-[3-(4-Acetyl-3-hydroxy-2-n-propyl-phenoxy)-propyloxy]-4,6-dimethyl-phenyl}-oxaminsäure oder eines Salzes davon, N-{4-[3-(4-Acetyl-3-hydroxy-2-n-propyl-phenoxy)-propyloxy]-3-methoxy-phenyl}-oxaminsäuremethylester, N-{4-[3-(4-Acetyl-3-hydroxy-2-n-propyl-phenoxy)-propyloxy]-3-methoxy-phenyl}-oxaminsäure oder eines Salzes davon, N-{4-[3-(4-Acetyl-3-hydroxy-2-n-propyl-phenoxy)-propyloxy]-2-methyl-phenyl}-oxaminsäuremethylester, N-{4-[3-(4-Acetyl-3-hydroxy-2-n-propyl-phenoxy)-propyloxy]-2-methyl-phenyl}-oxaminsäure oder eines Salzes davon, N-{2-[3-(4-Acetyl-3-hydroxy-2-n-propyl-phenoxy)-propyloxy]-phenyl}-oxaminsäuremethylester, N-{2-[3-(4-Acetyl-3-hydroxy-2-n-propyl-phenoxy)-propyloxy]-phenyl}-oxaminsäure oder eines Salzes davon, N-{4-[3-(4-Acetyl-3-hydroxy-2-n-propyl-phenoxy)-propyloxy]-phenyl}-oxaminsäuremethylester, N-{4-[3-(4-Acetyl-3-hydroxy-2-n-propyl-phenoxy)-propyloxy]-phenyl}-oxaminsäure oder eines Salzes davon, N-{3-[3-(4-Acetyl-3-hydroxy-2-n-propyl-phenoxy)-propyloxy]-4-brom-6-methylphenyl}-tetrazol-5-carboxamid oder eines Salzes davon, N-{3-[3-(4-Acetyl-3-hydroxy-2-n-propyl-phenoxy)-propyloxy]-4-carboxy-phenyl}-oxaminsäure oder eines sauren oder neutralen Salzes davon, N-{3-[3-(4-Acetyl-3-hydroxy-2-n-propyl-phenoxy)-propyloxy]-4-methoxycarbonyl-phenyl}-oxaminsäure oder eines Salzes davon, N-{3-[3-(4-Acetyl-3-hydroxy-2-n-propyl-phenoxy)-propyloxy]-4-brom-6-methyl-phenyl}-oxaminsäureäthylester, N-{3-[3-(4-Acetyl-3-hydroxy-2-n-propyl-phenoxy)-propyloxy]-6-chlor-4-methyl-phenyl}-oxaminsäuremethylester, N-{3-[3-(4-Acetyl-3-hydroxy-2-n-propyl-phenoxy)-propyloxy]-4-chlor-6-methyl-phenyl}-oxaminsäure-methylester, N-{3-[3-(4-Acetyl-3-hydroxy-2-n-propyl-phenoxy)-propyloxy]-6-brom-4-methyl-phenyl}-oxaminsäuremethylester, N-{3-[3-(4-Acetyl-3-hydroxy-2-n-propyl-phenoxy)-propyloxy]-4-chlor-6-methyl-phenyl}-oxaminsäure oder eines Salzes davon, oder N-{3-[3-(4-Acetyl-3-hydroxy-2-n-propyl-phenoxy)-propyloxy]-2-cyano-phenyl}-oxaminsäureäthylester.

7. Verfahren zur Herstellung einer salzbindenden Verbindung gemäss einem der Ansprüche 1-6 in Form des Triäthanolammoniumsalzes.

8. Verfahren zur Herstellung einer Verbindung gemäss einem der Ansprüche 1-4, oder von N-{3-[3-(4-Acetyl-3-hydroxy-2-n-propyl-phenoxy)-propyloxy]-6-methyl-phenyl}-oxaminsäure N-{3-[3-(4-Acetyl-3-hydroxy-2-n-propyl-phenoxy)-propyloxy]-4-brom-6-methyl-phenyl}-oxaminsäure oder N-{3-[5-(4-Acetyl-3-hydroxy-2-n-propyl-phenoxy)-pentyloxy]-phenyl} oxaminsäure in Form des Natriumsalzes.

9. Verfahren zur Herstellung pharmazeutischer Präparate, dadurch gekennzeichnet, dass man eine gemäss einem der Ansprüche 1-8 erhältliche Verbindung mit üblichen pharmazeutischen Hilfs- und/oder Trägerstoffen vermischt.

10. Verfahren gemäss Anspruch 9 zur Herstellung eines Antiallergikums, Antiasthmatikums, Antiinflammatorikums und/oder Antiphogistikums.

**Claims** (for the Contracting States : BE, CH, DE, FR, GB, IT, LI, LU, NL, SE)

1. Novel 4-acylresorcinol ethers of the formula

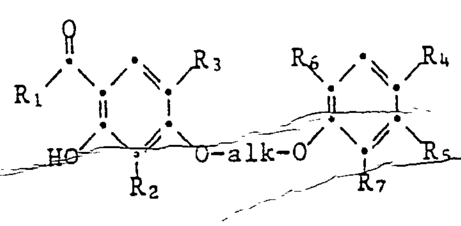

(I)

in which $R_1$ represents lower alkyl, $R_2$ represents lower alkyl, lower alkenyl or lower alkynyl, $R_3$ represents hydrogen, lower alkoxy, trifluoromethyl or halogen, alk represents an alkylene radical having from 2 to 9 chain members, one of the radicals $R_4$, $R_5$ and $R_7$ is a group of the formula —NH—C(=O)—$R_8$, a radical $R_4$ or $R_5$ that is other than a group of the formula —NH—C(=O)—$R_8$ is a radical $R_9$, and a radical $R_7$ that is other than a group of the formula —NH—C(=O)—$R_8$ is a radical $R_{10}$, $R_6$ represents hydrogen, lower alkyl, halogen, trifluoromethyl, optionally esterified or amidated carboxy, cyano or lower alkanoyl, $R_8$ represents optionally esterified or amidated carboxy, or 5-tetrazolyl, $R_9$ represents hydrogen, lower alkyl, lower alkoxy, halogen or trifluoromethyl, and $R_{10}$ represents hydrogen, lower alkyl, lower alkoxy, halogen, trifluoromethyl, cyano or optionally esterified or amidated carboxy, and « lower » organic groups have up to and including 7 carbon atoms, with the proviso that in compounds of the formula I in which $R_4$ represents a group —NH—(C=O)—$R_8$ and $R_8$ represents 5-tetrazolyl, at least one of the radicals $R_3$, $R_6$, $R_9$ and $R_{10}$ is other than hydrogen, and their salts.

2. Compounds as claimed in claim 1 of the formula

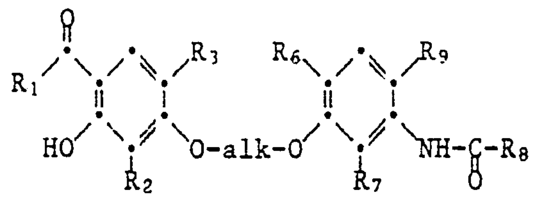

(I')

or

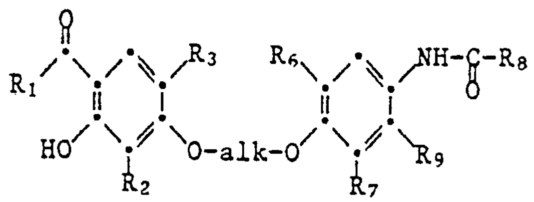

(I'')

in which $R_1$ represents lower alkyl having up to and including 4 carbon atoms, $R_8$ on the one hand represents carboxy, lower alkoxycarbonyl having up to and including 4 carbon atoms in the lower alkoxy moiety, di-lower alkylamino-lower alkoxycarbonyl having up to and including 4 carbon atoms in the di-lower alkylamino moiety and also in the lower alkoxy moiety, carbamoyl, N-mono- or N,N-di-lower alkylcarbamoyl in which lower alkyl has up to and including 4 carbon atoms, N—(N',N'-di-lower alkylamino-lower alkyl)-carbamoyl having up to and including 4 carbon atoms in the di-lower alkylamino moiety and also in the lower alkyl moiety, or $R_8$ on the other hand represents 5-tetrazolyl, $R_2$ represents lower alkyl having up to and including 4 carbon atoms, $R_3$ represents hydrogen, $R_9$ represents hydrogen, lower alkyl having an atomic number of from 17 up to and including 53, or trifluoromethyl, $R_7$ represents hydrogen, lower alkyl having up to and including 4 carbon atoms, lower alkoxy having up to and including 4 carbon atoms, or halogen having an atomic number of up to and including 35, and $R_6$ represents hydrogen, lower alkyl having up to and including 4 carbon atoms, halogen having up to and including 4 carbon atoms, halogen having an atomic number of up to and including 35, lower alkoxycarbonyl having up to and including 4 carbon atoms in the lower alkoxy moiety, carboxy, or lower alkanoyl having up to and including 7 carbon atoms, and alk represents lower alkylene having up to and including 4 carbon atoms, and their salts, especially their pharmaceutically acceptable salts.

3. Compounds as claimed in claim 1 of the formula I, in which $R_1$ represents lower alkyl having up to and including 4 carbon atoms, $R_2$ represents straight-chain lower alkyl having from 2 up to and including 4 carbon atoms, $R_3$ represents hydrogen and $R_5$ represents oxaloamino or 5-tetrazolylcarbonylamino, and in which either $R_4$ represents hydrogen and each of $R_6$ and $R_7$, independently of the other, represents hydrogen or lower alkyl having up to and including 4 carbon atoms or $R_4$ represents lower alkyl having up to and including 4 carbon atoms, or trifluoromethyl, $R_6$ represents hydrogen or halogen having an atomic number of up to and including 35 and $R_7$ is hydrogen, or $R_4$, $R_6$ and $R_7$ are identical or different halogen atoms having an atomic number of up to and including 35, and alk represents straight-chain, terminally bonded lower alkylene having from 2 up to and including 4 carbon atoms, and their salts, especially their pharmaceutically acceptable salts with bases.

4. Compounds as claimed in claim 1 of the formula I, in which $R_1$ represents lower alkyl having up to and including 4 carbon atoms, $R_2$ represents straight-chain lower alkyl having up to and including 4 carbon atoms, $R_3$ and $R_7$ represent hydrogen, one of the radicals $R_4$ and $R_6$ represents lower alkyl having up to and including 4 carbon atoms, and the other represents halogen having an atomic number of up to and including 35, $R_5$ represents oxaloamino or 5-tetrazolylcarbonylamino, and alk represents straight-chain, terminally bonded lower alkylene having up to and including 7 carbon atoms, and their salts, especially their pharmaceutically acceptable salts with bases.

5. N-{3-[3-(4-acetyl-3-hydroxy-2-n-propylphenoxy)-propoxy]-6-methylphenyl}-oxamic acid or a salt thereof, N-{3-[3-(4-acetyl-3-hydroxy-2-n-propylphenoxy)-propoxy]-4-bromo-6-methylphenyl}-oxamic acid or a salt thereof, N-{3-[3-(4-acetyl-3-hydroxy-2-n-propylphenoxy)-propoxy]-2,4,6-trichlorophenyl}-oxamic acid or a salt thereof or N-{3-[3-(4-acetyl-3-hydroxy-2-n-propylphenoxy)-propoxy]-phenyl}-tetrazole-5-carboxamide or a salt thereof as claimed in claim 1.

6. N-{3-[3-(4-acetyl-3-hydroxy-2-n-propylphenoxy)-propoxy]-phenyl}-oxamic acid methyl ester, N-{3-[3-(4-acetyl-3-hydroxy-2-n-propylphenoxy)-propoxy]-phenyl}-oxamic acid or a salt thereof, N-{3-[3-(4-acetyl-3-hydroxy-2-n-propylphenoxy)-propoxy]-4-bromo-6-methylphenyl}-oxamic acid methyl ester, N-{3-[3-(4-acetyl-3-hydroxy-2-n-propylphenoxy)-propoxy]-6-methylphenyl}-oxamic acid methyl ester, N-{3-[3-(4-acetyl-3-hydroxy-2-n-propylphenoxy)-propoxy]-2-methylphenyl}-oxamic acid methyl ester, N-{3-[3-(4-acetyl-3-hydroxy-2-n-propylphenoxy)-propoxy]-2-methylphenyl}-oxamic acid or a salt thereof, N-{3-[5-(4-acetyl-3-hydroxy-2-n-propylphenoxy)-pentyloxy]-phenyl}-oxamic acid methyl ester, N-{3-[3-(4-acetyl-3-hydroxy-2-n-propylphenoxy)-propoxy]-4-chloro-6-trifluoromethylphenyl}-oxamic acid methyl ester, N-{3-[3-(4-acetyl-3-hydroxy-2-n-propylphenoxy)-propoxy]-4-chloro-6-trifluoromethylphenyl}-oxamic acid or a salt thereof, N-{3-[3-(4-acetyl-3-hydroxy-2-n-propylphenoxy)-propoxy]-2,4,6-trichlorophenyl}-oxamic acid methyl ester, N-{3-[3-(4-acetyl-3-hydroxy-2-n-propylphenoxy)-propoxy]-4,6-dimethylphenyl}-oxamic acid methyl ester, N-{3-[3-(4-acetyl-3-hydroxy-2-n-propylphenoxy)-propoxy-4,6-dimethylphenyl}-oxamic acid or a salt thereof, N-{4-[3-(4-acetyl-3-hydroxy-2-n-propylphenoxy)-propoxy]-3-methoxyphenyl}-oxamic acid methyl ester, N-{4-[3-(4-acetyl-3-hydroxy-2-n-propylphenoxy)-propoxy]-2-methylphenyl}-oxamic acid methyl ester, N-{4-[3-(4-acetyl-3-hydroxy-2-n-propylphenoxy)-propoxy]-2-methylphenyl}-oxamic acid or a salt thereof, N-{4-[3-(4-acetyl-3-hydroxy-2-n-propylphenoxy)-propoxy]-phenyl}-oxamic acid methyl ester, N-{4-[3-(4-acetyl-3-hydroxy-2-n-propylphenoxy)-propoxy]-phenyl}-oxamic acid or a salt thereof, N-{3-[5-(4-acetyl-3-hydroxy-2-n-propylphenoxy)-pentyloxy]-phenyl}-oxamic acid or a salt thereof, N-{3-[3-(4-acetyl-3-hydroxy-2-n-propylphenoxy)-propoxy]-4-bromo-6-methylphenyl}-tetrazole-5-carboxamide or a salt thereof, N-{3-[5-(4-acetyl-3-hydroxy-2-n-propylphenoxy)-pentyloxy]-phenyl}-oxamic acid or a salt thereof, or N-{3-[3-(4-acetyl-3-hydroxy-2-n-propylphenoxy)-propoxy]-4-carboxyphenyl}-oxamic acid or an acidic or neutral salt thereof as claimed in claim 1.

7. N-{3-[3-(4-acetyl-3-hydroxy-2-n-propylphenoxy)-propoxy]-4-bromo-6-methylphenyl}-oxamic acid ethyl ester as claimed in claim 1.

8. N-{3-[3-(4-acetyl-3-hydroxy-2-n-propylphenoxy)-propoxy]-6-bromo-4-methylphenyl}-1H-tetrazole-5-carboxamide or a salt thereof, N-{3-[3-(4-acetyl-3-hydroxy-2-n-propylphenoxy)-propoxy]-2-cyanophenyl}-oxamic acid or a salt thereof, N-{3-[3-(4-acetyl-3-hydroxy-2-n-propylphenoxy)-propoxyl]-4-chloro-6-methylphenyl}-oxamic acid or a salt thereof, N-{3-[3-(4-acetyl-3-hydroxy-2-n-propylphenoxy)-propoxy]-6-chloro-4-methylphenyl}-tetrazole-5-carboxamide or a salt thereof or N-{3-[3-(4-acetyl-3-hydroxy-2-n-propylphenoxy)-propoxy]-4-chloro-6-methylphenyl}-1H-tetrazole-5-carboxamide or a salt thereof as claimed in claim 1.

9. N-{3-[3-(4-acetyl-3-hydroxy-2-n-propylphenoxy)-propoxy]-2-cyanophenyl}-1H-tetrazole-5-carboxamide or a salt thereof, N-{3-[3-(4-acetyl-3-hydroxy-2-n-propylphenoxy)-propoxy]-6-chloro-4-methylphenyl}-oxamic acid methyl ester, N-{3-[3-(4-acetyl-3-hydroxy-2-n-propylphenoxy)-propoxy]-4-chloro-6-methylphenyl}-oxamic acid methyl ester, N-{3-[3-(4-acetyl-3-hydroxy-2-n-propylphenoxy)-propoxy]-6-bromo-4-methylphenyl}-oxamic acid methyl ester, N-{3-[3-(4-acetyl-3-hydroxy-2-n-propylphenoxy)-propoxy]-6-chloro-4-methylphenyl}-oxamic acid or a salt thereof or N-{3-[3-(4-acetyl-3-hydroxy-2-n-propylphenoxy)-propoxy]-2-cyanophenyl}-oxamic acid ethyl ester as claimed in claim 1.

10. A salt-forming compound as claimed in any one of claims 4 to 6 in the form of its triethanolammonium salt.

11. A salt-forming compound as claimed in any one of claims 1 to 3 and 7 to 9 in the form of its triethanolammonium salt.

12. A salt-forming compound as claimed in claim 4 or N-{3-[3-(4-acetyl-3-hydroxy-2-n-propylphenoxy)-propoxy]-6-methylphenyl}-oxamic acid, N-{3-[3-(4-acetyl-3-hydroxy-2-n-propylphenoxy)-propoxy]-4-bromo-6-methylphenyl}-oxamic acid, N-{3-[5-(4-acetyl-3-hydroxy-2-n-propylphenoxy)-pentyloxy]-phenyl}-oxamic acid or N-{3-[3-(4-acetyl-3-hydroxy-2-n-propylphenoxy)-propoxy]-phenyl}-oxamic acid in the form of its sodium salt.

13. A compound as claimed in any one of claims 1 to 12 for use in a method for the therapeutic treatment of the human or animal body.

14. A compound as claimed in any one of claims 1 to 12 for use as claimed in claim 12 as an anti-allergic, anti-asthmatic, anti-inflammatory and/or antiphlogistic agent.

15. Pharmaceutical preparations containing a compound as claimed in any one of claims 1, 2, 8, 9 and 11 to 14 in addition to customary adjuvants and carriers.

16. Pharmaceutical preparations containing a compound as claimed in claim 3 or 7 in addition to customary adjuvants and carriers.

17. Pharmaceutical preparations containing a compound as claimed in any one of claims 4 to 6 and 10 in addition to customary adjuvants and carriers.

18. Use of a compound as claimed in any one of claims 1 to 12 for the manufacture of anti-allergic and/or anti-inflammatory medicaments.

19. A process for the manufacture of novel 4-acyl-resorcinol ethers of the formula

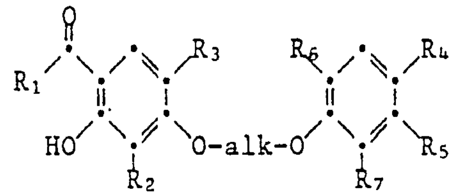

(I)

in which $R_1$ represents lower alkyl, $R_2$ represents lower alkyl, lower alkenyl or lower alkynyl, $R_3$ represents hydrogen, lower alkoxy, trifluoromethyl or halogen, alk represents an alkylene radical having from 2 to 9 chain members, one of the radicals $R_4$, $R_5$ and $R_7$ is a group of the formula —NH—C(=O)—$R_8$, a radical $R_4$ or $R_5$ that is other than a group of the formula —NH—C(=O)—$R_8$ is a radical $R_9$, and a radical $R_7$ that is other than a group of the formula —NH—C(=O)—$R_8$ is a radical $R_{10}$, $R_6$ represents hydrogen, lower alkyl, trifluoromethyl, halogen, optionally esterified or amidated carboxy, cyano or lower alkanoyl, $R_8$ represents optionally esterified or amidated carboxy, or 5-tetrazolyl, $R_9$ represents hydrogen, lower alkyl, lower alkoxy, halogen or trifluoromethyl, and $R_{10}$ represents hydrogen, lower alkyl, lower alkoxy, halogen, trifluoromethyl, cyano or optionally esterified or amidated carboxy, with the proviso that in compounds of the formula I in which $R_4$ represents a group —NH—(C=O)—$R_8$ and $R_8$ represents 5-tetrazolyl, at least one of the radicals $R_3$, $R_6$, $R_9$ and $R_{10}$ is other than hydrogen, and their salts, characterised in that

a) in a compound of the formula

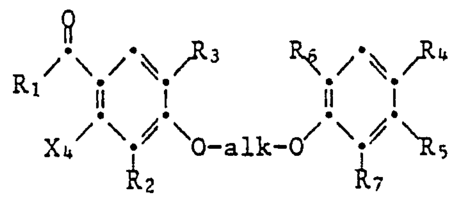

(VI)

in which $X_4$ represents a radical that can be converted into hydroxy, $X_4$ is converted into hydroxy, or

b) there are reacted with one another compounds of the formulae

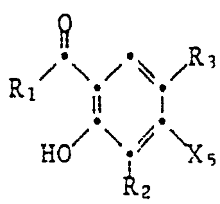

(VII) and

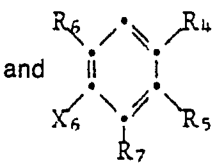

(VIII)

in which one of the radicals $X_5$ and $X_6$ is hydroxy that is optionally in salt form and the other is a radical —O—alkH that is substituted by reactive esterified hydroxy, that is to say an alkoxy radical substituted by reactive esterified hydroxy, or

c) a compound of the formula

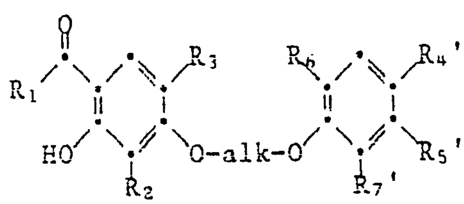

(X)

in which one of the radicals $R'_4$, $R'_5$ and $R'_7$ is an amino group, a radical $R'_4$ or $R'_5$ that is other than an amino group is a radical $R_9$ and a radical $R'_7$ that is other than an amino group is a radical $R_{10}$, or a salt thereof, is reacted with a compound of the formula

$$X_7—R'_8 \quad (XI)$$

in which $R'_8$ represents an optionally esterified or amidated carboxy group or 5-tetrazolyl that is optionally protected in the 1-position, and $X_7$ represents an optionally esterified, amidated or anhydridised carboxy group, or, if $R'_8$ represents 5-tetrazolyl that is protected in the 1-position, $X_7$ represents a carboxy group that is optionally in salt form, and, optionally, the protecting group in the 1-position of a tetrazolyl group $R_8$ is removed, or

d) in a compound of the formula

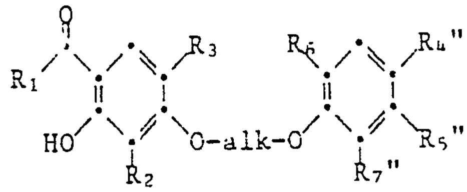

(XII)

in which one of the radicals $R''_4$, $R''_5$ and $R''_7$ is a radical $X_8$, a radical $R''_4$ or $R''_5$ that is other than a radical $X_8$ is a radical $R_9$ and a radical $R''_7$ that is other than a radical $X_8$ is a radical $R_{10}$, and $X_8$ represents a radical that can be converted into the desired group of the formula —NH—C(=O)—$R_8$, $X_8$ is converted into that group,
and, if desired, a compound obtainable according to the process is converted into a different compound of the formula I, an isomeric mixture obtainable according to the process is separated and the desired isomer(s) is(are) isolated, and/or a free compound obtainable according to the process in converted into a salt or a salt obtainable according to the process is converted into the free compound or into a different salt.

**Claims** (for the Contracting State AT)

1. A process for the manufacture of novel 4-acyl-resorcinol ethers of the formula

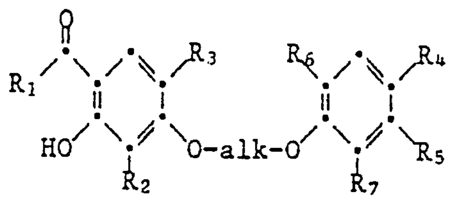

(I)

in which $R_1$ represents lower alkyl, $R_2$ represents lower alkyl, lower alkenyl or lower alkynyl, $R_3$ represents hydrogen, lower alkoxy, trifluoromethyl or halogen, alk represents an alkylene radical having from 2 to 9 chain members, one of the radicals $R_4$, $R_5$ and $R_7$ is a group of the formula —NH—C(=O)—$R_8$, a radical $R_4$ or $R_5$ that is other than a group of the formula —NH—C(=O)—$R_8$ is a radical $R_9$, and a radical $R_7$ that is other than a group of the formula —NH—C(=O)—$R_8$ is a radical $R_{10}$, $R_6$ represents hydrogen, lower alkyl, halogen, trifluoromethyl, optionally esterified or amidated carboxy, cyano or lower alkanoyl, $R_8$ represents optionally esterified or amidated carboxy, or 5-tetrazolyl, $R_9$, represents hydrogen, lower alkyl, lower alkoxy, halogen or trifluoromethyl, and $R_{10}$ represents hydrogen, lower alkyl, lower alkoxy, halogen, trifluoromethyl, cyano or optionally esterified or amidated carboxy and lower organic groups have up to and including 7 carbon atoms, with the proviso that in compounds of the formula I in which $R_4$ represents a group —NH—(C=O)—$R_8$ and $R_8$ represents 5-tetrazolyl, at least one of the radicals $R_3$, $R_6$, $R_9$ and $R_{10}$ is other than hydrogen, and their salts, characterised in that

a) in a compound of the formula

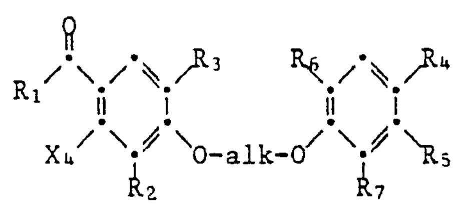

(VI)

in which $X_4$ represents a radical that can be converted into hydroxy, $X_4$ is converted into hydroxy, or

b) there are reacted with one another compounds of the formulae

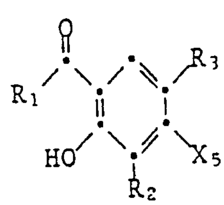

(VII) and

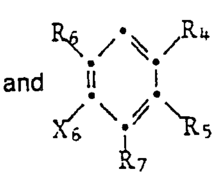

(VIII)

in which one of the radicals $X_5$ and $X_6$ is hydroxy that is optionally in salt form and the other is a radical —O—alkH that is substituted by reactive esterified hydroxy, that is to say an alkoxy radical substituted by reactive esterified hydroxy, or

c) a compound of the formula

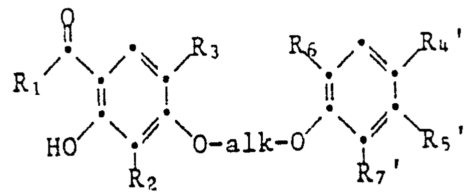

(X)

in which one of the radicals $R'_4$, $R'_5$ and $R'_7$ is an amino group, a radical $R'_4$ or $R'_5$ that is other than an amino group is a radical $R_9$ and a radical $R'_7$ that is other than an amino group is a radical $R_{10}$, or a salt thereof, is reacted with a compound of the formula

$$X_7—R'_8 \qquad (XI)$$

in which $R'_8$ represents an optionally esterified or amidated carboxy group or 5-tetrazolyl that is optionally protected in the 1-position, and $X_7$ represents an optionally esterified, amidated or anhydridised carboxy group, or, if $R'_8$ represents 5-tetrazolyl that is protected in the 1-position, $X_7$ represents a carboxy group that is optionally in salt form, and, optionally, the protecting group in the 1-position of a tetrazolyl group $R_8$ is removed, or

d) in a compound of the formula

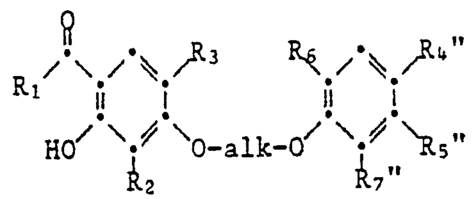

(XII)

in which one of the radicals $R''_4$, $R''_5$ and $R''_7$ is a radical $X_8$, a radical $R''_4$ or $R''_5$ that is other than a radical $X_8$ is a radical $R_9$ and a radical $R''_7$ that is other than a radical $X_8$ is a radical $R_{10}$, and $X_8$ represents a radical that can be converted into the desired group of the formula —NH—C(=O)—$R_8$, $X_8$ is converted into that group,

and, if desired, a compound obtainable according to the process is converted into a different compound of the formula I, an isomeric mixture obtainable according to the process is separated and the desired isomer(s) is(are) isolated, and/or a free compound obtainable according to the process is converted into a salt or a salt obtainable according to the process is converted into the free compound or into a different salt.

2. A process as claimed in claim 1 for the manufacture of compounds as claimed in claim 1 of the formula

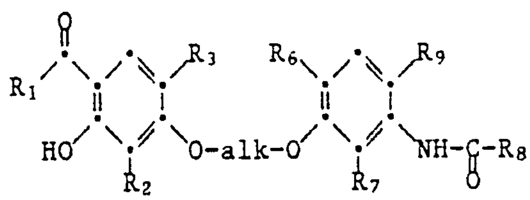

(I')

or

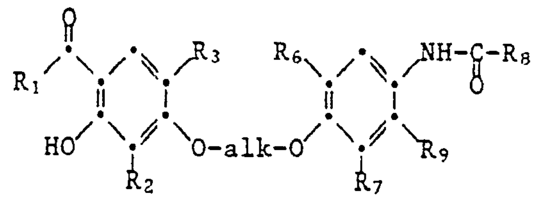

(I")

in which $R_1$ represents lower alkyl having up to and including 4 carbon atoms, such as methyl, $R_8$ on the one hand represents carboxy, lower alkoxycarbonyl having up to and including 4 carbon atoms in the

lower alkoxy moiety, di-lower alkylamino-lower alkoxycarbonyl having up to and including 4 carbon atoms in the di-lower alkylamino moiety and also in the lower alkoxy moiety, carbamoyl, N-mono- or N,N-di-lower alkylcarbamoyl in which lower alkyl has up to and including 4 carbon atoms, N-(N',N'-di-lower alkylamino-lower alkyl)-carbamoyl having up to and including 4 carbon atoms in the di-lower alkylamino moiety and also in the lower alkyl moiety, or $R_8$ on the other hand represents 5-tetrazolyl, $R_2$ represents hydrogen or lower alkyl having up to and including 4 carbon atoms, $R_3$ represents hydrogen, lower alkyl having up to and including 4 carbon atoms, lower alkoxy having up to and including 4 carbon atoms or halogen having an atomic number of from 17 up to and including 53, $R_9$ represents hydrogen, lower alkyl having up to and including 4 carbon atoms, halogen having an atomic number of from 17 up to and including 53, or trifluoromethyl, $R_7$ represents hydrogen, lower alkyl having up to and including 4 carbon atoms, lower alkoxy having up to and including 4 carbon atoms, or halogen having an atomic number of up to and including 35, and $R_6$ represents hydrogen, lower alkyl having up to and including 4 carbon atoms, halogen having an atomic number of up to and including 35, lower alkoxycarbonyl having up to and including 4 carbon atoms in the lower alkoxy moiety, carboxy, or lower alkanoyl having up to and including 7 carbon atoms, and alk represents lower alkylene having up to and including 4 carbon atoms, and their salts, especially their pharmaceutically acceptable salts.

3. A process as claimed in claim 1 for the manufacture of compounds as claimed in claim 1 of the formula I, in which $R_1$ represents lower alkyl having up to and including 4 carbon atoms, $R_2$ represents straight-chain lower alkylene having from 2 up to and including 4 carbon atoms, $R_3$ represents hydrogen and $R_5$ represents oxaloamino or 5-tetrazolylcarbonylamino, and in which either $R_4$ represents hydrogen and each of $R_6$ and $R_7$, independently of the other, represents hydrogen or lower alkyl having up to and including 4 carbon atoms or $R_4$ represents lower alkyl having up to and including 4 carbon atoms, or trifluoromethyl, $R_6$ represents hydrogen or halogen having an atomic number of up to and including 35 and $R_7$ is hydrogen, or $R_4$, $R_6$ and $R_7$ are identical or different halogen atoms having an atomic number of up to and including 35, and alk represents straight-chain, terminally bonded lower alkylene having from 2 up to and including 4 carbon atoms, and their salts, especially their pharmaceutically acceptable salts with bases.

4. A process as claimed in claim 1 for the manufacture of compounds as claimed in claim 1 of the formula I, in which $R_1$ represents lower alkyl having up to and including 4 carbon atoms, $R_2$ represents straight-chain lower alkyl having up to and including 4 carbon atoms, $R_3$ and $R_7$ represent hydrogen, one of the radicals $R_4$ and $R_6$ represents lower alkyl having up to and including 4 carbon atoms, and the other represents halogen having an atomic number of up to and including 35, $R_5$ represents oxaloamino or 5-tetrazolylcarbonylamino, and alk represents straight-chain, terminally bonded lower alkylene having up to and including 7 carbon atoms, and their salts, especially their pharmaceutically acceptable salts, with bases.

5. A process as claimed in claim 1 for the manufacture of N-{3-[3-(4-acetyl-3-hydroxy-2-n-propylphenoxy)-propoxy]-6-methylphenyl}-oxamic acid or a salt thereof, N-{3-[3-(4-acetyl-3-hydroxy-2-n-propylphenoxy)-propoxy]-4-bromo-6-methylphenyl}-oxamic acid or a salt thereof, N-{3-[3-(4-acetyl-3-hydroxy-2-n-propylphenoxy)-propoxy]-2-cyanophenyl}-1H-tetrazole-5-carboxamide or a salt thereof, N-{3-[3-(4-acetyl-3-hydroxy-2-n-propylphenoxy)-propoxy]-6-bromo-4-methylphenyl}-1H-tetrazole-5-carboxamide or a salt thereof, N-{3-[3-(4-acetyl-3-hydroxy-2-n-propylphenoxy)-propoxy]-2-cyanophenyl}-oxamic acid or a salt thereof, N-{3-[3-(4-acetyl-3-hydroxy-2-n-propylphenoxy)-propoxy]-2,4,6-trichlorophenyl}-oxamic acid or a salt thereof, N-{3-[3-(4-acetyl-3-hydroxy-2-n-propylphenoxy)-propoxy]-phenyl}-tetrazole-5-carboxamide or a salt thereof, N-{3-[3-(4-acetyl-3-hydroxy-2-n-propylphenoxy)-propoxy]-6-chloro-4-methylphenyl}-oxamic acid or a salt thereof, N-{3-[3-(4-acetyl-3-hydroxy-2-n-propylphenoxy)-propoxy]-6-chloro-4-methylphenyl}-1H-tetrazole-5-carboxamide or a salt thereof or N-{3-[3-(4-acetyl-3-hydroxy-2-n-propylphenoxy)-propoxy]-4-chloro-6-methylphenyl}-1H-tetrazole-5-carboxamide or a salt thereof.

6. A process as claimed in claim 1 for the manufacture of N-{3-[5-(4-acetyl-3-hydroxy-2-n-propylphenoxy)-pentyloxy]-phenyl}-oxamic acid or a salt thereof, N-{3-[3-(4-acetyl-3-hydroxy-2-n-propylphenoxy)-propoxy]-phenyl}-oxamic acid methyl ester, N-{3-[3-(4-acetyl-3-hydroxy-2-n-propylphenoxy)-propoxy]-phenyl}-oxamic acid or a salt thereof, N-{3-[3-(4-acetyl-3-hydroxy-2-n-propylphenoxy)-propoxy]-4-bromo-6-methylphenyl}-oxamic acid methyl ester, N-{3-[3-(4-acetyl-3-hydroxy-2-n-propylphenoxy)-propoxy]-6-methylphenyl}-oxamic acid methyl ester, N-{3-[3-(4-acetyl-3-hydroxy-2-n-propylphenoxy)-propoxy]-2-methylphenyl}-oxamic acid methyl ester, N-{3-[3-(4-acetyl-3-hydroxy-2-n-propylphenoxy)-propoxy]-2-methylphenyl}-oxamic acid or a salt thereof, N-{3-[5-(4-acetyl-3-hydroxy-2-n-propylphenoxy)-pentyloxy]-phenyl}-oxamic acid methyl ester, N-{3-[3-(4-acetyl-3-hydroxy-2-n-propylphenoxy)-propoxy]-4-chloro-6-trifluoromethylphenyl}-oxamic acid methyl ester, N-{3-[3-(4-acetyl-3-hydroxy-2-n-propylphenoxy)-propoxy]-4-chloro-6-trifluoromethylphenyl}-oxamic acid or a salt thereof, N-{3-[3-(4-acetyl-3-hydroxy-2-n-propylphenoxy)-propoxy]-2,4,6-trichlorophenyl}-oxamic acid methyl ester, N-{3-[3-(4-acetyl-3-hydroxy-2-n-propylphenoxy)-propoxy]-4,6-dimethylphenyl}-oxamic acid methyl ester, N-{3-[3-(4-acetyl-3-hydroxy-2-n-propylphenoxy)-propoxy]-4,6-dimethylphenyl}-oxamic acid or a salt thereof, N-{4-[3-(4-acetyl-3-hydroxy-2-n-propylphenoxy)-propoxy]-3-methoxyphenyl}-oxamic acid methyl ester, N-{4-[3-(4-acetyl-3-hydroxy-2-n-propylphenoxy)-propoxy]-3-methoxyphenyl}-oxamic acid or a salt thereof, N-{4-[3-(4-acetyl-3-hydroxy-2-n-propylphenoxy)-propoxy]-2-methylphenyl}-oxamic acid methyl ester, N-{4-[3-(4-acetyl-3-hydroxy-2-n-propylphenoxy)-propoxy]-2-methylphenyl}-oxamic acid or a salt thereof, N-{2-[3-(4-acetyl-3-hydroxy-2-n-propylphenoxy)-propoxy]-phenyl}-oxamic acid methyl ester, N-{2-[3-(4-acetyl-3-hydroxy-2-n-propylphenoxy)-propoxy]-phenyl}-oxamic acid or a salt thereof, N-{4-[3-(4-acetyl-3-hydroxy-2-n-propylphenoxy)-propoxy]-phenyl}-oxamic acid methyl ester, N-{4-[3-(4-acetyl-3-hydroxy-2-n-propylphenoxy)-propoxy]-phenyl}-oxamic acid or a salt thereof, N-{3-[3-(4-acetyl-3-hydroxy-2-n-propylphenoxy)-propoxy]-4-bromo-6-methylphenyl}-tetrazole-5-carboxamide or a salt thereof, N-{3-[3-(4-acetyl-3-hydroxy-2-n-propylphenoxy)-propoxy]-4-carboxyphenyl}-oxamic acid or an acidic or a neutral salt thereof, N-{3-[3-(4-acetyl-3-hydroxy-2-n-propylphenoxy)-propoxy]-4-methoxycarbonylphenyl}-oxamic acid or a salt thereof, N-{3-[3-(4-acetyl-3-hydroxy-2-n-propylphenoxy)-propoxy]-4-bromo-6-methylphenyl}-oxamic acid ethyl ester, N-{3-[3-(4-acetyl-3-hydroxy-2-n-propylphenoxy)-propoxy]-6-chloro-4-methylphenyl}-oxamic acid methyl ester, N-{3-[3-(4-acetyl-3-hydroxy-2-n-propylphenoxy)-propoxyl]-4-chloro-6-methylphenyl}-oxamic acid methyl ester, N-{3-[3-(4-acetyl-3-hydroxy-2-n-propylphenoxy)-propoxy]-6-bromo-4-methylphenyl}-oxamic acid methyl ester, N-{3-[3-(4-acetyl-3-hydroxy-2-n-propylphenoxy)-propoxy]-4-chloro-6-methylphenyl}-oxamic acid or a salt thereof, or N-{3-[3-(4-acetyl-3-hydroxy-2-n-propylphenoxy)-propoxy]-2-cyanophenyl}-oxamic acid ethyl ester.

7. A process for the manufacture of a salt-forming compound as claimed in any one of claims 1 to 6 in the form of its triethanolammonium salt.

8. A process for the manufacture of a compound as claimed in any one of claims 1 to 4, or of N-{3-[3-(4-acetyl-3-hydroxy-2-n-propylphenoxy)-propoxy]-6-methylphenyl}-oxamic acid, N-{3-[3-(4-acetyl-3-hydroxy-2-n-propylphenoxy)-propoxy]-4-bromo-6-methylphenyl}-oxamic acid or N-{3-[5-(4-acetyl-3-hydroxy-2-n-propylphenoxy)-pentyloxy]-phenyl}-oxamic acid in the form of its sodium salt.

9. A process for the manufacture of pharmaceutical preparations, characterised in that a compound obtainable in accordance with any one of claims 1 to 8 is mixed with customary pharmaceutical adjuvants and carriers.

10. A process as claimed in claim 9 for the manufacture of an anti-allergic, anti-asthmatic, anti-inflammatory and/or antiphlogistic agent.

**Revendications** (pour les Etats contractants : BE, CH, DE, FR, GB, IT, LI, LU, NL, SE)

1. Nouveaux éthers de 4-acylrésorcinols de formule :

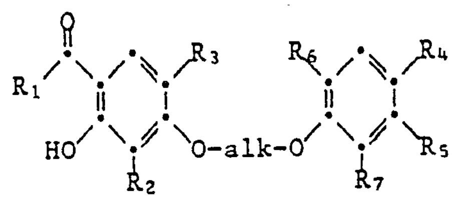

(I)

dans laquelle $R_1$ représente un groupe alkyle inférieur, $R_2$ représente un groupe alkyle inférieur, alcényle inférieur ou alcynyle inférieur, $R_3$ représente l'hydrogène, un groupe alcoxy inférieur, trifluorométhyle ou un halogène, alk représente un groupe alkylène contenant 2 à 9 chaînons, l'un des symboles $R_4$, $R_5$ et $R_7$ représente un groupe de formule —NH—C(=O)$R_8$, l'un des symboles $R_4$ et $R_5$, autre que le précédent, représente un substituant $R_9$, et un symbole $R_7$, autre que les précédents, représente un substituant $R_{10}$, $R_6$ représente l'hydrogène, un groupe alkyle inférieur, un halogène, un groupe trifluorométhyle, un groupe carboxy éventuellement estérifié ou amidé, un groupe cyano ou alcanoyle inférieur, $R_8$ représente un groupe carboxy éventuellement estérifié ou amidé ou un groupe 5-tétrazolyle, $R_9$ représente l'hydrogène, un groupe alkyle inférieur, alcoxy inférieur, un halogène ou un groupe trifluorométhyle et $R_{10}$ représente l'hydrogène, un groupe alkyle inférieur, alcoxy inférieur, un halogène, un groupe trifluorométhyle, cyano ou carboxy éventuellement estérifié ou amidé, l'expression « inférieur » s'appliquant à des groupes organiques qui contiennent jusqu'à 7 atomes de carbone inclus, étant spécifié que, dans les composés de formule I dans lesquels $R_4$ représente un groupe —NH—(C=O)—$R_8$ et $R_8$ représente le groupe 5-tétrazolyle, au moins un des symboles $R_3$, $R_6$, $R_9$ et $R_{10}$ a une signification autre que l'hydrogène, et leurs sels.

2. Composés selon la revendication 1, de formules respectives :

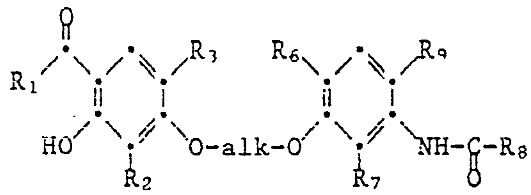

(I')

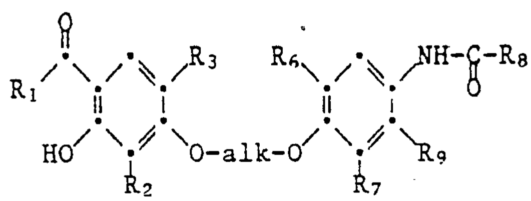

(I")

dans lesquelles $R_1$ représente un groupe alkyle inférieur contenant jusqu'à 4 atomes de carbone inclus, $R_8$ représente d'une part un groupe carboxy (alcoxy inférieur)-carbonyle contenant jusqu'à 4 atomes de carbone inclus dans la partie alcoxy inférieur, di-(alkyle inférieur)-amino-(alcoxy inférieur)-carbonyle contenant jusqu'à 4 atomes de carbone inclus dans chacune des parties di-(alkyle inférieur)-amino et alcoxy inférieur, un groupe carbamyle, N-mono- ou N,N,di-(alkyle inférieur)-carbamyle dans lesquels les groupes alkyle inférieurs contiennent jusqu'à 4 atomes de carbone inclus, N-(N',N'-di-(alkyle inférieur)-amino-(alkyle inférieur))-carbamyle contenant jusqu'à 4 atomes de carbone inclus dans les parties di-(alkyle inférieur)-amino et alcoxy inférieur, ou d'autre part le groupe 5-tétrazolyle, $R_2$ représente un groupe alkyle inférieur contenant jusqu'à 4 atomes de carbone inclus, $R_3$ représente l'hydrogène $R_9$ représente l'hydrogène, un groupe alkyle inférieur contenant jusqu'à 4 atomes de carbone inclus, un halogène de numéro atomique allant de 17 jusqu'à 53 inclus ou un groupe trifluorométhyle, $R_7$ représente l'hydrogène, un groupe alkyle inférieur contenant jusqu'à 4 atomes de carbone inclus, alcoxy inférieur contenant jusqu'à 4 atomes de carbone inclus ou un halogène de numéro atomique allant jusqu'à 35 inclus et $R_6$ représente l'hydrogène, un groupe alkyle inférieur contenant jusqu'à 4 atomes de carbone inclus, un halogène de numéro atomique allant jusqu'à 35 inclus, un groupe (alcoxy inférieur)-carbonyle contenant jusqu'à 4 atomes de carbone inclus dans la partie alcoxy inférieur, un groupe carboxy ou alcanoyle inférieur contenant jusqu'à 7 atomes de carbone inclus et alk représente un groupe alkylène inférieur contenant jusqu'à 4 atomes de carbone inclus, et leurs sels, en particulier leurs sels acceptables pour l'usage pharmaceutique.

3. Composés selon la revendication 1, de formule I dans laquelle $R_1$ représente un groupe alkyle inférieur contenant jusqu'à 4 atomes de carbone inclus, $R_2$ représente un groupe alkyle inférieur à chaîne droite contenant de 2 à 4 atomes de carbone inclus, $R_3$ représente l'hydrogène et $R_5$ un groupe oxaloamino ou 5-tétrazolylcarbonylamino, et ou bien $R_4$ représente l'hydrogène et $R_6$ et $R_7$, indépendamment l'un de l'autre, l'hydrogène ou un groupe alkyle inférieur contenant jusqu'à 4 atomes de carbone inclus, ou bien $R_4$ représente un groupe alkyle inférieur contenant jusqu'à 4 atomes de carbone inclus ou un groupe trifluorométhyle, $R_6$ représente l'hydrogène ou un halogène de numéro atomique allant jusqu'à 35 inclus et $R_7$ représente l'hydrogène, ou bien $R_4$, $R_6$ et $R_7$ représentent des atomes d'halogènes identiques ou différents de numéro atomique allant jusqu'à 35 inclus, et alk représente un groupe alkylène inférieur à chaîne droite en position terminale contenant 2 à 4 atomes de carbone inclus, et leurs sels, en particulier leurs sels de bases acceptables pour l'usage pharmaceutique.

4. Composés selon la revendication 1, de formule I dans laquelle $R_1$ représente un groupe alkyle inférieur contenant jusqu'à 4 atomes de carbone inclus, $R_2$ représente un groupe alkyle inférieur à chaîne droite contenant jusqu'à 4 atomes de carbone inclus, $R_3$ et $R_7$ représentent l'hydrogène, l'un des symboles $R_4$ et $R_6$ représente un groupe alkyle inférieur contenant jusqu'à 4 atomes de carbone inclus et l'autre un halogène de numéro atomique allant jusqu'à 35 inclus, $R_5$ représente un groupe oxaloamino ou 5-tétrazolylcarbonylamino et alk représente un groupe alkylène inférieur à chaîne droite en position terminale contenant jusqu'à 7 atomes de carbone inclus, et leurs sels, en particulier leurs sels de bases acceptables pour l'usage pharmaceutique.

5. L'acide N-{3-[3-(4-acétyl-3-hydroxy-2-n-propylphénoxy)-propyloxy]-6-méthylphényl}-oxamique ou l'un de ses sels, l'acide N-{3-[4-acétyl-3-hydroxy-2-n-propylphénoxy)-propyloxy]-4-bromo-6-méthylphényl}-oxamique ou l'un de ses sels, l'acide N-{3-[3-(4-acétyl-3-hydroxy-2-n-propylphénoxy)-propyloxy]-2,4,6-trichlorophényl}-oxamique ou l'un de ses sels ou le N-{3-[4-acétyl-3-hydroxy-2-n-propylphénoxy)-propyloxy]-phényl}-tétrazole-5-carboxamide ou l'un des sels selon la revendication 1.

6. Le N-{3-[3-(4-acétyl-3-hydroxy-2-n-propylphénoxy)-propyloxy]-phényl}-oxamate de méthyle, l'acide N-{3-[3-(4-acétyl-3-hydroxy-2-n-propylphénoxy)-propyloxy]-phényl}-oxamique ou l'un de ses sels, le N-{3-[3-(4-acétyl-3-hydroxy-2-n-propyl-phénoxy)-propyloxy]-4-bromo-6-méthylphényl}-oxamate de méthyle, le N-{3-[3-(4-acétyl-3-hydroxy-2-n-propylphénoxy)-propyloxy]-6-méthylphényl}-oxamate de méthyle, le N-{3-[3-(4-acétyl-3-hydroxy-2-n-propylphénoxy)-propyloxy]-2-méthylphényl}-oxamate de méthyle, l'acide N-{3-[3-(4-acétyl-3-hydroxy-2-n-propylphénoxy)-propyloxy]-2-méthylphényl}-oxamique ou l'un de ses sels, le N-{3-[5-(4-acétyl-3-hydroxy-2-n-propylphénoxy)-pentyloxy]-phényl}-oxamate de méthyle le N-{3-[3-(4-acétyl-3-hydroxy-2-n-propylphénoxy)-propyloxy]-4-chloro-6-trifluorométhylphényl}-oxamate de méthyle, l'acide N-{3-[3-(4-acétyl-3-hydroxy-2-n-propylphénoxy)-propyloxy]-4-chloro-6-trifluorométhylphényl}-oxamique ou l'un de ses sels, le N-{3-[3-(4-acétyl-3-hydroxy-2-n-propylphénoxy)-propyloxy]-2,4,6-trifluorophényl}-oxamate de méthyle, le N-{3-[3-(4-acétyl-3-hydroxy-2-n-propylphénoxy)-propyloxy]-4,6-diméthylphényl}-oxamate de méthyle, l'acide N-{3-[3-(4-acétyl-3-hydroxy-2-n-propylphénoxy)-propyloxy]-4,6-diméthylphényl}-oxamique ou l'un de ses sels, le N-{4-[3-(4-acétyl-3-hydroxy-2-n-propylphénoxy)-propyloxy]-3-méthoxyphényl}-oxamate de méthyle, le N-{4-[3-(4-acétyl-3-hydroxy-2-n-propylphénoxy)-propyloxy]-2-méthylphényl}-oxamate de méthyle, l'acide N-{4-[3-(4-acétyl-3-hydroxy-2-n-propylphénoxy)-propyloxy]-2-méthylphényl}-oxamique ou l'un de ses sels, le N-{4-[3-(4-acétyl—3-hydroxy-2-n-propylphénoxy)-propyloxy]-phényl}-oxamate de méthyle, l'acide N-{4-[3-(4-acétyl-3-hydroxy-2-n-propylphénoxy)-propyloxy]-phényl}-oxamique ou l'un de ses sels, l'acide N-{3-[5-(4-acétyl-3-hydroxy-2-n-propylphénoxy)-pentyloxy]-phényl}-oxamique ou l'un de ses sels, le N-{3-[3-(4-acétyl-3-hydroxy-2-n-propylphénoxy)-propyloxy]-4-bromo-6-méthylphényl}-tétrazole-5-carboxamide ou l'un de ses sels, l'acide N-{3-[5-(4-acétyl-3-hydroxy-2-n-propylphénoxy)-pentyloxy]-phényl}-oxamique ou l'un de ses sels, ou bien l'acide N-{3-[3-(4-acétyl-3-hydroxy-2-n-propylphénoxy)-propyloxy]-4-carboxyphényl}-oxamique ou un sel acide ou neutre de celui-ci selon la revendication 1.

7. Le N-{3-[3-(4-acétyl-3-hydroxy-2-n-propylphénoxy)-propyloxy]-4-bromo-6-méthylphényl}-oxamate d'éthyle selon la revendication 1.

8. Le N-{3-[3-(4)acétyl-3-hydroxy-2-n-propylphénoxy)-propyloxy]-6-bromo-4-méthylphényl}-1H-tétrazole-5-carboxamide ou l'un de ses sels, l'acide N-{3-[3-(4-acétyl-3-hydroxy-2-n-propylphénoxy)-propyloxy]-2-cyanophényl}-oxamique ou l'un de ses sels, l'acide N-{3-[3-(4-acétyl-3-hydroxy-2-n-propylphénoxy)-propyloxy]-4-chloro-6-méthylphényl}-oxamique ou l'un de ses sels, le N-{3-[3-(4-acétyl-3-hydroxy-2-n-propylphénoxy)-propyloxy]-6-chloro-4-méthylphényl}-tétrazole-5-carboxamide ou l'un de ses sels ou bien le N-{3-[3-(4-acétyl-3-hydroxy-2-n-propylphénoxy)-propyloxy]-4-chloro-6-méthylphényl}-1H-tétrazole-5-carboxamide ou l'un de ses sels selon la revendication 1.

9. Le N-{3-[3-(4-acétyl-3-hydroxy-2-n-propylphénoxy)-propyloxy]-2-cyanophényl}-1H-tétrazole-5-carboxamide ou l'un de ses sels, le N-{3-[3-(4-acétyl-3-hydroxy-2-n-propylphénoxy)-propyloxy]-6-chloro-4-méthylphényl}-oxamate de méthyle, le N-{3-[3-(4-acétyl-3-hydroxy-2-n-propylphénoxy)-propyloxy]-4-chloro-6-méthylphényl}-oxamate de méthyle, le N-{3-[3-(4-acétyl-3-hydroxy-2-n-propylphénoxy)-propyloxy]-6-bromo-4-méthylphényl}-oxamate de méthyle, l'acide N-{3-[3-(4-acétyl-3-hydroxy-2-n-propylphénoxy)-propyloxy]-6-chloro-4-méthylphényl}-oxamique ou l'un de ses sels ou le N-{3-[3-(4-acétyl-3-hydroxy-2-n-propylphénoxy)-propyloxy]-2-cyanophényl}-oxamate d'éthyle selon la revendication 1.

10. Un composé salifiable selon l'une des revendications 4 à 6, à l'état de sel de triéthylammonium.

11. Un composé salifiable selon l'une des revendications 1 à 3 et 7 à 9, à l'état de sel de triéthanolammonium.

12. Un composé salifiable selon la revendication 4 ou l'acide N-{3-[3-(4-acétyl-3-hydroxy-2-n-propylphénoxy)-propyloxy]-6-méthylphényl}-oxamique, l'acide N-{3-[3-(4-acétyl-3-hydroxy-2-n-propylphénoxy)-propyloxy]-4-bromo-6-méthylphényl}-oxamique, l'acide N-{3-[5-(4-acétyl-3-hydroxy-2-n-propylphénoxy)-pentyloxy]-phényl}-oxamique ou l'acide N-{3-[3-(4-acétyl-3-hydroxy-2-n-propylphénoxy)-propyloxy]-phényl}-oxamique à l'état de sel de sodium.

13. Un composé selon l'une des revendications 1 à 12, pour l'utilisation dans un procédé pour le traitement thérapeutique de l'organisme humain ou animal.

14. Un composé selon l'une des revendications 1 à 12, pour l'utilisation selon la revendication 12 en tant qu'anti-allergique, anti-asthmatique, anti-inflammatoire et/ou antiphlogistique.

15. Compositions pharmaceutiques contenant un composé selon l'une des revendications 1, 2, 8, 9 et 11 à 14, avec des produits auxiliaires et des véhicules usuels.

16. Compositions pharmaceutiques contenant un composé selon l'une des revendications 3 à 7, avec des produits auxiliaires et véhicules usuels.

17. Compositions pharmaceutiques contenant un composé selon l'une des revendications 4 à 6 et 10, avec des produits auxiliaires et véhicules usuels.

18. Utilisation d'un composé selon l'une des revendications 1 à 12, pour la préparation de médicaments anti-allergiques et/ou anti-inflammatoires.

19. Procédé de préparation de nouveaux éthers de 4-acylrésorcinols de formule :

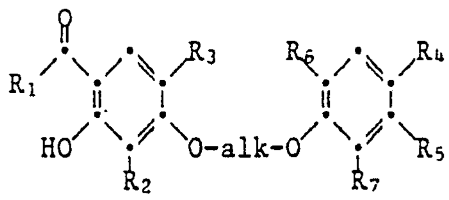

(I)

dans laquelle $R_1$ représente un groupe alkyle inférieur, $R_2$ représente un groupe alkyle inférieur, alcényle inférieur ou alcynyle inférieur, $R_3$ représente l'hydrogène, un groupe alcoxy inférieur, trifluorométhyle ou un halogène, alk représente un groupe alkylène contenant 2 à 9 chaînons, l'un des symboles $R_4$, $R_5$ et $R_7$ représente un groupe de formule —NH—C(=O)—$R_8$, l'un des groupes $R_4$ et $R_5$, différent du précédent, représente un substituant $R_9$, et un symbole $R_7$, différent des précédents, représente un substituant $R_{10}$, $R_6$ représente l'hydrogène, un groupe alkyle inférieur, trifluorométhyle, un halogène, un groupe carboxy éventuellement estérifié ou amidé, un groupe cyano ou alcanoyle inférieur, $R_8$ représente un groupe carboxy éventuellement estérifié ou amidé ou un groupe 5-tétrazolyle, $R_9$ représente l'hydrogène, un groupe alkyle inférieur, alcoxy inférieur, un halogène ou un groupe trifluorométhyle, et $R_{10}$ représente l'hydrogène, un groupe alkyle inférieur, alcoxy inférieur, un halogène, un groupe trifluorométhyle, cyano

ou carboxy éventuellement estérifié ou amidé, étant spécifié que dans les composés de formule I dans laquelle $R_4$ représente un groupe —NH—(C=O)—$R_8$ et $R_8$ un groupe 5-tétrazolyle, l'un au moins des symboles $R_3$, $R_6$, $R_9$ et $R_{10}$ a une signification autre que l'hydrogène, et de leurs sels, caractérisé en ce que :

(a) dans un composé de formule :

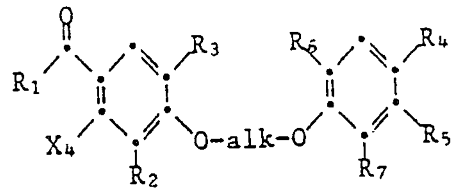

(VI)

dans laquelle $X_4$ représente un groupe convertible en groupe hydroxy, on convertit $X_4$ en un groupe hydroxy, ou bien

(b) on fait réagir entre eux les composés de formules :

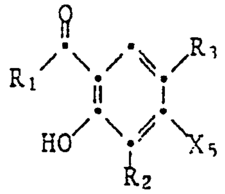

(VII) et

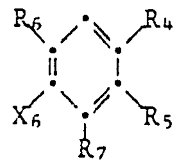

(VIII)

dans lesquelles l'un des symboles $X_5$ et $X_6$ représente un groupe hydroxy éventuellement à l'état de sel et l'autre un groupe —O-alkH substitué par un groupe hydroxy estérifié réactif, c'est-à-dire un groupe alcoxy substitué par un groupe hydroxy estérifié réactif, ou bien

(c) on fait réagir un composé de formule

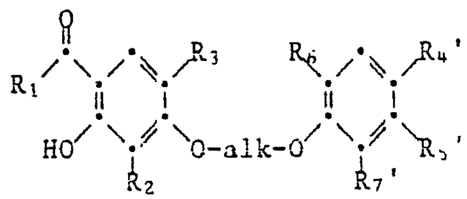

(X)

dans laquelle l'un des symboles $R'_4$, $R'_5$ et $R'_7$ représente le groupe amino, l'un des symboles $R'_4$ et $R'_5$, différent du précédent, représente un substituant $R_9$, et un symbole $R'_7$, différent des précédents, représente un substituant $R_{10}$, ou un sel d'un tel composé, avec un composé de formule

$$X_7—R'_8 \qquad (XI)$$

dans laquelle $R'_8$ représente un groupe carboxy éventuellement estérifié ou amidé ou un groupe 5-tétrazolyle éventuellement protégé en position 1 et $X_7$ représente un groupe carboxy éventuellement estérifié, amidé, anhydrisé ou bien, lorsque $R'_8$ représente un groupe 5-tétrazolyle protégé en position 1, à l'état de sel, et le cas échéant, on élimine le groupe protecteur en position 1 d'un groupe tétrazolyle $R_8$, ou bien

(d) dans un composé de formule

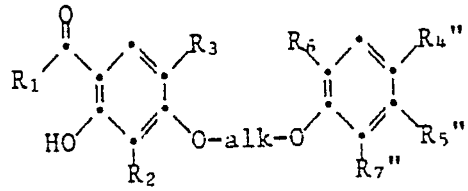

(XII)

dans laquelle l'un des symboles $R''_4$, $R''_5$ et $R''_7$ représente un groupe $X_8$, l'un des symboles $R''_4$ et $R''_5$, différent du précédent, représente un substituant $R_9$, et un symbole $R''_7$, différent des précédents, représente un substituant $R_{10}$ et $X_8$ représente un groupe convertible en le groupe recherché de formule —NH—C(=O)—$R_8$, on convertit $X_8$ en ce groupe et si on le désire, dans tous les cas ci-dessus, on convertit un composé obtenu en un autre composé de formule I, on sépare un mélange d'isomères

obtenu et on isole le ou les isomères recherchés et/ou on convertit un composé libre obtenu en un sel ou un sel obtenu en le composé libre ou en un autre sel.

**Revendications** (pour l'Etat contractant AT)

1. Procédé de préparation de nouveaux éthers de 4-acylrésorcinols de formule :

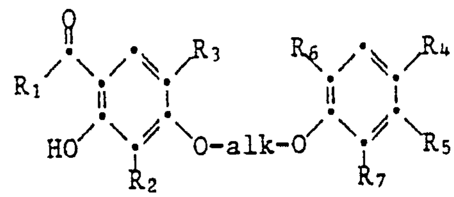

(I)

dans laquelle $R_1$ représente un groupe alkyle inférieur, $R_2$ représente un groupe alkyle inférieur, alcényle inférieur ou alcynyle inférieur, $R_3$ représente l'hydrogène, un groupe alcoxy inférieur, trifluorométhyle ou un halogène, alk représente un groupe alkylène contenant 2 à 9 chaînons, l'un des symboles $R_4$, $R_5$ et $R_7$ représente un groupe de formule —NH—C(=O)—$R_8$, l'un des symboles $R_4$ et $R_5$, différent du précédent, représente un substituant $R_9$, et un symbole $R_7$, différent des précédents, représente un substituant $R_{10}$, $R_6$ représente l'hydrogène, un groupe alkyle inférieur, un halogène, un groupe trifluorométhyle, carboxy éventuellement estérifié ou amidé, cyano ou alcanoyle inférieur, $R_8$ représente un groupe carboxy éventuellement estérifié ou amidé ou un groupe 5-tétrazolyle, $R_9$ représente l'hydrogène, un groupe alkyle inférieur, alcoxy inférieur, un halogène ou un groupe trifluorométhyle, et $R_{10}$ représente l'hydrogène, un groupe alkyle inférieur, alcoxy inférieur, un halogène, un groupe trifluorométhyle, cyano ou carboxy éventuellement estérifié ou amidé, l'expression « inférieur » s'appliquant à des groupes organiques qui contiennent jusqu'à 7 atomes de carbone inclus, étant spécifié que dans les composés de formule I dans lesquels $R_4$ représente un groupe —NH—(C=O)—$R_8$ et $R_8$ représente un groupe 5-tétrazolyle, au moins un des symboles $R_3$, $R_6$, $R_9$ et $R_{10}$ a une signification autre que l'hydrogène, et de leurs sels, caractérisé en ce que :

(a) dans un composé de formule :

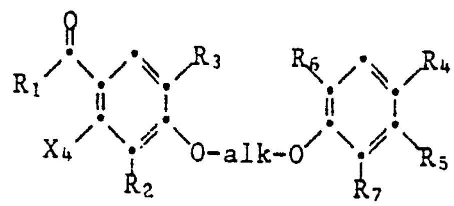

(VI)

dans laquelle $X_4$ représente un groupe convertible en groupe hydroxy, on convertit $X_4$ en un groupe hydroxy, ou bien

(b) on fait réagir entre eux les composés de formules :

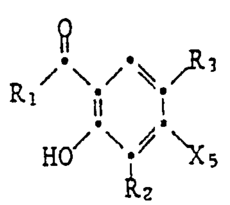

(VII) et

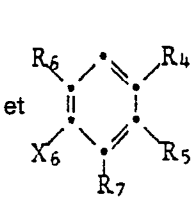

(VIII)

dans lesquelles l'un des symboles $X_5$ et $X_6$ représente un groupe hydroxy éventuellement à l'état de sel et l'autre un groupe —O-alkH substitué par un groupe hydroxy estérifié réactif, c'est-à-dire un groupe alcoxy substitué par un groupe hydroxy estérifié réactif, ou bien

(c) on fait réagir un composé de formule

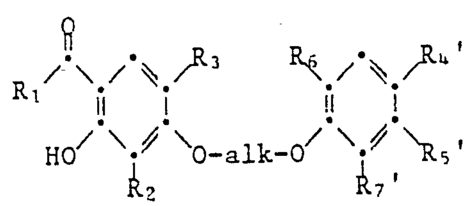

(X)

dans laquelle l'un des symboles $R'_4$, $R'_5$ et $R'_7$ représente le groupe amino, l'un des symboles $R'_4$ et $R'_5$, différent du précédent, représente un substituant $R_9$, et un symbole $R'_7$, différent des précédents, représente un substituant $R_{10}$, ou un sel d'un tel composé, avec un composé de formule

$$X_7—R'_8 \qquad (XI)$$

dans laquelle $R'_8$ représente un groupe carboxy éventuellement estérifié ou amidé ou un groupe 5-tétrazolyle éventuellement protégé en position 1 et $X_7$ représente un groupe carboxy éventuellement estérifié, amidé, anhydrisé ou bien, lorsque $R'_8$ représente un groupe 5-tétrazolyle protégé en position 1, à l'état de sel, et le cas échéant, on élimine le groupe protecteur en position 1 d'un groupe tétrazolyle $R_8$, ou bien

(d) dans un composé de formule

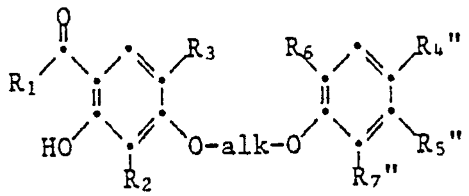

(XII)

dans laquelle l'un des symboles $R''_4$, $R''_5$ et $R''_7$ représente un groupe $X_8$, l'un des symboles $R''_4$ et $R''_5$, différent du précédent, représente un substituant $R_9$, et un symbole $R''_7$, différent des précédents, représente un substituant $R_{10}$ et $X_8$ représente un groupe convertible en le groupe recherché de formule —NH—C(=O)—$R_8$, on convertit $X_8$ en ce groupe et si on le désire, dans tous les cas ci-dessus, on convertit un composé obtenu en un autre composé de formule I, on sépare un mélange d'isomères obtenu et on isole le ou les isomères recherches et/ou on convertit un composé libre obtenu en un sel ou un sel obtenu en le composé libre ou en un autre sel.

2. Procédé selon la revendication 1, pour préparer des composés selon la revendication 1, de formules respectives :

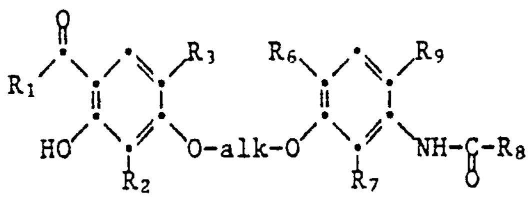

(I'),

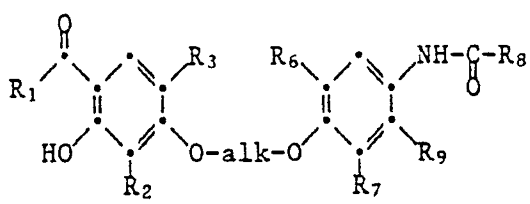

(I"),

dans lesquelles $R_1$ représente un groupe alkyle inférieur contenant jusqu'à 4 atomes de carbone inclus, $R_8$ représente d'une part un groupe carboxy, (alcoxy inférieur)-carbonyle contenant jusqu'à 4 atomes de carbone inclus dans la partie alcoxy inférieur, di-(alkyle inférieur)-amino-(alcoxy inférieur)-carbonyle contenant jusqu'à 4 atomes de carbone inclus dans chacune des parties di-(alkyle inférieur)-amino et alcoxy inférieur, un groupe carbamyle, N-mono- ou N,N-di-(alkyle inférieur)-carbamyle dans lesquels les groupes alkyle inférieurs contiennent jusqu'à 4 atomes de carbone inclus, N-(N',N'-di(alkyle inférieur)-amino-(alkyle inférieur))-carbamyle contenant jusqu'à 4 atomes de carbone inclus dans les parties di-(alkyle inférieur)-amino et alcoxy inférieur, ou d'autre part le groupe 5-tétrazolyle, $R_2$ représente un groupe alkyle inférieur contenant jusqu'à 4 atomes de carbone inclus, $R_3$ représente l'hydrogène $R_9$ représente l'hydrogène, un groupe alkyle inférieur contenant jusqu'à 4 atomes de carbone inclus, un halogène de numéro atomique allant de 17 jusqu'à 53 inclus ou un groupe trifluorométhyle, $R_7$ représente l'hydrogène, un groupe alkyle inférieur contenant jusqu'à 4 atomes de carbone inclus, alcoxy inférieur contenant jusqu'à 4 atomes de carbone inclus ou un halogène de numéro atomique allant jusqu'à 35 inclus et $R_6$ représente l'hydrogène, un groupe alkyle inférieur contenant jusqu'à 4 atomes de carbone inclus, un halogène de numéro atomique allant jusqu'à 35 inclus, un groupe (alcoxy inférieur)-carbonyle contenant jusqu'à 4 atomes de carbone inclus dans la partie alcoxy inférieur, un groupe

carboxy ou alcanoyle inférieur contenant jusqu'à 7 atomes de carbone inclus et alk représente un groupe alkylène inférieur contenant jusqu'à 4 atomes de carbone inclus, et leurs sels, en particulier leurs sels acceptables pour l'usage pharmaceutique.

3. Procédé selon la revendication 1, pour préparer des composés selon la revendication 1, de formule I dans laquelle $R_1$ représente un groupe alkyle inférieur contenant jusqu'à 4 atomes de carbone inclus, $R_2$ représente un groupe alkyle inférieur à chaîne droite contenant de 2 à 4 atomes de carbone inclus, $R_3$ représente l'hydrogène et $R_5$ un groupe oxaloamino ou 5-tétrazolyl-carbonylamino, et ou bien $R_4$ représente l'hydrogène et $R_6$ et $R_7$, indépendamment l'un de l'autre, l'hydrogène ou un groupe alkyle inférieur contenant jusqu'à 4 atomes de carbone inclus, ou bien $R_4$ représente un groupe alkyle inférieur contenant jusqu'à 4 atomes de carbone inclus ou un groupe trifluorométhyle, $R_6$ représente l'hydrogène ou un halogène de numéro atomique allant jusqu'à 35 inclus et $R_7$ représente l'hydrogène, ou bien $R_4$, $R_6$ et $R_7$ représentent des atomes d'halogènes identiques ou différents de numéro atomique allant jusqu'à 35 inclus, et alk représente un groupe alkylène inférieur à chaîne droite en position terminale contenant 2 à 4 atomes de carbone inclus, et leurs sels, en particulier leurs sels de bases acceptables pour l'usage pharmaceutique.

4. Procédé selon la revendication 1, pour préparer des composés selon la revendication 1, de formule I dans laquelle $R_1$ représente un groupe alkyle inférieur contenant jusqu'à 4 atomes de carbone inclus, $R_2$ représente un groupe alkyle inférieur à chaîne droite contenant jusqu'à 4 atomes de carbone inclus, $R_3$ et $R_7$ représentent l'hydrogène, l'un des symboles $R_4$ et $R_6$ représente un groupe alkyle inférieur contenant jusqu'à 4 atomes de carbone inclus et l'autre un halogène de numéro atomique allant jusqu'à 35 inclus, $R_5$ représente un groupe oxaloamino ou 5-tétrazolylcarbonylamino et alk représente un groupe alkylène inférieur à chaîne droite en position terminale contenant jusqu'à 7 atomes de carbone inclus, et leurs sels, en particulier leurs sels de bases acceptables pour l'usage pharmaceutique.

5. Procédé selon la revendication 1, pour préparer l'acide N-{3-[3-(4-acétyl-3-hydroxy-2-n-propylphénoxy)-propyloxy]-6-méthylphényl}-oxamique ou l'un de ses sels, l'acide N-{3-[3-(4-acétyl-3-hydroxy-2-n-propylphénoxy)-propyloxy]-4-bromo-6-méthylphényl}-oxamique ou l'un de ses sels, le N-{3-[3-(4-acétyl-3-hydroxy-2-n-propylphénoxy)-propyloxy]-2-cyanophényl}-1H-tétrazole-5-carboxamide ou l'un de ses sels, le N-{3-[3-(4-acétyl-3-hydroxy-2-n-propylphénoxy)-propyloxy]-6-bromo-4-méthylphényl}-1H-tétrazole-5-carboxamide ou l'un de ses sels, l'acide N-{3-[3-(4-acétyl-3-hydroxy-2-n-propylphénoxy)-propyloxy]-2-cyanophényl}-oxamique ou l'un de ses sels, l'acide N-{3-[3-(4-acétyl-3-hydroxy-2-n-propylphénoxy)-propyloxy]-2,4,6-trichlorophényl}-oxamique ou l'un de ses sels, le N-{3-[3-(4-acétyl-3-hydroxy-2-n) propylphénoxy)-propyloxy]-phényl}-tétrazole-5-carboxamide ou l'un de ses sels, l'acide N-{3-[3-(4-acétyl-3-hydroxy-2-n-propylphénoxy)-propyloxy]-6-chloro-4-méthylphényl}-oxamique ou l'un de ses sels, le N-{3-[3-(4-acétyl-3-hydroxy-2-n-propyl-phénoxy)-propyloxy]-6-chloro-4-méthyl-phényl}-1H-tétrazole-5-carboxamide ou l'un de ses sels, ou le N-{3-[3-(4-acétyl-3-hydroxy-2-n-propylphénoxy)-propyloxy]-4-chloro-6-méthylphényl}-1H-tétrazole-5-carboxamide ou l'un de ses sels.

6. Procédé selon la revendication 1, pour préparer l'acide N-{3-[5-(4-acétyl-3-hydroxy-2-n-propylphénoxy)-pentyloxy]-phényl}-oxamique ou l'un de ses sels, le N-{3-[3-(4-acétyl-3-hydroxy-2-n-propylphénoxy)-propyloxy]-phényl}-oxamate de méthyle, l'acide N-{3-[3-(4-acétyl-3-hydroxy-2-n-propyl-phénoxy)-propyloxy]-phényl}-oxamique ou l'un de ses sels, le N-{3-[3-(4-acétyl-3-hydroxy-2-n-propylphénoxy)-propyloxy}-4-bromo-6-méthylphényl}-oxamate de méthyle, le N-{3-[3-(4-acétyl-3-hydroxy-2-n-propylphénoxy)-propyloxy]-6-méthylphényl}-oxamate de méthyle, le N-{3-[3-(4-acétyl-3-hydroxy-2-n-propylphénoxy)-propyloxy]-2-méthylphényl}-oxamate de méthyle, l'acide N-{3-[3-(4-acétyl-3-hydroxy-2-n-propylphénoxy)-propyloxy]-2-méthylphényl}-oxamique ou l'un de ses sels, le N-{3-[5-(4-acétyl-3-hydroxy-2-n-propylphénoxy)-pentyloxy]-phényl}-oxamate de méthyle, le N-{3-[3-(4-acétyl-3-hydroxy-2-n-propylphénoxy)-propyloxy]-4-chloro-6-trifluorométhylphényl}-oxamate de méthyle, l'acide N-{3-[3-(4-acétyl-3-hydroxy-2-n-propylphénoxy)-propyloxy]-4-chloro-6-trifluorométhylphényl}-oxamique ou l'un de ses sels, le N-{3-[3-(4-acétyl-3-hydroxy-2-n-propylphénoxy)-propyloxy] 2,4,6-trichlorophényl}-oxamate de méthyle, le N-{3-[3-(4-acétyl-3-hydroxy-2-n-propylphénoxy)-propyloxy]-4,6-diméthylphényl}-oxamate de méthyle, l'acide N-{3-[3-(4-acétyl-3-hydroxy-2-n-propylphénoxy)-propyloxy]-4,6-diméthylphényl}-oxamique ou l'un de ses sels, le N-{4-[3-(4-acétyl-3-hydroxy-2-n-propylphénoxy)-propyloxy]-3-méthoxyphényl}-oxamate de méthyle, l'acide N-{4-[3-(4-acétyl-3-hydroxy-2-n-propylphénoxy)-propyloxy]-3-méthoxyphényl}-oxamique ou l'un de ses sels, le N-{4-[3-(4-acétyl-3-hydroxy-2-n-propylphénoxy)-propyloxy]-2-méthylphényl}-oxamate de méthyle, l'acide N-{4-[3-(4-acétyl-3-hydroxy-2-n-propylphénoxy)-propyloxy]-2-méthylphényl}-oxamique ou l'un de ses sels, le N-{2-[3-(4-acétyl-3-hydroxy-2-n-propylphénoxy)-propyloxy]-phényl}-oxamate de méthyle, l'acide N-{2-[3-(4-acétyl-3-hydroxy-2-n-propylphénoxy)-propyloxy]-phényl}-oxamique ou l'un de ses sels, le N-{4-[3-(4-acétyl-3-hydroxy-2-n-propylphénoxy)-propyloxy]-phényl]-oxamate de méthyle, l'acide N-{4-[3-(4-acétyl-3-hydroxy-2-n-propylphénoxy)-propyloxy]-phényl}-oxamique ou l'un de ses sels, le N-{3-[3-(4-acétyl-3-hydroxy-2-n-propylphénoxy)-propyloxy]-4-bromo-6-méthylphényl}-tétrazole-5-carboxamide ou l'un de ses sels, l'acide N-{3-[3-(4-acétyl-3-hydroxy-2-n-propylphénoxy)-propyloxy]-4-carboxyphényl}-oxamique ou un sel acide ou neutre de cet acide, l'acide N-{3-[3-(4-acétyl-3-hydroxy-2-n-propylphénoxy)-propyloxy]-4-méthoxycarbonylphényl}-oxamique ou l'un de ses sels, le N-{3-[3-(4-acétyl-3-hydroxy-2-n-propylphénoxy)-propyloxy]-4-bromo-6-méthylphényl}-oxamate d'éthyle, le N-{3-[3-(4-acétyl-3-hydroxy-2-n-propylphénoxy)-propyloxy]-6-chloro-4-méthylphényl}-oxamate de méthyle, le N-{3-[3-(4-acétyl-3-hydroxy-2-n-propylphénoxy)-propyloxy]-4-chloro-6-méthylphényl}-oxamate de méthyle,

le N-{3-[3-(4-acétyl-3-hydroxy-2-n-propylphénoxy)-propyloxy]-6-bromo-4-méthylphényl}-oxamate de méthyle, l'acide N-{3-[3-(4-acétyl-3-hydroxy-2-n-propylphénoxy)-propyloxy]-4-chloro-6-méthylphényl}-oxamique ou l'un de ses sels, ou le N-{3-[3-(4-acétyl-3-hydroxy-2-n-propylphénoxy)-propyloxy]-2-cyano-phényl}-oxamate d'éthyle.

7. Procédé de préparation d'un composé salifiable selon l'une des revendications 1 à 6, à l'état de sels de triéthanolammonium.

8. Procédé de préparation d'un composé selon l'une des revendications 1 à 4, ou de l'acide N-{3-[3-(4-acétyl-3-hydroxy-2-n-propylphénoxy)-propyloxy]-6-méthylphényl}-oxamique, de l'acide N-{3-[3-(4-acétyl-3-hydroxy-2-n-propylphénoxy)-propyloxy]-4-bromo-6-méthylphényl}-oxamique ou de l'acide N-{3-[5-(4-acétyl-3-hydroxy-2-n-propylphénoxy)-pentyloxy]-phényl}-oxamique à l'état de sel de sodium.

9. Procédé de préparation de compositions pharmaceutiques, caractérisé en ce que l'on mélange un composé obtenu selon l'une des revendications 1 à 8 avec des produits auxiliaires et/ou véhicules pharmaceutiques usuels.

10. Procédé selon la revendication 9 pour la préparation d'un anti-allergique, d'un anti-asthmatique, d'un anti-inflammatoire et/ou d'un antiphlogistique.